(19) Europäisches Patentamt
European Patent Office
Office européen des brevets

(11) EP 2 441 447 A1

(12) # EUROPEAN PATENT APPLICATION

(43) Date of publication:
**18.04.2012 Bulletin 2012/16**

(51) Int Cl.:
***A61K 9/51*** *(2006.01)*

(21) Application number: **11192165.6**

(22) Date of filing: **05.05.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **06.05.2008 US 50775 P**
**20.06.2008 US 74171 P**

(62) Document number(s) of the earlier application(s) in accordance with Art. 76 EPC:
**09742071.5 / 2 271 323**

(27) Previously filed application:
**05.05.2009 PCT/EP2009/055436**

(71) Applicant: **Glaxo Group Limited**
**Middlesex UB6 0NN (GB)**

(72) Inventors:
- **Catchpole, Ian Richard**
  **Stevenage, Hertfordshire SG1 2NY (GB)**
- **Gough, Gerald, Wayne**
  **Stevenage, Hertfordshire SG1 2NY (GB)**
- **Papanicolaou, Irene**
  **Stevenage, Hertfordshire SG1 2NY (GB)**

(74) Representative: **Harrison, Anna et al**
**GlaxoSmithKline**
**Corporate Intellectual Property**
**980 Great West Road**
**(CN925.1)**
**Brentford, Middlesex TW8 9GS (GB)**

Remarks:
This application was filed on 06-12-2011 as a divisional application to the application mentioned under INID code 62.

(54) **ENCAPSULATION OF BIOLOGICALLY ACTIVE AGENTS**

(57) The present invention provides particulate carriers comprising encapsulated biologically active agents such as proteins for ocular delivery. Also provided are methods of delivery of proteins to the eye in nanoparticles, and uses of compositions comprising such particulate carriers in treatment.

EP 2 441 447 A1

**Description**

**Background**

A number of drugs have activity at targets in the brain or in the eye, in order to get these to their target they must pass through a biological barrier such as the blood brain barrier. While some molecules are able to cross biological barriers, there are others which do not pass these barriers efficiently or in fact at all. Many drugs are also only efficient when given directly into the target tissue and if this target tissue cannot be reached the drug simply cannot work. Therefore many potentially potent drugs are not useful clinically due to their inability to pass such biological barriers.

A number of approaches have been described in the art to increase drug penetration through these biological barriers.

One approach has been to alter the function of the barrier itself. For instance, osmotic agents or cholinomimetic arecolines result in the opening, or a change in the permeability, of the blood brain barrier *(*Saija A et al, J Pharm. Pha. 42:135-138 (1990*))*.

Another approach resides in the modification of the drug molecules themselves. For instance modifications of proteins to attempt passage across the blood brain barrier include glycating such proteins, or alternatively by forming a prodrug.
*(*WO/2006/029845*).*

Still another approach is the implantation of controlled release polymers which release the active ingredient from a matrix system directly into the nervous tissue. However, this approach is invasive and requires surgical intervention if implanted directly into the brain or spinal cord (sable et al. US patent no. 4,833,666) this presents problems with patient compliance and often only allows for localised delivery within the brain with the administered drug usually draining away very quickly. *(*WO/2006/029845*).*

To overcome these limitations drug carrier systems have been used however, a major problem in targeted drug delivery is the rapid opsonisation and uptake of injected carriers by the reticuloendothelial system (RES) especially by the macrophages in the liver and spleen.

There remains therefore a need for an efficient and effective means of delivering macromolecules such as proteins to the brain and to the eye. In particular, it would be desirable to find a method of delivery of macromolecules across the blood brain barrier, which would retain activity on entry into the brain, and which may also provide desirable release kinetics, maintain protein stability and activity, and have the ability to evade clearance mechanisms.

**Brief Description of the Figures.**

Figure 1 shows the sizing data obtained for a nanoparticle formulation by dynamic light scattering (DLS) that indicate the presence of nanoparticles prepared via the hollow method in suspension.

Fig. 1(a) Correlogram obtained following analysis of a nanoparticle suspension by dynamic light scattering.

Fig 1 (b) Multimodal size distribution (derived data) of the nanoparticles plotted to depict the distribution of the particle population (number) over a range of sizes.

Fig. 1(c) Multimodal size distribution (derived data) of the nanoparticles plotted to depict the distribution of the particle population (number) over a range of sizes.

Fig 1(d) Multimodal size distribution (derived data) of the nanoparticles plotted to depict the distribution of the particle population (number) over a range of sizes.

Figure 2. - Nanoparticles analysed by SEM

Figure 3 - Image of hollow nanoparticles by TEM, with a superimposed image of solid PBCA nanoparticles for comparison.

Figure 4 - Encapsulation efficiency measurements of monoclonal IgG1 (anti-CD23).

Figure 5 - Release profile obtained following enzymatic degradation of particles and analysis of the released enzyme by ELISA.

Figure 6 - Determination of the encapsulation efficiency of a domain antibody (hen egg lysozyme dAb) by the bicinchoninic acid assay (BCA assay) in a hollow PBCA nanoparticle.

Figure 7. Sizing data obtained by DLS that indicate the presence of nanoparticles in suspension.

Fig 7(a) - Correlogram obtained following analysis of a nanoparticle suspension by dynamic light scattering.

Fig 7(b) - Multimodal size distribution (derived data) of the nanoparticles plotted to depict the distribution of the particle population (number) over a range of sizes.

Fig 7(c) - Multimodal size distribution (derived data) of the nanoparticles plotted to depict the distribution of the particle population (number) over a range of sizes.

Fig 7(d) - Multimodal size distribution (derived data) of the nanoparticles plotted to depict the distribution of the particle population (number) over a range of sizes.

Figure 8. Comparison of the amounts of encapsulated Dalargin achieved with the HIP method to those achieved by the common method of adsorption onto the particle surface.

Figure 9 - Dalargin levels in the brain following delivery with HIP-PBCA nanoparticles. The peptide was detectable in the brain only when encapsulated within the particles using the HIP process.

Figure 10. - Encapsulation of dalargin into PBCA nanoparticles using the HIP process. Determination of the effect of pH of the aqueous phase on the encapsulation efficiency.

Figure 11. - Encapsulation of anti-hen egg lysozyme domain antibody into PBCA nanoparticles using the HIP process. The nanoparticles were analysed by Edman sequencing.

Figure 12 - Encapsulation efficiency measurements of monoclonal IgG1 (anti-CD23).

Figure 13 - Confirmation of encapsidation of dAbs into HIP-PBCA nanoparticles by SDS-PAGE analysis. The nanoparticles were centrifuged to remove any free dAb and the pellets analysed by SDS-PAGE to visualise encapsidated dAb.

Figure 14 - Determination of the loading of VEGF dAb (DOM15-26-593) into HIP-PBCA nanoparticles by SDS-PAGE analysis. Nanoparticle formulations were compared to dAb standards in order to quantify the amount of dAb present in the nanoparticles. A total of 3.31 mg of dAb had been encapsidated in the nanoparticles out of the starting input of 12 mg. Therefore, the loading efficiency was 27.6 %. The dAb loading was 3.31 % w/w.

Figure 15 - Results from the *in vivo* evaluation of HIP PBCA nanoparticles containing domain antibodies for their ability to deliver their protein load to the brain in the mouse via the intravenous route. At 10 minutes post administration, the dAb in nanoparticles resulted in detectable brain uptake which amounted to 8.0 ng/ml. The free dAb was also detectable in the brain at the slightly lower concentration of 3.3 ng/ml (preliminary data). Therefore, the nanoparticles appeared to marginally increase the brain uptake of the protein (preliminary data). At 60 minutes the opposite was observed as the free dAb appeared to accumulate into the brain resulting in a further increase in its brain levels to 13.5 ng/ml. Brain levels were corrected.

Figure 16 - Brain to blood ratios of dAb derived from the *in vivo* evaluation of HIP PBCA nanoparticles containing domain antibodies via the intravenous route. The results show that higher proportions of dAb were present in the brain compared to the blood when given with nanoparticles compared to when the dAb was given free in solution.

Figure 17 - Results from the *in vivo* evaluation of HIP PBCA nanoparticles containing domain antibodies for their ability to deliver their protein load to the brain in the mouse via the intracarotid route. At 10 minutes post administration, the dAb in nanoparticles group exhibited high levels of dAb in the brain, at an average of 627.60 ng/ml.

Figure 18 - Brain to blood ratios of dAb derived from the *in vivo* evaluation of HIP PBCA nanoparticles containing domain antibodies via the intracarotid route. The dAb in nanoparticles group exhibited brain to blood ratios that were greater than 1 at both time points (1.569 and 1.845 at10 and 60 minutes respectively) suggesting that the majority of formulated dAb had successfully reached the brain.

Figure 19 - Confirmation of generation of microspheres by light microscopy.
Formulations of microspheres were all generated by the HIP process using polycaprolactone.

(a) Vit E TPGS 2% surfactant 4000rpm 2 min 20x mag
(b) Vit E TPGS 2% surfactant 7500rpm 2min 20x mag
(c) Vit E TPGS 2% surfactant 7500rpm 2min + dAb1 20x mag
(d) Vit E TPGS 2% surfactant 7500rpm 2min + dAb2 20x mag

Figure 20 - Confirmation of generation of microspheres by laser diffraction.
Formulations of microspheres were all generated by the HIP process using polycaprolactone.

(a) Vit E TPGS 2% surfactant 4000rpm 2 min 20x mag
(b) Vit E TPGS 2% surfactant 7500rpm 2min 20x mag
(c) Vit E TPGS 2% surfactant 7500rpm 2min + dAb1 20x mag
(d) Vit E TPGS 2% surfactant 7500rpm 2min + dAb2 20x mag

Figure 21 - Confirmation of encapsidation of dAbs into HIP-PC microspheres by SDS-PAGE analysis. The microspheres were filtered, (F) centrifuged, (3k or 13K rpm) to remove any free dAb and the supernatant, (S), and the pellets, (P), analysed by SDS-PAGE to visualise encapsidated dAb.

Figure 22 - Confirmation of release of encapsidated dAb from HIP-PC microspheres by SDS-PAGE analysis. The microspheres were washed and then heat treated at 56°C for 0, 20, 40 or 60 mins to release dAb, the debris pelleted, (5 mins @ 5k) and the supernatant, (S), analysed by SDS-PAGE to visualise encapsidated dAb. Molecular markers - SeeBlue Plus 2 pre-stained standard, (invitrogen), molecular weight (kd), The gel confirmed that release of the dAbs had taken place. The gel also confirmed that the dAbs were intact and that they had not fragmented due to the release process.

## Summary of Invention

In one aspect of the present invention there is provided a method of encapsulating biologically active agents in particulate carriers such as methods of encapsulating proteins and or peptides in, or in and on, or with nanoparticles and a method of delivery of proteins and or peptides across the blood brain barrier by encapsulation in, or in and on, or with nanoparticles and a method of delivery of proteins and or peptides to the eye by encapsulation in, or in and on, or with particulate carriers.

In another embodiment of the present invention there are provided particulate carriers comprising a particle forming substance and a biologically active agent such as a protein and or peptide, for delivery of a protein and or peptide from the blood to the brain across the blood brain barrier or for delivery to the eye. In another embodiment of the invention are compositions of nanoparticles and their use in treating disorders or diseases of the central nervous system and or eye.

## Detailed Description of invention

The present invention provides particulate carriers comprising a particle forming substance and a biologically active agent, and methods of making said particulate carriers.

In one embodiment the nanoparticles of the present invention comprise biologically active agents such as proteins or peptides. Such proteins may be antigen binding molecules which as used herein refers to antibodies, antibody fragments and other protein constructs which are capable of binding to a target.

Antigen binding molecules may comprise a domain. A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. A "single antibody variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

Antigen binding molecules may comprise at least one immunoglobulin variable domain, for example such molecules may comprise an antibody, a domain antibody, Fab, Fab', F(ab')2, Fv, ScFv, diabody, heteroconjugate antibody. Such antigen binding molecules may be capable of binding to a single target, or may be multispecific, i.e. bind to a number of targets, for example they may be bispecific or trispecfic. In one embodiment the antigen binding molecule is an antibody. In another embodiment the antigen binding molecule is a domain antibody (dAb). In yet a further embodiment the antigen binding molecule may be a combination of antibodies and antigen binding fragments such as for example, one or more dAbs and or one or more ScFvs attached to a monoclonal antibody. In yet a further embodiment the antigen binding molecule may be a combination of antibodies and peptides. Antigen binding molecules may comprise at least one non-Ig binding domain such as a domain that specifically binds an antigen or epitope independently of a different V region or domain, this may be a dAb, for example a human, camelid or shark immunoglobulin single variable domain or it may be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human -crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand.

CTLA-4 (Cytotoxic T Lymphocyte-associated Antigen 4) is a CD28-family receptor expressed on mainly CD4+ T-cells. Its extracellular domain has a variable domain-like Ig fold. Loops corresponding to CDRs of antibodies can be substituted with heterologous sequence to confer different binding properties. CTLA-4 molecules engineered to have different binding specificities are also known as Evibodies. For further details see Journal of Immunological Methods 248 (1-2), 31-45 (2001)

Lipocalins are a family of extracellular proteins which transport small hydrophobic molecules such as steroids, bilins, retinoids and lipids. They have a rigid -sheet secondary structure with a numer of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633

An affibody is a scaffold derived from Protein A of *Staphylococcus aureus* which can be engineered to bind to antigen. The domain consists of a three-helical bundle of approximately 58 amino acids. Libraries have been generated by randomisation of surface residues. For further details see Protein Eng. Des. Sel. 17, 455-462 (2004) and EP1641818A1

Avimers are multidomain proteins derived from the A-domain scaffold family. The native domains of approximately 35 amino acids adopt a defined disulphide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007) A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two -helices and a -turn. They can be engineered to bind different target antigens by randomising residues in the first -helix and a -turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the - sandwich can be engineered to enable an Adnectin to specifically recognize a therapeutic target of interest. For further details see Protein Eng. Des. Sel. 18, 435-444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataB1 and conotoxin and knottins. The microproteins have a loop which can be engineered to include upto 25 amino acids without affecting the overall fold of the microprotein. For further details of engineered knottin domains, see WO2008098796.

Other non Ig binding domains include proteins which have been used as a scaffold to engineer different target antigen binding properties include human -crystallin and human ubiquitin (affilins), kunitz type domains of human protease inhibitors, PDZ-domains of the Ras-binding protein AF-6, scorpion toxins (charybdotoxin), C-type lectin domain (tetranectins) are reviewed in Chapter 7 - Non-Antibody Scaffolds from Handbook of Therapeutic Antibodies (2007, edited by Stefan Dubel) and Protein Science 15:14-27 (2006). Non Ig binding domains of the present invention could be derived from any of these alternative protein domains.

In one embodiment of the invention the antigen binding molecule binds to a target found in the central nervous system such as for example in the brain or spinal cord, or for example in neuronal tissue.

In yet a further embodiment of the invention described herein the antigen binding molecule specifically binds to a target known to be linked to neurological diseases or disorders such as for example MAG (myelin associated glycoprotein), NOGO (neurite outgrowth inhibitory protein) or β-amyloid.

Such antigen binding molecules include antigen binding molecules capable of binding to NOGO for example anti-NOGO antibodies. One example of an anti-NOGO antibody for use in the present invention is the antibody defined by the heavy chain of SEQ ID NO 1 and the light chain of SEQ ID NO 2 or an anti-NOGO antibody or antigen binding fragment thereof which comprises the CDRs of the antibody set out in SEQ ID NO 1 and 2.. Further details of this antibody (H28 L16) can be found in PCT application WO2007068750 which is herein incorporated by reference.

Such antigen binding molecules include antigen binding molecules capable of binding to MAG for example anti-MAG antibodies. One example of the anti-MAG antibody for use in the present invention is the antibody defined by the heavy chain variable region of SEQ ID NO 11 and the light chain variable region of SEQ ID NO 12 or an anti-MAG antibody or antigen binding fragment thereof which comprises the CDRs of the antibody set out in SEQ ID NO 1 and 2.. Further details of this antibody (BvH1 CvL1) can be found in PCT application WO2004014953 which is herein incorporated by reference.

Such antigen binding molecules include antigen binding molecules capable of binding to β-amyloid for example anti- β-amyloid antibodies. One example of the anti- β-amyloid antibody for use in the present invention is the antibody defined by the heavy chain of SEQ ID NO 5 and or the light chain of SEQ ID NO 6 or an anti- β-amyloid antibody or antigen binding fragment thereof which comprises the CDRs of the antibody set out in SEQ ID NO 5 and 7. Further details of this antibody (H2L1) can be found in PCT application WO2007113172 which is herein incorporated by reference.

The CDR sequences of such antibodies can be determined by the Kabat numbering system (Kabat et al; Sequences of proteins of Immunological Interest NI H, 1987), the Chothia numbering system (Al-Lazikani et al., (1997) JMB 273,927-948), the contact definition method (MacCallum R.M., and Martin A.C.R. and Thornton J.M, (1996), Journal of Molecular Biology, 262 (5), 732-745) or any other established method for numbering the residues in an antibody and determining CDRs known to the skilled man in the art.

In one embodiment of the invention the antigen binding protein binds to a target found in the eye such as for example TNF, TNFr-1, TNFr-2, TGFbeta receptor-2, VEGF, NOGO, MAG, IL-1, IL-2, IL-6, IL-8, IL-17, CD20, Beta amyloid, FGF-2, IGF-1, PEDF, PDGF or a complement factor for example C3, C5, C5aR, CFD, CFH, CFB, CFI, sCR1 or C3,

In one embodiment of the present invention there is provided a composition comprising nanoparticles according to any method of the invention as presented herein. In a further embodiment at least about 90% of the nanoparticles by number are within the range of about 1 nm to about 1000nm when measured using dynamic light scattering techniques. In a further embodiment at least about 90% of the nanoparticles by number are within the range of about 1nm to about 400nm, or about 1 nm to about 250nm or about 1 nm to about 150nm, or about 40nm to about 250nm, or about 40nm to about 150nm, or about 40nm to about 100nm when measured using dynamic light scattering techniques.

In yet a further embodiment of the present invention at least about 90% of the nanoparticles by number are within the range of about 40nm to about 250 nm when measured using dynamic light scattering techniques.

In yet a further embodiment of the present invention at least about 90% of the nanoparticles by number are within the range of about 40nm to about 150 nm when measured using dynamic light scattering techniques.

In yet a further embodiment there is provided a composition comprising the nanoparticles of the present invention wherein the median size of the nanoparticles in the composition is less than about 1000nm in diameter, for example is less than about 400nm in diameter for example is less than about 250nm in diameter, for example is less than about 150nm in diameter when measured by light scattering techniques.

In yet a further embodiment the median size of the nanoparticles in the composition is about 40nm to about 250nm.

In yet a further embodiment the median size of the nanoparticles in the composition is about 40nm to about 150nm.

In one embodiment of the present invention there is provided a method of encapsulating a biologically active agent in a particulate carrier comprising the steps of:

a) solubilising a biologically active agent in the presence of a hydrophobic ion pairing (HIP) agent and in an organic solvent;

b) dissolving a monomer and or oligomer of a polymer forming substance in the organic phase formed in (a);

c) forming an emulsion of the organic phase formed in (b) in a continuous aqueous phase to allow polymerisation of the monomer; and

d) obtaining particulate carriers formed from the emulsion.

In a further embodiment of the present invention there is provided a method of encapsulating biologically active agents in a particulate carrier comprising the steps of:

a) mixing a biologically active agent in an aqueous phase with a hydrophobic ion pairing (HIP) agent in an organic solvent phase to form a biologically active agent-HIP complex;
b) separation of the complex from the aqueous phase.
c) removal of the aqueous phase and homogenisation of the complex with the organic phase;
d)

(i) dissolving a polymer in the organic phase formed in (c) and then forming an emulsion of the organic phase in a continuous aqueous phase; or
(ii) dissolving a monomer or oligomer of a polymer forming substance, in the organic phase formed in (c) and then forming an emulsion of the organic phase in a continuous aqueous phase; to allow polymerisation of the monomer or oligomer to form a polymer; and

e) obtaining a particulate carrier formed from the emulsion of step (d).

This method using hydrophobic ion pairing agents allows encapsulation of biologically active agents for example proteins such as for example hydrophilic proteins within the core of the hydrophobic polymer particles. Hydrophobic ion pairing allows extraction of protein into an organic medium and therefore the method enables preparation of a particulate carrier with a single emulsion.

In a further embodiment the biologically active agents to be encapsulated in accordance with the method are peptides or proteins, such as antigen binding molecules.

In one embodiment of the invention the protein binds to a target found in the central nervous system such as for example in the brain or spinal cord or for example in the neuronal tissue.

In yet a further embodiment of the invention described herein the protein specifically binds to an epitope known to be linked to neurological diseases or disorders such as for example MAG (myelin associated glycoprotein), NOGO (neurite outgrowth inhibitory protein) or β-amyloid.

In a further embodiment of the invention described herein the protein is an antibody which binds to NOGO, for example any of the anti-NOGO antibodies described in PCT application WO2007068750, in particular the antibody referred to as H28 L16 in PCT application WO2007068750 which is herein incorporated by reference.

In a further embodiment of the invention described herein the protein is an antibody which binds to MAG for example any of the anti-MAG antibodies described in PCT application WO2004014953 in particular the antibody referred to as BvL1 CvL1 in PCT application WO2004014953 which is herein incorporated by reference.

In a further embodiment of the invention described herein the protein is an antibody which binds to β-amyloid for example any of the anti- β-amyloid antibodies described in PCT application WO2007113172 in particular the antibody referred to as H2 L1 in PCT application WO2007113172 which is herein incorporated by reference.

In one embodiment of the present invention the particulate carriers may be microspheres or nanoparticles. In one such embodiment the particulate carrier is a nanoparticle and the biologically active agent is a protein. In another embodiment the particulate carrier is a nanoparticle and the biologically active agent is a peptide. In a further embodiment the particulate carrier is a nanoparticle and the biologically active agent comprises an antigen binding molecule for example a domain antibody or antibody. In yet a further embodiment the particulate carrier is a nanoparticle and the biologically active agent comprises a domain. In another embodiment the particulate carrier is a microsphere and the biologically active agent is a protein. In a further embodiment the particulate carrier is a microsphere and the biologically active agent is a peptide. In yet a further embodiment the particulate carrier is a microsphere and the biologically active agent comprises an antigen binding molecule for example a domain antibody or antibody. In yet a further embodiment the particulate carrier is a microsphere and the biologically active agent comprises a domain.

In one embodiment of the invention described herein the biologically active agent specifically binds to a target known to be linked to neurological diseases or disorders such as for example to MAG, NOGO, or β-amyloid.

In one embodiment the biologically active agent is insoluble in the organic phase without the presence of hydrophobic ion pairing agents.

In one embodiment of the invention as herein described the hydrophobic ion pairing agent is a cationic HIP agent when the protein is anionic. In another embodiment the hydrophobic ion pairing agent is an anionic HIP agent when the protein is cationic. In a further embodiment the anionic HIP agent is selected from the group consisting of Alkyl quaternary ammonium cations, preferably alkyl ammonium bromides, more preferably tetrabutyl ammonium bromide, tetrahexyl ammonium bromide, tetraoctyl ammonium bromide, Sodium dodecyl sulphate (SDS), sodium oleate or docusate sodium (aka Aerosol OT™) and the HIP agent is present in stoichiometric amounts equal to or greater than the number of net positive charges on the protein. In another embodiment, the cationic HIP agent is selected from the group consisting of: dimethyldioctadecyl-ammonium bromide (DDAB18); 1,2-dioleoxy-3-(trimethylammonium propane (DOTAP); or cetrimonium bromide (CTAB) and the HIP agent is present in stoichiometric amounts equal to or greater than the number of net negative charges on the protein.

In a further embodiment any hydrophobic cation or anion could potentially be used as a HIP agent to solubilise the protein. Hydrophobic ion pairing (HIP) involves stoichiometric replacement of polar counter ions with a species of similar charge but less easily solvated. As disclosed herein, the invention provides a method that uses HIP to change the solubility properties of proteins, allowing extraction of the protein into an organic solvent, such as methylene chloride. Docusate sodium (Bis(2-ethylhexyl) sodium sulfosuccinate) is one example of a suitable ion-pairing agent. In one embodiment, methylene chloride containing docusate sodium is mixed with an aqueous protein solution. This results in ion pairing of the docusate ion with the protein and subsequent partitioning of the protein into the oil phase. Dissolution of the protein in methylene chloride allows the protein to be encapsulated in nanoparticles or microspheres prepared via a single oil-in-water emulsion method.

In one embodiment of the invention herein described the continuous aqueous phase has a pH of about 7.0 or higher when the protein is anionic and the HIP agent is cationic, for example the pH may be at least about 8.0 or at least about 10.0 or is at least about 12.0.

In an alternative embodiment of the invention herein described the continuous aqueous phase has a pH of about 7.0 or lower when the protein is cationic and the HIP agent is anionic, for example the pH may be less than about 6.0 or less than about 4.0 or less than about 2.0.

In one such embodiment the weight/weight (w/w) ratio of protein to polymer may be 0.5% to 90% for example is at least about 0.5% or is at least about 1% or is at least about 2% or is at least about 2.5% or is at least about 5% or is at least about 9% or is at least about 10% or is at least about 15% or is at least about 20% or is at least about 40%, or is at least about 50%, or is at least about 60%, or is at least about 70%, or is at least about 80% or is at least about 90%. For example when the protein is a peptide the peptide to polymer ratio may be at least about 9%, when the protein is an antibody the antibody to polymer ratio may be at least about 2%, or when the protein is a domain antibody the domain antibody to polymer ratio may be at least about 2.5%.

In one embodiment of the present invention the w/w ratio of protein to total formulation (polymer +HIP and optionally surfactants) may be 0.5% to 50% for example is at least about 5% or at least about 9% or at least about 15% or at least about 16% or at least about 20% or at least about 25%. For example when the protein is a peptide the peptide to total formulation ratio may be at least about 16% or when the protein is an antibody the antibody to polymer ratio may be at least about 1 %, or when the protein is a domain antibody the domain antibody to total formulation ratio may be at least about 9%.

In one embodiment of the present invention the encapsulation efficiency of the particles is at least about 1% or is at least about 2% or is at least about 10% or is at least about 20% or is at least about 40% or is at least about 50% or is at least about 60% or is at least about 70% or is at least about 80% or is at least about 90% or is alt least about 95% or is least about 97% or is at least about 99%. For example when the protein is a peptide the encapsulation efficiency may be at least about 90%, when the protein is an antibody the encapsulation efficiency may be at least about 1%, or when the protein is a domain antibody the encapsulation efficiency may be at least about 70%.

In one embodiment of the present invention the monomer or oligomer is selected from the group consisting of methylmethacrylates, alkylcyanoacrylates, hydroxyethylmethacrylates, methacrylic acid, ethylene glycol dimethacrylate, acrylamide, N, N'-bismethylene acrylamide and 2-dimethylaminoethyl methacrylate. In a further embodiment the monomer is an alkylcyanoacrylate for example is butylcyanoacrylate (BCA).

In one embodiment there is provided a polymeric particulate carrier comprising a biologically active agent in an aqueous phase in a hollow lumen.

In yet another embodiment of the present invention there is provided a method of encapsulating biologically active agents in particulate carriers for ocular delivery comprising the steps of:

a) dissolving a polymer in an organic solvent to form a polymer solution;
b) adding an aqueous solution containing a biologically active agent to the polymer solution to form a primary emulsion of aqueous phase droplets in a continuous organic phase;
c) mixing the primary emulsion with an aqueous medium to form a W/O/W emulsion; and
d) allowing the organic phase to evaporate and thereby obtain particulate carriers comprising a hollow lumen containing said biologically active agent in an aqueous phase.

Allowing the organic phase to evaporate may be passive or active. For example active evaporation may be by the use of heat.

In one such embodiment the ocular delivery is periocular, for example trans-scleral, subconjunctival, sub-tenon, peribulbar, topical, retrobulbar or is delivered to the inferior, superior or lateral rectus muscle. In one embodiment the ocular delivery is trans-scleral.

In a further embodiment the biologically active agents to be encapsulated in accordance with the method are peptides or proteins, such as antigen binding molecules.

The particulate carrier used in such methods may be a microsphere or a nanoparticle For example the particulate carrier may be a nanoparticle and the biologically active agent a protein or the particulate carrier may be a nanoparticle and the biologically active agent a peptide or the particulate carrier may be a nanoparticle and the biologically active agent comprises an antigen binding molecule such as a domain antibody or antibody. In yet a further embodiment the particulate carrier is a nanoparticle and the biologically active agent comprises a domain.

Alternatively the particulate carrier may be a microsphere and the biologically active agent a protein or the particulate carrier may be a microsphere and the biologically active agent a peptide or the particulate carrier may be a microsphere and the biologically active agent comprises an antigen binding molecule such as a domain antibody or antibody or a combination thereof. In yet a further embodiment the particulate carrier is a microsphere and the biologically active agent comprises a domain.

In one embodiment of the present invention there is provided a composition comprising microspheres according to any method of the invention as presented herein. In a further embodiment at least about 90% of the microspheres by number have a diameter within the range of about 1 μm to about 100 μm when measured using Low angle laser light scattering techniques. In a further embodiment at least about 90% of the particles by number are within the range of about 1 μm to about 80μm, or about 1μm to about 60μm or about 1μm to about 40μm, or about 1μm to about 30μm or about 1μm to about 10μm when measured using Low angle laser light scattering techniques.

In yet a further embodiment of the present invention at least about 90% of the microspheres by number are within the range of about 1 μm to about 60 μm when measured using Low angle laser light scattering techniques.

In yet a further embodiment of the present invention at least about 90% of the microspheres by number are within the range of about 1 μm to about 30μm when measured using Low angle laser light scattering techniques.

In yet a further embodiment there is provided a composition comprising the microspheres of the present invention wherein the median size of the microspheres in the composition is less than about 100μm in diameter, for example is less than about 80μm in diameter for example is less than about 60μm in diameter, for example is less than about 40μm in diameter when measured by Low angle laser light scattering techniques.

In yet a further embodiment the median size of the microspheres in the composition is about 1μm to about 6μm, or 1μm to about 30μm.

In another embodiment of the invention the particulate carriers continue to release therapeutic amounts of active biological molecules over a period of at least 3 months or longer, or of up to 6 months or longer or of up to 12 months or longer.

Hollow method (limited to nanoparticles of the invention)

In one embodiment of the present invention there is provided a method of producing the nanoparticles of the invention as herein described comprising the steps of:

a) dissolving a polymer in an organic solvent to form a polymer solution;
b) adding an aqueous solution containing protein to the polymer solution to form a primary emulsion of aqueous phase droplets in a continuous organic phase;
c) mixing the primary emulsion with an aqueous medium to form a W/O/Wemulsion; and
d) allowing the organic phase to evaporate and thereby obtain nanoparticles comprising a hollow lumen containing said proteins in an aqueous phase.

Allowing the organic phase to evaporate may be passive or active. For example active evaporation may be by the use of heat.

In a further embodiment the polymer used in any of the methods as described herein is selected from but not limited to: poly - L -lactide (PLA), poly(lacto-co-glycolide) (PLG), poly(lactide), poly(caprolactone), poly(hydroxybutyrate) and/or copolymers thereof. Suitable particle-forming materials include, but are not limited to, poly(dienes) such as poly(butadiene) and the like; poly(alkenes) such as polyethylene, polypropylene, and the like; poly(acrylics) such as poly(acrylic acid) and the like; poly(methacrylics) such as poly(methyl methacrylate), poly(hydroxyethyl methacrylate), and the like; poly(vinyl ethers); poly(vinyl alcohols); poly(vinyl ketones); poly(vinylhalides) such as poly(vinyl chloride) and the like; poly(vinyl nitriles), poly(vinyl esters) such as poly(vinyl acetate) and the like; poly(vinyl pyridines) such as poly(2-vinyl pyridine), poly(5-methyl-2-vinyl pyridine) and the like; poly(styrenes); poly(carbonates); poly(esters); poly(orthoesters); poly(esterarnides); poly(anhydrides); poly(urethanes); poly(amides); cellulose ethers such as methyl cellulose, hydroxyethyl cellulose, hydroxypropyl methyl cellulose, and the like; cellulose esters such as cellulose acetate, cellulose acetate phthalate, cellulose acetate butyrate, and the like; poly(saccharides), proteins, gelatin, starch, gums, resins, and the like. These materials may be used alone, as physical mixtures (blends), or as copolymers.Also polyacrylates, polymethacrylates, polybutylcyanoacrylates, polyalkylcyanoacrylates, polyarylamides, polyanhydrates, polyorthoesters, N,N-L-lysinediylterephthalate, polyanhydrates, desolvated biologically active agents or carbohydrates, polysaccharides, polyacrolein, polyglutaraldehydes and derivatives, copolymers and polymer blends.

In another embodiment of the present invention there is provided a method of encapsulating biologically active agents by producing particulate carriers comprising the steps of:

a) dissolving polybutylcyanoacrylate (PBCA) in an organic solvent to form a polymer solution;
b) adding an aqueous solution containing a biologically active agent to the polymer solution to form a primary emulsion of aqueous phase droplets in a continuous organic phase;
c) mixing the primary emulsion with an aqueous medium to form a W/O/W emulsion; and
d) allowing the organic phase to evaporate and thereby obtain particulate carriers comprising a hollow lumen containing said biologically active agent in an aqueous phase.

Allowing the organic phase to evaporate may be passive or active. For example active evaporation may be by the use of heat.

In one embodiment the particulate carriers may be microspheres or nanoparticles. In a further embodiment the particulate carrier is a nanoparticle and the biologically active agent is a protein. In a further embodiment the particulate carrier is a nanoparticle and the biologically active agent is a peptide. In yet another embodiment the particulate carrier is a nanoparticle and the biologically active agent is an antigen binding molecule, for example a mAb or a dAb, or a combination thereof. In yet another embodiment the particulate carrier is a nanoparticle and the biologically active agent comprises a domain.

In a further embodiment the particulate carrier is a microsphere and the biologically active agent is a protein. In a further embodiment the particulate carrier is a microsphere and the biologically active agent is a peptide. In yet another embodiment the particulate carrier is a microsphere and the biologically active agent is an antigen binding molecule, for example a mAb or a dAb, or a combination thereof. In yet another embodiment the particulate carrier is a microsphere and the biologically active agent comprises a domain.

In one embodiment the w/w ratio of protein to polymer may be 0.5% to 50% for example is at least about 0.5% or is at least about 1% or is at least about 2% or is at least about 5% or is at least about 7% or is at least about 10% or is at least about 11% or is at least about 14% or is at least about 20% or is at least about 40%, or is at least about 50%. For example when the protein is a peptide the peptide to polymer ratio may be at least about 11, or when the protein is an antibody the antibody to polymer ratio may be at least about 14%, or when the protein is a domain antibody the domain antibody to polymer ratio may be at least about 11 %.

In one embodiment of the present invention the encapsulation efficiency of the particles is at least about 1% or is at least about 2% or is at least about 10% or is at least about 20% or is at least about 40% or is at least about 50% or is at least about 60% or is at least about 70% or is at least about 80% or is at least about 90% or is alt least about 95% or is least about 97% or is at least about 99%. For example when the protein is a peptide the encapsulation efficiency may be at least about 60%, when the protein is an antibody the encapsulation efficiency may be at least about 90%, or when the protein is a domain antibody the encapsulation efficiency may be at least about 60%.

In a further embodiment of the method, step (d) additionally comprises the addition of gel forming polymers. In a further embodiment the gel forming polymer is agarose.

Examples of organic solvents suitable for use with the methods of the invention include but are not limited to water-immiscible esters such as ethyl acetate, isopropyl acetate, n-propyl acetate, isobutyl acetate, n-butyl acetate, isobutyl isobutyrate, 2-ethylhexyl acetate, ethylene glycol diacetate; water-immiscible ketones such as methyl ethyl ketone, methyl isobutyl ketone, methyl isoamyl ketone, methyl n-amyl ketone, diisobutyl ketone; water-immiscible aldehydes such as acetaldehyde, n-butyraldehyde, crotonaldehyde, 2-ethylhexaldehyde, isobutylaldehyde and propionaldehyde; water-immiscible ether esters such as ethyl 3-ethoxypropionate; water-immiscible aromatic hydrocarbons such as toluene xylene and benzene; water-immiscible halohydrocarbons such as 1,1,1 trichloroethane; water-immiscible glycol ether esters such as propylene glycol monomethyl ether acetate, ethylene glycol monoethyl ether acetate, ethylene glycol monobutyl ether acetate, diethylene glycol monobutyl ether acetate; water-immiscible phthalate plasticisers such as dibutyl phthalate, diethyl phthalate, dimethyl phthalate, dioctyl phthalate, dioctyl terephthalate, butyl octyl phthalate, butyl benzyl phthalate, alkyl benzyl phthalate; water-immiscible plasticisers such as dioctyl adipate, triethylene glycol di-2-ethylhexanoate, trioctyl trimellitate, glyceryl triacetate, glyceryl/tripropionin, 2,2,4-trimethyl-1,3-pentanediol diisobutyrate, methylene chloride, ethylacetate or dimethylsulfoxide, carbon tetrachloride, chloroform, cyclohexane, 1,2-dichloroethane, dichloromethane, diethyl ether, dimethyl formamide, heptane, hexane and other hydrocarbons, methyl-tert-butyl ether, pentane, toluene, 2,2,4-trimethylpentane, 1-octanol and its isomers or benzyl alcohol.

In one embodiment of the invention the solvent used in the methods of the invention will be selected from methylene chloride, ethylacetate or dimethylsulfoxide, carbon tetrachloride, chloroform, cyclohexane, 1,2-dichloroethane, dichloromethane, diethyl ether, dimethyl formamide, heptane, hexane and other hydrocarbons, methyl-tert-butyl ether, pentane, toluene, 2,2,4-trimethylpentane, 1-octanol and its isomers, benzyl alcohol.

The particulate carriers, compositions comprising them or methods of making them in all aspects of the present invention as herein described may further comprise the addition of a surfactant such as but not limited to: sodium cholate, poloxamer 188 (pluronic F68™, or F127), polyvinyl alcohol, polyvinyl pyrrolidone, polysorbate 80, dextrans. poloxamers, poloxamines, carboxylic acid esters of multifunctional alcohols, alkoxylated ethers, alkoxylated esters, alkoxylated mono-, di and triglycerides, alkoxylated phenols and diphenols, ethoxylated ethers, ethoxylated esters, ethoxylated triglycerides, substances of the GenapolR™ and BaukiR™ series, metal salts of fatty acids, metal salts of carboxylic acids, metal salts of alcohol sulfates, and metal salts of fatty alcohol sulfates and metal salts of sulfosuccinates and mixtures of two or more of said substances.

In a further embodiment the surfactant is selected from sodium cholate, poloxamer 188 (pluronic F68™), polyvinyl alcohol, polyvinyl pyrrolidone, polysorbate 80 and dextrans.

In one embodiment of the present invention there is provided particulate carriers comprising biologically active agents, obtainable by any of the methods of the invention herein described.

The biologically active agent encapsulated in particulate carriers and or compositions of the present invention retains at least some biological activity on its release from the particulate carrier, for example, a proportion of the molecules in the composition may retain at least some ability to bind to their target when the agent is a binding agent and elicit a biological response on the release of the biologically active agent from the particles. Such binding can be measured in a suitable biological binding assay, examples of suitable assays include but are not limited to ELISA or Biacore™. In a further embodiment the composition retains at least 50% of its affinity for the target, or at least 70% or at least 90% of its affinity (Kd) for the target when measured by a biological binding assay on release from the particles for example in one embodiment as determined by ELISA, Biacore. In one embodiment the composition will be capable of eliciting a therapeutic effect in the subject to which it is administered. The biological activity of the compositions of the invention can be measured by any suitable assay which measures activity of the encapsulated biologically active molecule, for example where the biologically active molecule is a VEGF dAb, the assay described in Example 18 can be used.

In one embodiment of the present invention there is provided a method of delivering a protein across a biological barrier such as the blood brain barrier by encapsulation of the protein in a nanoparticle to a patient. In a further embodiment the patient is human.

In one embodiment of the present invention there is provided a method of delivering a protein encapsulated in a particulate carrier such as a microsphere to the eye of a mammal, for example a human.

In another embodiment there is provided a pharmaceutical composition comprising a biologically active agent encapsulated in a particulate carrier of the present invention as herein described.

In a further embodiment there is provided a pharmaceutical composition comprising a protein encapsulated in the nanoparticles of the present invention as herein described. In a further embodiment there is provided a pharmaceutical composition comprising a protein encapsulated in microspheres for ocular delivery as herein described for treating and or preventing a disease of the eye.

In a further embodiment a composition of the invention as herein described may be used to treat and or prevent disorders or diseases of the Central nervous system, for example it may be used to treat and or prevent Alzheimer's disease, Huntington's disease, bovine spongiform encephalopathy, West Nile virus encephalitis, Neuro-AIDS, brain injury, spinal cord injury, metastatic cancer of the brain, or multiple sclerosis, stroke.

In a further embodiment the composition may comprise an anti-MAG antibody for the treatment and or prevention of stroke or neuronal injury.

In another embodiment the composition may comprise an anti-NOGO antibody for the treatment and or prevention of stroke or neuronal injury or for example for the treatment or prophylaxis of neurodegenerative diseases such as Alzheimer's disease.

In another embodiment the composition may comprise an anti-ßamyloid antibody for the treatment and or prevention of stroke or neuronal injury or for example for the treatment or prophylaxis of neurodegenerative diseases such as Alzheimer's disease.

In one embodiment of the invention as herein described the particulate carriers may be administered to the patient by parenteral injection or infusion, intravenous, or intraarterial administration.

In a further embodiment the compositions of the invention as herein described may be used to treat and or prevent disorders or diseases of the eye. In a further embodiment a composition of the invention as herein described may be used to treat and or prevent disorders such as but not limited to age related macular degeneration (neovascular/wet), diabetic retinopathy, retinal venous occlusive disease, uveitis, corneal neovascularisation or glaucoma.

In yet a further embodiment the composition is used to treat and or prevent AMD (age related macular degeneration), for example wet AMD, or dry AMD.

In another embodiment of the present invention there is provided biologically active agents encapsulated in nanoparticles and or microspheres as described herein for use in medicine.

In one embodiment of the present invention there is provided the use of compositions of the invention as described herein in the manufacture of a medicament for the treatment and or prevention of a disease of the central nervous system. In yet another embodiment there is provided the use of a composition of the invention as described herein in the manufacture of a medicament for the treatment and or prevention of Alzheimer's disease. In yet a further embodiment there is provided the use of a composition of the invention as described herein in the manufacture of a medicament for the treatment and or prevention of stroke or neuronal injury.

In another embodiment of the invention there is provided the use of a composition of the invention as described herein in the manufacture of a medicament for the treatment or prevention of ocular diseases such as for example in the manufacture of a medicament for the treatment and or prevention of AMD.

The invention provides methods of treating and or preventing a disease of the central nervous system using a composition of the present invention. In a further embodiment there is provided a method of treating Alzheimer's disease using a composition of the present invention. In yet another embodiment of the present invention there is provided a method of treating and or preventing stroke or neuronal injury using a composition of the present invention.

The invention also provides methods of treating and or preventing ocular disease using a composition of the present invention. In a further embodiment there is provided a method of treating and or preventing AMD using a composition of the present invention.

**Definitions:**

As used herein the term "particle forming substance" is used to describe any monomer and or oligomer capable of polymerising, or a polymer which can form an insoluble particle in an aqueous environment for example PBCA, PLGA. The particle forming substance will be soluble in an organic solvent when not polymerised.

The term "particulate carrier" as used throughout this specification is used to cover both nanoparticles and microspheres. "Microspheres" are particles composed of various natural and synthetic materials with diameters larger than 1 μm whereas "nanoparticles" as used herein are submicron sized particles such as for example 1-1000nm.
In one embodiment the terms particulate carrier, nanoparticles and microspheres as used herein denotes a carrier structure which is biocompatible and sufficiently resistant to chemical and/or physical destruction by the environment of use such that a sufficient amount of the particles remain substantially intact after entry in to the human or animal body following administration and for sufficient time so as to be able to reach the desired target organ or tissue e.g. the brain or the eye.

The term "Biologically active agent" as used herein is a term used to indicate that the molecule must be capable of at least some biological activity when reaching their desired target. For the avoidance of doubt the term "Biologically active agent" and the "biologically active molecule" as used throughout the specification are intended as to have the same meaning and able to be used interchangeably.

The term "solubilisation" is defined as either formation of a solution, in the form of individual molecules in the solvent, or formation of a solid in liquid suspension, in the form of fine solid aggregates of molecules suspended in the liquid. The solubilisation process may also result in a mixture of fully dissolved molecules and suspended solid aggregates.

The term "protein" as used throughout this specification for encapsulation in particulate carriers includes proteins having a molecular weight of at least 11 kDa, or at least 12kDa, or at least 50kDa, or at least 100kDa, or at least 150kDa or at least 200kDa. Proteins for encapsulation may also be of considerable length such as at least 70 amino acids in length or at least 100 amino acids in length or at least 150 amino acids in length or at least 200 amino acids in length.

The term "peptide" as used throughout this specification for encapsulation in particulate carriers includes shorter sequences of amino acids having a molecular weight of no more than about 10 kDa, or no more than about 8 kDa, or no more than about 5 kDa, or no more than about 2 kDa or no more than about 1 kDa or is less than 1 Kda. Peptides for encapsulation are no more than 70 amino acids in length or are no more than 50 amino acids in length, or are no more than are no more than 40 amino acids in length, or are no more than 20 amino acids in length or are less than 10 amino acids in length.

The term "Peri-ocular" refers to local administration to positions surrounding the outside of the eye and includes but is not limited to:

"Sub-conjuctival" - underneath the conjuctiva - a clear mucus membrane that covers the eyeball over the sclera; "Sub-tenon" - underneath the Tenon's membrane that envelopes the eye but outside of the sclera; "peribulbar" - the space underneath the globe of the eye where it sits in the eye socket; "retrobulbar" - the space at the very back of the globe of the eye, close to the optic nerve; "supra-choroidal" - underneath the sclera but outside of the choroid into the supra-choroidal space; "trans-scleral" - this term can also be used to mean delivery across, i.e. from outside of the sclera.

The phrase "immunoglobulin single variable domain" refers to an antibody variable domain ($V_H$, $V_{HH}$, $V_L$) that specifically binds an antigen or epitope independently of a different V region or domain. An immunoglobulin single variable domain can be present in a format (e.g., homo- or hetero-multimer) with other, different variable regions or variable domains where the other regions or domains are not required for antigen binding by the single immunoglobulin variable domain (i.e., where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004, nurse shark and Camelid $V_{HH}$ dAbs. Camelid $V_{HH}$ are immunoglobulin single variable domain polypeptides that are derived from species including camel, llama, alpaca, dromedary, and guanaco, which produce heavy chain antibodies naturally devoid of light chains. Such $V_{HH}$ domains may be humanised according to standard techniques available in the art, and such domains are still considered to be "domain antibodies" according to the invention. As used herein "$V_H$ includes camelid $V_{HH}$ domains.

The term "antigen binding molecule" as used herein refers to antibodies, antibody fragments and other protein constructs which are capable of binding to a target.

A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. A "single antibody variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The term "Light scattering techniques" as used herein is a means used to determine the size distribution profile of small particles in solution - such as for example dynamic light scattering which is used to measure nanoparticles and static light scattering or low angle light scattering used to measure microspheres.

The term "Dynamic light scattering" (DLS) as used herein is a method which utilises the light scattered by particle dispersions to derive information on the size of the particles. Dynamic light scattering relies on the fact that when in liquid suspension, the Brownian motion of particles is dependent on particle size and that the Brownian motion of the particles produces fluctuations in the intensity of light scattered from a particle sample. The particle diameter is derived by analysing these fluctuations by means of a correlation function. The Stokes-Einstein equation is then applied to yield the mean hydrodynamic diameter of the particles.

A multi-exponential analysis can produce a size distribution, providing insight into the presence of different species inside a sample. DLS is generally accepted for the analysis of nanoparticles.

"Static light scattering" or "Low angle laser light scattering" as used throughout the specification is sometimes referred to as Laser diffraction. Laser diffraction relies on the fact that the diffraction angle is inversely proportional to particle size. The method utilises the full Mie theory which completely solves the equations for the interaction of light with matter. Laser diffraction can be used for the analysis of nanoparticles and microparticles (0.02 to 2000 micrometers in diameter).

The term "Blood brain barrier" (BBB) as used herein is a membranic structure that acts primarily to protect the brain from chemicals in the blood, while still allowing essential metabolic function. It is composed of cerebral microvascular endothelial cells, which are packed very tightly in brain capillaries. This higher density restricts passage of substances from the bloodstream much more than endothelial cells in capillaries elsewhere in the body.

Throughout this specification the percentage drug loading is defined as the percentage of weight of drug per weight of material used in the particle formulation (polymer weight) w/w.

$$\%\ \text{drug loading} = (\text{weight of drug} / \text{weight of material used in the particle formulation}) \times 100\%.$$

Within this specification the invention has been described, with reference to embodiments, in a way which enables a clear and concise specification to be written. It is intended and should be appreciated that embodiments may be variously combined or separated without parting from the invention.

## Examples

Example 1 - Polymerisation of BCA (butylcyanoacrylate) monomer:

A rapid polymerisation reaction in organic solvent was used to form the polymer:

BCA monomer (200 µl, Vetbond, 3M) was added to 1 ml absolute ethanol in a 25 ml beaker with slow swirling. The resulting solution was gently mixed until the polymerisation reaction was initiated. The polymerisation reaction resulted in the formation of a white solid dispersion. The mixing of the dispersion was stopped as soon as the reaction mixture became too viscous to agitate.

The ethanol in the reaction mixture was then allowed to evaporate in the fume-hood for at least 1 hour. Following evaporation of the ethanol, a cracked white solid cake was obtained. The solid was collected and used in the nanoparticle preparation process.

Example 2 - Preparation of hollow nanoparticles by the double emulsion method:

The PBCA polymer was dissolved in dichloromethane at a concentration of 1% w/v and used to prepare hollow PBCA nanoparticles by emulsification into a double emulsion (water in oil in water, w/o/w) as follows:

(i) Primary emulsion (w/o)

Inner phase (w): 5% sodium cholate (SIGMA) in water or buffer, prepared by mixing:

500 μl water or buffer; and

500 μl sodium cholate (10% w/v stock solution).

The total volume of the inner aqueous phase was 1 ml. The solution was kept on ice until it was time to use it. Each solution was drawn into a 1 ml insulin syringe (Terumo 1ml, BD microlance needle 19G 1.5") prior to use.
Outer (organic) phase (o): PBCA polymer (1 % w/v) in dichloromethane ("DCM, Fischer).
The organic phase (PBCA polymer in DCM, 6 ml) was poured into a 10ml beaker (resting on ice to keep cool) and the probe of the homogeniser was inserted (Ultra-Turrax, T25, 50ml probe). The solution was covered with parafilm (attached to the beaker and probe) and homogenised at a speed of 24,000 rpm using a rotor stator homogeniser (Ulltra-Turrax T25 basic).

Formation of a primary emulsion:

As soon as the homogeniser reached the required speed, the inner aqueous phase was added by injecting inside the solution close to the probe. The resulting emulsion was homogenised for 2 minutes (on ice) and then transferred to a glass syringe (SGE, 25 ml, gas-tight, suitable for organic solvents, P/N 009462 25MDR-LL-GT, Batch # F06-A2190, fitted with a blunt 5 cm 2R2 needle, 0.7 mm ID).

(ii) Secondary emulsion (w/o/w)

Inner phase (w/o): primary emulsion from homogenisation step described above.
Outer phase (w): sodium cholate (1.25% w/v) in water.

Formation of the secondary emulsion:

The primary single emulsion (w/o) was used to form a double emulsion (w/o/w) by addition to a secondary aqueous phase (1.25% w/v sodium cholate) with homogenisation. The sodium cholate solution (1.25 % w/v, 30 ml) was transferred to a tall 50 ml beaker (resting on ice to keep emulsion cool) and the probe of a Silverson L4RT homogeniser was inserted (3/4 inch probe, high emulsor screen). The solution was covered with parafilm (attached to the beaker and probe) and homogenised at a speed of 8,000 rpm. The primary emulsion was injected into the solution close to the probe as soon as the 8,000 rpm speed was reached. The resulting emulsion was homogenised for 6 minutes.
The double emulsion that was formed was transferred to a short 50 ml beaker and the organic phase allowed to evaporate in the fume hood under constant stirring (IKA magnetic stirrer, setting 4) for 3 hours.

Washing of the nanoparticles by centrifugation:

Following removal of the organic solvent, the nanoparticles that were formed were washed once by centrifugation at 16,200 rcf and re-suspended in water (10 ml).

Example 3 - Confirmation of nanoparticle formation by dynamic light scattering

The formation of nanoparticles was confirmed by sizing using quasi-elastic light scattering (QELS), also known as dynamic light scattering (DLS). The particles were analysed using a Brookhaven Instruments corporation particle size analyser (BIC 90 plus) following the standard procedure provided by the manufacturer. The particle suspension was diluted 200x in water and sized using standard sizing parameters (temperature of 25°C, laser beam angle of 90°, laser wavelength of 658 nm). The particles were analysed by performing 10 sizing runs of 1 minute in duration each.

The instrument presented the raw data in the standard form of a correlogram. This depicts the autocorrelation function C (i) of scattered light intensity from the particles at different time intervals and how the autocorrelation decays with the decay timer. The decay in the autocorrelation of scattered light is dependent on particle diameter and is more rapid for smaller particles. The instrument derives information on particle size by applying the Stokes-Einstein equation. This yields the mean hydrodynamic diameter of the particles in the sample and further derived data on the particle population.

Dynamic light scattering is very sensitive to the presence of large particles, which even when they represent less than 1% of the sample can significantly influence the measurements. As a result, the mean hydrodynamic diameter that the instrument gives, which is heavily influenced by the large particles in the sample could vary substantially. As a result, differences in size of tens of nanometers between batches can be observed and for this reason it is important to look at the complete data set that the instrument provides, with the correlogram being the most important.
By looking at the complete data set, it is possible to accurately characterise a sample as the instrument can easily detect the small particle majority that is present in the sample despite the presence of few large particles. The shape of the correlogram itself provides a very clear indication of whether the particles are small, as well as whether the sample is polydisperse. The baseline index also gives an accurate representation of the quality of the data. All of the data in this document exhibited a baseline index that did not fall below 5, with 10 being the maximum for the highest possible quality of a reading.

Figure 1 a shows the correlogram (raw data) obtained following sizing of the particle suspension by QELS. The correlogram clearly showed that a nanoparticle suspension had been generated by the particle preparation process, as absence of particles would not generate any light scattering. The shape of the correlogram suggested that the suspension was of good pharmaceutical quality, as the particles were small and no large aggregates were present. Sizing of the nanoparticle suspension by QELS showed that nanoparticles of a mean hydrodynamic diameter of 262.6 nm had formed (figure 1a). The particle population was also found to be relatively monodisperse, with the polydispersity index, which is a measure of how broad the range of particle sizes in the sample is, at 0.262 (figure 1a). This is below the maximum acceptable value of 0.300 for a particle formulation. In general, the correlogram confirmed that the double emulsion process had successfully generated a good quality suspension of PBCA nanoparticles.

The derived data (generated by the instrument using the Stokes Einstein equation) suggest that the majority of the particles were small (Figures 1 b-d). The results suggest that approximately 87.5 % of the particle population had a diameter of 138.19 nm or lower (figure 1 b). It was found that the suspension was free of large aggregates and did not contain any particles that exceeded 506.81 nm in diameter, with the majority of the particle population being significantly smaller (figure 1c). In addition, the formulation did not contain any particles that were smaller than 99.86 nm (figure 1d). Therefore, the majority of the particles were of a diameter between 99.86 and 138.19 nm, a size that is ideal for intravenous administration but not too small so that drug loading is compromised.

Figure 1. - Sizing data obtained by QELS that indicate the presence of nanoparticles in suspension.

Fig. 1(a) Correlogram obtained following analysis of a nanoparticle suspension by dynamic light scattering. According to the data obtained, the mean hydrodynamic diameter of the particles was 262.6 nm and the polydispersity index 0.262.

Fig 1 (b) Multimodal size distribution (derived data) of the nanoparticles plotted to depict the distribution of the particle population (number) over a range of sizes. The majority (87.5 %) of the particle population appeared to have a diameter of 138.19 nm or lower.

Fig. 1(c) Multimodal size distribution (derived data) of the nanoparticles plotted to depict the distribution of the particle population (number) over a range of sizes. The data suggests that 87.5% possessed a diameter of 138.19nm or lower and that 100% of the particle sample possessed a diameter of 506.81 nm or lower. Therefore, the suspension was free of large aggregates and was therefore considered to be suitable for intravenous administration.

Fig 1(d) Multimodal size distribution (derived data) of the nanoparticles plotted to depict the distribution of the particle population (number) over a range of sizes. The data suggest that 14.9% of the particle sample possesses a diameter of 99.86 nm or lower.

The process was found to yield similar nanoparticle sizes when different nanoparticle formulations were prepared. Table 1 summarises the sizing data obtained from a series of four different formulation runs:

Table 1

| Formulation | Mean hydrodynamic diameter (nm) | Polydispersity index |
|---|---|---|
| 1 | 259.6 | 0.164 |
| 2 | 437.6 | 0.281 |
| 3 | 319.2 | 0.303 |
| 4 | 320.6 | 0.248 |
| Average | 334.25 | 0.249 |

In general, the hollow PBCA nanoparticle preparation process was found to generate nanoparticle suspensions that were of the desired diameter and polydispersity.

Example 4 - Analysis of nanoparticles - confirmation of nanoparticle formation and hollow morphology by electron microscopy

In order to confirm that the particles were formed and that they were hollow, samples were visualised by electron microscopy. Nanoparticle suspensions were examined by transmission electron microscopy (TEM). Freeze-dried nanoparticles were analysed by scanning electron microscopy (SEM). Analysis by both microscopy techniques confirmed the formation of nanoparticles. SEM showed that stable nanoparticles were formed. TEM confirmed that the nanoparticles were hollow, possessing an aqueous core surrounded by a PBCA polymer wall.

Figure 2 shows nanoparticles analysed by SEM

Figure 3 shows an image of hollow nanoparticles by TEM, with a superimposed image of solid PBCA nanoparticles for comparison.

Example - 5 - Encapsulation of monoclonal antibody (human anti-CD23) within the hollow PBCA nanoparticles

Monoclonal antibody (human anti-CD 23 mAb as disclosed in WO99/58679) was entrapped within the aqueous core of the nanoparticles by inclusion into the inner aqueous phase of the homogenisation process. A solution of the antibody was used to prepare the primary emulsion (w/o), which was then homogenised with the secondary aqueous phase to form the double emulsion (w/o/w) as follows:

(iii) Primary emulsion (w/o)

Inner phase (w): anti-CD23 mAb as disclosed in WO99/58679 (600 μg in 5% sodium cholate (SIGMA), prepared by mixing:

78 μl mAb solution (7.2 mg/ml)

344 μl H2O

500 μl sodium cholate solution (10% w/v stock solution)

The total volume of the inner aqueous phase was 1 ml. The solution was kept on ice until it was time to use it. Each solution was drawn into a 1 ml insulin syringe (Terumo 1ml, BD microlance needle 19G 1.5") prior to use.

Outer phase (o): PBCA polymer (1 % w/v) in dichloromethane (Fischer).

The organic phase (PBCA polymer in DCM, 6 ml) was poured into a 10ml beaker (resting on ice to keep cool) and the probe of the homogeniser was inserted (Ultra-Turrax, T25, 50ml probe). The solution was covered with parafilm (attached to the beaker and probe) and homogenised at a speed of 24,000 rpm.

Formation of a primary emulsion:

As soon as the homogeniser reached the top speed, the inner aqueous phase was added by injecting inside the solution close to the probe. The resulting emulsion was homogenised for 2 minutes (on ice) and then transferred to a glass syringe (SGE, 25 ml, gas-tight, suitable for organic solvents, P/N 009462 25MDR-LL-GT, Batch # F06-A2190, fitted with blunt 5 cm 2R2 needle, 0.7 mm ID).

(iv) Secondary emulsion (w/o/w)

Inner phase (w/o): primary emulsion from homogenisation step described above.
Outer phase (w) : sodium cholate (1.25% w/v) in water.

Formation of the secondary emulsion :

The primary, single emulsion (w/o) was used to form a double emulsion (w/o/w) by addition to a secondary aqueous phase (1.25% w/v sodium cholate) with homogenisation. The sodium cholate solution (1.25 % w/v, 30 ml) was transferred to a tall 50 ml beaker (resting on ice to keep emulsion cool) and the probe of a Silverson L4RT homogeniser was inserted (3/4 inch probe, high emulsor screen). The solution was covered with parafilm (attached to the beaker and probe) and homogenised at a speed of 8,000 rpm. The primary emulsion was injected into the solution close to the probe as soon as the 8,000 rpm speed was reached. The resulting emulsion was homogenised for 6 minutes.
The double emulsion that was formed was transferred to a short 50 ml beaker and the organic phase allowed to evaporate in the fume hood under constant stirring (IKA magnetic stirrer, setting 4) for 3 hours.

Washing of the nanoparticles by centrifugation:

The resulting nanoparticles were pelleted by centrifugation to separate free from encapsulated antibody. Both pellet (entrapped antibody) and supernatant (free antibody) were analysed by total protein assay in order to determine the encapsulation efficiency.
The encapsulation efficiency was found to be 52%, when a total amount of 600 μg antibody was used. The efficiency of encapsulation was found to be sufficiently high to permit the delivery of potentially therapeutic amounts of antibody without exceeding the maximum tolerated dose of PBCA polymer (50 mg/kg in the mouse). Moreover, it was later possible to prepare particles containing a different monoclonal antibody, human anti-IL13, which suggests that the method is applicable to any water-soluble biopharmaceutical.
Figure 4 show the results obtained from the encapsulation efficiency measurements.

Example 6 - Release of monoclonal antibody from the nanoparticles

In addition to achieving efficient encapsulation of a biopharmaceutical, it was necessary to demonstrate that the material could be released from the particles following administration and that it retained its activity. The release of active antibody from the particles was initially investigated in vitro by degradation of the particles followed by detection of any released antibody by ELISA. In order to release the encapsulated antibody, particles were treated with a butyl esterase (from porcine liver, SIGMA), which has been reported to cleave the butyl ester of the PBCA polymer. During the reaction (Ringers solution, pH 7.0, 37°C), samples were taken at different time points (0, 1, 2, 3, 4 and 24h) and analysed for the presence of active antibody by ELISA.
Figure 5 shows the release profile obtained following enzymatic degradation of the particles and analysis of the released enzyme by ELISA.

Example 7 -Encapsulation of domain antibody (anti-hen egg lysozyme dAb) within the hollow PBCA nanoparticles

In the following examples the BCA assay was performed using a BCA kit obtained from Sigma (QPBCA) and carried out according to the instructions. Free dAb was diluted 2 fold and 10 fold for analysis. The encapsulated dAb was diluted 100 fold.
Domain antibody (anti-hen egg lysozyme dAb) was entrapped within the aqueous core of the nanoparticles by inclusion into the inner aqueous phase of the homogenisation process. In this case, the inner aqueous phase was prepared by mixing 0.5 ml of a 20 mg/ml solution of dAb (10 mg protein) and 0.5 ml of a stabiliser solution (sodium cholate, 10% w/v). The nanoparticles were then prepared by the double emulsion process as described in example 4. The resulting nanoparticles were pelleted by centrifugation to separate free from encapsulated antibody. Both pellet (entrapped antibody) and supernatant (free antibody) were analysed by total protein assay (bicinchoninic acid assay) in order to determine the encapsulation efficiency. The results of the analysis are shown in figure 6. The amount of encapsulated dAb was found to be 6.66 mg. The amount of free dAb was 4.83 mg. The efficiency of encapsulation was therefore around 66.6%, with the loading efficiency at 11.1 %. It was therefore possible to efficiently encapsulate milligram amounts of protein in the hollow PBCA nanoparticles using the double emulsion method.

Example 8 - Preparation of PBCA nanoparticles by the HIP process

The nanoparticles were prepared by adding 100 μl BCA monomer to an organic phase containing solubilised HIP ion (docusate sodium, 3.058-6.116 % w/v in 1 ml dichloromethane). The resulting solution was pipetted into an aqueous phase (1% w/v dextran, 0.2 % w/v pluronic F68, 10 ml, pH 7.0) with homogenisation at 7,000 using a Silverson L4RT homogeniser. Exposure to the neutral pH of the aqueous phase resulted in rapid polymerisation of the BCA monomer to form PBCA polymer. The emulsion that was formed was homogenised for 45 seconds and then incubated in a fume hood for 3 hours to allow the organic solvent to evaporate and nanoparticles to form. The resulting nanoparticle suspension was stored at 4°C.

Example 9 - Confirmation of nanoparticle formation by dynamic light scattering

The formation of PBCA nanoparticles by the HIP process was confirmed by sizing using dynamic light scattering (DLS). The particles were analysed using a Brookhaven Instruments corporation particle size analyser (BIC 90 plus). Figure 7 shows the sizing data obtained by DLS that indicate the presence of nanoparticles in suspension. Sizing by DLS showed that nanoparticles of a mean hydrodynamic diameter of 291.4 nm had formed (figure 7a). The particle population was also found to be relatively monodisperse, with the polydispersity index, which is a measure of how broad the range of particle sizes in the sample is, at 0.242 (figure 7a). This is below the maximum acceptable value of 0.300 for a particle formulation. In general, the correlogram confirmed that the particle preparation process had successfully generated a good quality suspension of PBCA nanoparticles.

The derived data suggest that the majority of the particles are small (Figures 7b-d). The results suggest that approximately 96.3 % of the particle population had a diameter of 201.37 nm or lower (figure 7b). The suspension also appeared to be generally free of large aggregates and did not contain any particles that exceeded 732.05 nm in diameter, with the majority of the particle population being significantly smaller (figure 7c). The formulation also did not seem to contain any particles that were smaller than 143.38 nm (figure 7d). Therefore, the majority of the particles were of a diameter between 143.38 and 201.37 nm, a size that is safe for intravenous administration but not too small so that it reduces drug loading efficiency.

Fig 7(a) - Correlogram obtained following analysis of a nanoparticle suspension by dynamic light scattering. According to the data obtained, the mean hydrodynamic diameter of the particles was 291.4 nm and the polydispersity index 0.242.

Fig 7(b) - Multimodal size distribution (derived data) of the nanoparticles plotted to depict the distribution of the particle population (number) over a range of sizes. The data suggest that 96.3% of the particle population appeared to have a diameter of 201.37 nm or lower.

Fig 7(c) - Multimodal size distribution (derived data) of the nanoparticles plotted to depict the distribution of the particle population (number) over a range of sizes. The data suggest that 96.3% of the particle population appeared to have a diameter of 201.37nm or lower and that 100% of the particle sample possessed a diameter of 732.05 nm or lower. Therefore, the suspension was found to be free of large aggregates and was therefore considered to be safe for intravenous administration.

Fig 7(d) - Multimodal size distribution (derived data) of the nanoparticles plotted to depict the distribution of the particle population (number) over a range of sizes. The data suggest that 6.2% of the particle sample possesses a diameter of 143.38 nm or lower.

The process was found to yield similar nanoparticle sizes when different nanoparticle formulations were prepared. Table 2 summarises the sizing data obtained from a series of six different formulations of varying compositions:

Table 2

| Formulation | Mean hydrodynamic diameter (nm) | Polydispersity index 10 |
|---|---|---|
| 1 | 367.7 | 0.280 |
| 2 | 291.4 | 0.242 |
| 3 | 236.3 | 0.259 |
| 4 | 244.7 | 0.256 |
| 5 | 255.4 | 0.213 |
| 6 | 269.8 | 0.221 |
| Average | 294.22 | 0.245 |

In general, the HIP process was found to generate nanoparticle suspensions that were of the desired diameter and polydispersity.

Example 10- Solubilisation of peptides into the organic phase using the HIP process and encapsulation into PBCA nanoparticles

A solution of the hexapeptide dalargin was prepared by dissolving 30-60 mg of the peptide into 3 ml of CaCl2 (18.3 mM) and lowering the pH to 3.05 by addition of concentrated HCl (2M). The resulting solution (500 μl, 10-20 mg/ml, and total amount of peptide 5-10 mg) was added to a solution of the HIP agent docusate sodium in dichloromethane (1ml, 3.058-6.116 % w/v) in a 2 ml eppendorf tube. The volume of HIP solution used was twice that of the peptide solution (1 ml HIP solution for 500 μl peptide solution). The molar ratio of HIP: peptide was 10:1 with 5 mg peptide and 5:1 with 10 mg peptide. The organic and aqueous phases were mixed by vortexing at maximum speed for 1 minute. The resulting suspension was then centrifuged to separate the two phases at 20,817 rcf for 50 minutes. The organic layer (containing solubilised peptide) was collected and used to prepare nanoparticles.
In order to confirm that the process had been successful at solubilising the peptide into the organic phase, the amount of peptide remaining in the aqueous phase was determined. Analysis by LC-MS and Edman sequencing showed that at least 99% of the peptide had been successfully extracted into the organic phase.

Example 11 - Encapsulation of peptide within the PBCA nanoparticles

The nanoparticles were prepared by adding 100 μl BCA monomer to the organic phase containing solubilised peptide and HIP (1 ml). The resulting solution was pipetted into an aqueous phase (1% w/v dextran, 0.2 % w/v pluronic F68, 10 ml, pH 7.0) with homogenisation at 7,500 using a Silverson L4RT homogeniser (fine emulsor screen, ¾ inch probe). Exposure to the neutral pH of the aqueous phase resulted in rapid polymerisation of the BCA monomer to form PBCA polymer. The emulsion that was formed was homogenised for 45 seconds and then incubated in a fume hood with stirring (IKA magnetic stirrer, speed setting 4) for 1 hour in order to evaporate the organic phase. The speed setting was then lowered to 3 and the formulations incubated for a further 2 hours to ensure evaporation of the organic phase and nanoparticle formation. The nanoparticle suspensions were collected and stored at 4 degrees C.
The resulting nanoparticles were centrifuged to remove any free peptide and re-suspended in water or PBS.
The encapsulation efficiency was determined by analysing the particles by LC-MS. It was found that approximately 90% of the peptide dose was encapsulated, even when high peptide amounts were used (10mg). Comparison of the amounts of encapsulated peptide achieved with the HIP to those achieved by the common method of adsorption onto the particle surface clearly demonstrated the superiority of the HIP-PBCA process (figure 8). When the adsorption method was used, a mere 1.5% of the peptide dose was loaded onto the particles. The analysis of the nanoparticles loaded with dalargin by the adsorption method was performed at a different time. The Kreuter adsorbed particles were made and analysed prior to the development of the current HIP method as a means aimed at evaluating the prior art. The LC/MS method and HPLC method that were used are equally as sensitive.

Example 12 - Evaluation of the HIP-PBCA nanoparticle delivery system in vivo

(mouse model)

The ability of the HIP-PBCA nanoparticles to deliver their peptide load to the brain was determined in vivo in the mouse model. HIP-PBCA nanoparticles containing encapsulated dalargin using the HIP process were compared to HIP-PBCA nanoparticles that had had the peptide adsorbed onto the particle surface as reported by Kreuter et al. The nanoparticles were prepared for brain delivery via the intravenous route by coating their surface with polysorbate 80 surfactant. Briefly, the nanoparticles were incubated in PBS containing 1% w/v surfactant for 30 minutes prior to injection. The surfactant has been reported in the literature to indirectly target the nanoparticles to the brain by promoting adsorption of serum apolipoproteins onto the nanoparticle surface. This allows the particles to bind to the apolipoprotein receptor on the blood brain barrier and transcytose to reach the brain. The following formulations were compared:

1. HIP-PBCA nanoparticles alone (5:1 HIP content)
2. HIP-PBCA nanoparticles alone (10:1 HIP content)
3. Dalargin in solution (2.0 mg/kg)
4. HIP-PBCA nanoparticles with dalargin adsorbed onto the surface (2.0 mg/kg total dose used in formulation)
5. HIP-PBCA nanoparticles (5:1 HIP:dalargin molar ratio) with dalargin encapsulated (2.0 mg/kg total dose used in formulation)
6. HIP-PBCA nanoparticles (5:1 HIP:dalargin molar ratio) with dalargin encapsulated (2.0 mg/kg total dose used in formulation) - the same formulation as above, but injected at 1/10 of the dose.

7. HIP-PBCA nanoparticles (10:1 HIP:dalargin molar ratio) with dalargin encapsulated (2.0 mg/kg total dose used in formulation)

The mice were sacrificed at 20 minutes following injection, and the brains and blood samples collected and analysed for the presence of peptide by LC-MS-MS. The brain data were corrected for blood contamination assuming a blood contamination of 15 μl per gram of brain. The results obtained are shown in figure 9:

The results of the in vivo study suggest that encapsulation of the peptide within the core of HIP-PBCA nanoparticles using the HIP process, is superior to adsorption of the peptide on the particle surface.

Example 13 - Effect of pH on the encapsulation efficiency of dalargin in HIP-PBCA nanoparticles

In the prior art, the PBCA nanoparticles are formed by slow polymerisation of the BCA monomer in an acidic water in oil emulsion, where the pH of the aqueous phase is around 2.0 (0.01 N HCl). The polymerisation reaction under acidic conditions requires a period of at least 3 hours to reach completion. This method however, employs a neutral pH to allow rapid polymerisation. The aqueous phase that is used is phosphate buffered saline (PBS, pH 7.2). At neutral pH, the BCA monomer is known to polymerise rapidly (within seconds). As a result, the production of HIP-PBCA nanoparticles requires the very quick formation of an emulsion. This is achieved in this method by means of homogenisation at high speed (7,500 rpm or higher) using a Silverson L4RT homogeniser. It was hypothesized that a faster polymerisation reaction at neutral pH would improve the encapsulation efficiency, by quickly entrapping the peptide in the particles. In contrast, prolonged polymerisation could lead to gradual loss of peptide from the emulsion into the aqueous phase. To test the hypothesis, nanoparticles were prepared, using the HIP-PBCA process, by emulsifying the BCA monomer with extracted peptide in either PBCS or the original medium of the prior art, 0.01 N HCl. Both the acidic and neutral aqueous phases contained the required stabilisers (0.2% pluronic F68, 1% dextran). The nanoparticles were prepared following the procedure described in example 3. The amount of peptide used per formulation was 5 mg. The following formulations were prepared (one preparation each):

1. HIP-PBCA nanoparticles alone (35:1 HIP content), pH2
2. HIP-PBCA nanoparticles alone (35:1 HIP content). pH7
3. HIP-PBCA nanoparticles (35:1 HIP:dalargin molar ratio) with dalargin encapsulated (5.0 mg input), pH2
4. HIP-PBCA nanoparticles (35:1 HIP:dalargin molar ratio) with dalargin encapsulated (5.0 mg input), pH7
5. HIP-PBCA nanoparticles (10:1 HIP:dalargin molar ratio) with dalargin encapsulated (5.0 mg input), pH2
6. HIP-PBCA nanoparticles (10:1 HIP:dalargin molar ratio) with dalargin encapsulated (5.0 mg input), pH7
7. HIP-PBCA nanoparticles (5:1 HIP:dalargin molar ratio) with dalargin encapsulated (5.0 mg input), pH2
8. HIP-PBCA nanoparticles (5:1 HIP:dalargin molar ratio) with dalargin encapsulated (5.0 mg input), pH7

The nanoparticle formulations were centrifuged to remove any free peptide and re-suspended in water or PBS. The encapsulation efficiency was determined by breaking up the particles in 10 mM NaOH (overnight incubation at room temperature) and then analysing by LC-MS. The results obtained are shown in figure 10.

The results support the hypothesis that rapid formation of the PBCA polymer at neutral pH would result in higher peptide encapsulation efficiency than slow formation of the polymer at acidic pH as is the case in the prior art. Despite loss of some of the peptide due to degradation by the treatment with NaOH, the results obtained clearly show the benefits of forming the particles at neutral pH. At a HIP:dalargin ratio of 10:1, 63.23% of the input peptide was entrapped into the nanoparticles when the particles were formed at pH 7. At pH 2, the encapsulation efficiency was significantly lower at 2.36%. Overall, the encapsulation efficiency was higher when the particles when the particles were prepared at pH 7 than when they were prepared at pH 2.

Example 14 - Encapsulation of domain antibody in PBCA nanoparticles using the HIP process

A domain antibody (anti-hen egg lysozyme dAb) was formulated in PBCA nanoparticles following the procedure described in example 3. The amount of protein used in the formulation was 10 mg. A total of two formulations were prepared. In order to determine the amount of encapsulated dAb, the particles were centrifuged to remove any free protein and then analysed by Edman sequencing. In addition to sequence information, Edman sequencing can also be used to provide quantitative information. The process involves harsh chemical treatment which destroys the particle and allows detection of the encapsulated material. The results obtained are shown in figure 11. The results suggest that it is possible to encapsulate a larger molecule using the HIP-PBCA process, but at a lower efficiency. However, it may be possible to increase the efficiency of encapsulation by optimising the protocol for use with the domain antibody. With the current protocol, which has been optimised for dalargin, it was possible to encapsulate approximately 2.56 mg of the 10 mg used. This amounts to an encapsulation efficiency of 25.6%, which is high for a single emulsion process where a protein is entrapped within a hydrophobic particles matrix.

Example 15 - Encapsulation of monoclonal antibody (anti-IL-13 mAb) within the hollow PBCA nanoparticles.

Monoclonal antibody (anti-IL-13 mAb) was entrapped within the aqueous core of the nanoparticles by inclusion into the inner aqueous phase of the homogenisation process. The inner aqueous phase was prepared by mixing 0.5 ml of a 20 mg/ml solution of mAb (10 mg protein) and 0.5 ml of a stabiliser solution (sodium cholate, 10% w/v). The nanoparticles were then prepared by the double emulsion process as described in Example 5. The resulting nanoparticles were pelleted by centrifugation to separate free from encapsulated antibody. Both pellet (entrapped antibody) and supernatant (free antibody) were analysed by total protein assay (bicinchoninic acid assay) in order to determine the encapsulation efficiency. The results of the analysis are shown in Figure 12. The amount of encapsulated mAb was found to be 8.62 mg. The amount of free mAb was 1.79 mg. The efficiency of encapsulation was 86.2%, with the loading efficiency 14.4% w/w.

Example 16 - Optimisation of the HIP process for improved encapsulation of domain antibody in PBCA nanoparticles

In order to improve the loading of domain antibodies, the dalargin protocol was optimised further. The protocol for dalargin that was used as a starting point for optimisation is described in examples 10 and 11.
The aim of the protocol modification was to achieve full solubilisation of the antibody in the organic phase and efficient incorporation into the nanoparticles.
This was accomplished by including an additional homogenisation step to form a suspension of the HIP dAb complex in the organic phase. In general, the following changes were made to the dalargin protocol:

The dAb that was used was the VEGF-myc dAb. The dAb is named DOM15-26-593 and is disclosed in PCT WO2008/149147
The dAb was formulated at an input amount of 12 mg (0.843 μmol) per 100 mg PBCA polymer (12% w/w dAb / PBCA, 12 mg dAb per 100 mg PBCA polymer.
The dAb was complexed with the HIP (docusate sodium) at a molar ratio of 82:1. The HIP solution concentration was 30.581 mg/ml (0.06879 mmol in 1 ml).
The acidification of the dAb solution was carried out gradually and with constant mixing to prevent exposure of the molecule to too low pH values and degradation. The pH of the dAb soltuion was lowered to a pH of 3.6 with HCl.
The $CaCl_2$ was not used as it could interfere with binding of the HIP to the dAb.

The acidified dAb was extracted from the aqueous phase by vortex mixing 500 μl acidified dAb solution (24 mg/ml, 12 mg protein) with1,000 μl docusate sodium in DCM (30.581 mg/ml, 3.058 %w/w) followed by centrifugation to separate the two phases. Unlike dalargin, the dAb was found not to fully solubilise into the organic phase. Instead, it formed a white precipitate at the interface. The precipitate clearly consisted of the dAb:HIP complex as its volume seemed to be proportional to the amounts of HIP and dAb used in the extraction. Attempts to fully extract the dAb using a reduced volume of aqueous phase from 500 μl to 367.76 μl (no addition of water) were less successful than using the 500 μl volume. A series of experiments suggested that a high isoelectric point was favourable, but it was possible to successfully precipitate dAbs with low pI's (VEGF dAb-myc, pI=6.6 in this case) as well. Following centrifugation, the aqueous layer was collected and stored at 4 degrees C. The organic layer, including the HIP-dAb solid pellet was used to prepare the nanoparticles.
In order to prepare the nanoparticles, it was necessary to solubilise the HIP-dAb pellet into the organic phase. This was achieved by introducing the following, additional homogenisation step to the process:

The aqueous phase was removed and the organic phase and dAb precipitate were homogenised in the 2 ml eppendorf using an Ultra-Turrax homogeniser (T25 basic, speed setting 1). The formulation was homogenised for 15 seconds to form a white suspension.
It was important to ensure that the HIP-dAb pellet came into contact with the homogeniser probe and started mixing immediately.
Homogenisation for a longer period of 1 minute gave a better suspension, but the dAb lost activity.
Following homogenisation, the organic phase was left into the 2 ml eppendorf and 100 μl BCA monomer was added. The liquid monomer was found to easily mix with the organic phase. The organic phase was then used to prepare the nanoparticles as described in example 12.

The modified procedure was also applied to the preparation of HIP-PBCA nanoparticles containing encapsidated mAb. A full length monoclonal antibody (anti-CD23 mAb as disclosed in PCT WO99/58679 150,000 Da, 12 mg per 100 mg PBCA polymer, 860:1 HIP:mAb molar ratio) was formulated by following the protocol developed for dAbs. The following observations were made:

> The mAb (anti-CD23 as disclosed in WO99/58679) was found to require higher amounts of HCl than the VEGF dAb. When extracted using the HIP agent, mAbs were found to behave similarly to the dAbs: they did not fully solubilise into the organic phase and formed a white precipitate at the interface.

The high concentration of the mAb stock solution allowed to experiment with the use of a 250 μl volume of aqueous phase instead of 500 μl in order to achieve full extraction of the mAb into the organic phase. This ratio of 4:1 organic phase to aqueous phase worked less well, it appeared to compromise the activity of the mAb as it was exposed to more solvent. A 1:1 ratio of organic to aqueous phase was clearly preferable with both mAbs and dAbs.
In order to prepare the nanoparticles the HIP-mAb pellet was solubilised into the organic phase by homogenisation by means of the same homogenisation step that was employed with dAbs. The homogenisation was found to be successful, but the suspension was less smooth, probably due to the larger size of the HIP-mAb complexes. Homogenisation for a longer period of 1 minute gave a better suspension, but the mAb appeared to denature. Therefore, the homogenisation step was kept brief at 15 seconds.
The nanoparticles were then prepared following the dAb protocol as described earlier in this example.
In general, the encapsidation of biopharms larger than peptides was found to require substantial modifications to the dalargin protocol.
Both dAbs and mAbs required higher HIP:biopharm molar ratios for solubilisation (82:1 and 860 respectively).
Both dAbs and mAbs were found not to fully solubilise into the organic phase. Instead, the formed a precipitate at the interface. In order to solubilise into the organic phase, the precipitate was homogenised into the organic phase to form a solid in oil suspension. This resulted in successful particle formation.

<u>Example 17 -Encapsulation of domain antibodies in PBCA nanoparticles using the modified HIP process.</u>

The modified HIP protocol for dAbs as described in example 16 was used to encapsidate a series of dAb molecules. The dAbs were selected on the basis of their isolelectric point. The aim was to cover the range of isolelectric points (pl) that would be likely to be used in the process in order to confirm that the process was versatile and suitable for a range of dAbs. The following dAbs were selected for the experiment (table 3):

Table 3

| dAb | DOM 15-10-11 Vk, NT tagless | DOM 15-10-11, Vk, myc | DOM 15-10-11, Vk, HA |
|---|---|---|---|
| Concentration (mg/ml) | 39.734 | 22.585 | 12.523 |
| Isoelectric point (pl) | 9.0 | 6.8 | 8.2 |

The DOM number refers to the domain antibody as disclosed in WO2008/149146. The myc refers to the myc-tag on the domain antibody or HA refers to an HA tag on the domain antibody.
Each dAb was individually formulated into PBCA nanoparticles using docusate sodium as the HIP agent at molar ratio of 70:1.
The reagents used in the HIP extractions are listed in the table below (table 4):

Table 4

| Formulation | DOM15-10-11 NT | DOM15-10-11 myc | DOM 15-10-11 HA |
|---|---|---|---|
| dAb amount (mg) | 6.0 | 6.0 | 6.0 |
| dAb amount (μmol) | 0.490 | 0.439 | 0.451 |
| dAb solution concentration (mg/ml) | 39.734 | 22.585 | 12.523 |
| Volume of dAb solution (μl) | 151.00 | 265.66 | 479.12 |
| Amount of docusate sodium (mg) Mr = 444.55 Da | 15.248 | 13.661 | 14.034 |

(continued)

| Formulation | DOM15-10-11 NT | DOM15-10-11 myc | DOM 15-10-11 HA |
|---|---|---|---|
| Amount of docusate sodium (μmol) Mr = 444.55 Da | 34.30 | 30.73 | 31.57 |
| Docusate sodium solution concentration (mg/ml) | 15.248 | 13.661 | 14.034 |
| Volume of docusate sodium solution (μl) | 1000 | 1000 | 1000 |

Acidification of dAb solution for extraction.

The dAb solutions were diluted prior to acidification by addition of water as follows (table 5):

Table 5

| Formulation | DOM15-10-11 NT | DOM15-10-11 myc | DOM15-10-11 HA |
|---|---|---|---|
| Volume of dAb solution needed (μl) | 151.00 | 265.66 | 479.12 |
| Volume of water needed (μl) | 328.12 | 213.46 | 0 |
| Total volume of aqueous phase | 479.12 | 479.12 | 479.12 |
| Volume of HCL (2 M) added (μl) | 2.00 | 5.00 | 3.00 |
| Final pH | 3.4 | 2.5 | 3.0 |

The solutions were acidified by addition of HCl (2 M). All dAb solutions were acidified to a pH of around 3.0 as measured by indicator strips. The final volume of each acidified solution was brought up to 500 μl with water.
The dAbs were then extracted into the organic phase as described in example 16. All dAbs were found not to fully solubilise in the organic phase and to form a precipitate at the interface. The untagged dAb (NT) was found to yield a precipitate that was much thinner than those of the other dAbs. The high isolelectric point and strong positive charge of the dAb had apparently permitted the formation of a stronger, more hydrophobic complex with the HIP and resulted in a greater degree of solubilisation and transfer into the organic layer.
Following removal of the aqueous phase (top layer) the organic phase and dAb precipitate were solubilised by homogenisation and the nanoparticles prepared as described in example 16.

Analysis of the nanoparticles by SDS-PAGE to assess loading of the dAbs.

In order to analyse the nanoparticle suspensions by SDS-PAGE, samples were spun for 10minutes at 13000rpm in a micro-centrifuge. The supernatant was aspirated and the pellet resuspended in 100μl of PBS. Supernatant and pellet fractions were disrupted with 1x NuPAGE LD and reducing agent, heated to 80°C for 4 minutes, and examined by SDS PAGE using commercially obtained NuPAGE gels. A sample of supernatant from hollow formulations containing dAb was also examined and used as a positive control.
Lanes 1 and 6: molecular weight markers. Lane 2: VEGF dAb DOM15-10-11, untagged encapsidated in HIP-PBCA nanoparticles. Lane 3: VEGF dAb DOM15-10-11, myc tagged, encapsidated in HIP-PBCA nanoparticles. Lane 4: VEGF dAb DOM15-10-11, HA tagged, encapsidated in HIP-PBCA nanoparticles. Lane 5: VEGF dAb DOM15-10-11, untagged, encapsulated in hollow nanoparticles (positive control). The gel confirmed that encapsidation of the dAbs had taken place. The gel also confirmed that the dAbs were intact and that they had not fragmented due to the particle preparation process.
The gel clearly showed that the dAb had been encapsidated within the particles, as they co-localised with the nanoparticle pellets following removal of any free dAb (figure 13).
The dAb had clearly been encapsidated within the nanoparticles, as analysis of the pellet under non-denaturing conditions (native gel) did not yield any bands on the gel as the dAb remained in the particles (results not shown). It was necessary to analyse the particles by SDS-PAGE, as the denaturing conditions (heat treatment in the presence of SDS) were required for the dAb to be released from the particles and run on the gel. The encapsidation was found to be successful with all dAbs tested. This suggests that the additional homogenisation step successfully solubilised the HIP-dAb complex into the organic phase to allow entrapment of the dAb into the particles. As a result, the fact that the HIP-dAb complexes precipitated at the interface following extraction, as was the case especially with low μl dAbs, did not compromise the encapsidation of the dAbs in the particles. Therefore, the modified protocol for the encapsidation of dAbs into HIP-PBCA particles was found to be independent of pI for the ranges tested and suitable for a range of dAbs.

Example 18 - Encapsulation of domain antibodies in PBCA nanoparticles using the modified HIP process; determination of loading efficiency and measurement of activity of formulated dAb.

In order to determine the loading efficiency that the modified HIP protocol could achieve, a HIP PBCA nanoparticle formulation was prepared with a tool dAb (VEGF-myc dAb, DOM15-26-593 as disclosed in WO2008/149147. The dAb was formulated at an input amount of 12 mg (0.843 μmol) per 100 mg PBCA polymer (12% w/w dAb /PBCA, 12 mg dAb per 100 mg PBCA polymer. The formulations were prepared using the modified HIP protocol for dAbs as described in example16.
Following preparation, the nanoparticles were characterised by SDS-PAGE in order to confirm that the dAb had remained intact and that it had been successfully entrapped within the particles. Analysis by SDS-PAGE was performed as described in example 17. A set of dAb standards of known amounts were also analysed on the gel alongside the formulation and were used to determine the amount of encapsidated dAb (figure 14). This was achieved by photographing the gel and measuring the signal intensity from the bands of the standards using labworks V4.6. The gel was set up as follow:

Lanes 1 and 7: molecular weight markers. Lanes 2-4: dAb nanoparticle formulations. Lane 5: empty nanoparticles (negative control). Lanes 7-10: dAb standards (500, 125, 31.25 and 7.8 μg/ml). Lanes 11-14: dAb standards (7.8, 31.25, 125 and 500μg/ml).The gel confirmed that encapsidation of the dAbs had taken place and that the dAb was intact. Comparison of the sample band intensities to those of the standards suggested that the concentration of the dAb in the nanoparticle sample was 413.7 μg/ml.

The band intensities were used to construct a standard curve. The curve was then used to calculate the amount of dAb in the nanoparticle formulation from the intensity of the band of the nanoparticle sample.
The gel confirmed that the dAb had been successfully entrapped within the particles and that it had remained intact following encapsidation. From comparison to the standards, the concentration of dAb in the nanoparticle formulation was found to be 413.7 μg/ml. This translated to a total of 3.31 mg of encapsidated dAb in the nanoparticles out of the 12 mg input. Therefore, the loading efficiency was 27.6 %. The dAb loading was 3.31 % w/w.
In order to release the encapsidated dAb and assess its activity, nanoparticle samples were also subjected to heat treatment. The dAb was released from the nanoparticles by incubation at temperatures ranging from 4 to 65°C for 1 hour in the presence of 1% Tween 20. The process is known to achieve release of at least part of the encapsidated dAb from the particles, however it can also cause some loss of dAb activity. To minimise loss of dAb activity as a result of the heat treatment, samples were also incubated at 65°C for 5 minutes, followed by a milder treatment at the lower temperature of 37°C for 55 minutes.
Following the incubation, the samples were centrifuged at 10,000 rcf for 10 minutes to separate any released dAb from the particles. The supernatants, which contained released dAb, were collected and analysed by ELISA for activity.
The released dAb was analysed by ELISA as follows:

Nunc maxisorb 96 well plates were coated with 0.5 μg/ml rVEGF overnight at 4°C. The plates were then washed with wash buffer (PBS + 0.1% Tween) 4 times and then blocked with blocking buffer (PBS + 1% BSA) for 1 hour at room temperature whilst being rocked. Plates were washed as above, then 50 μl triplicate supernatant samples were added to the wells and plates were incubated as above. The plates were washed, then 50 μl per well anti-myc Ab ( mouse) solution was added and the plates were again incubated as described above. Following washing of the plates, 50 μl per well anti mouse HRP was added and the plates were incubated as above. The plates were finally washed as above and then 50 μl/well TMB reagent was added.
The colour was allowed to develop and the reaction was stopped by addition of 50 μl per well HCl (1 M). The absorbance was measured at 450 nm using a Versamax plate reader and Softmax Pro V5.3 software.

The results obtained from the ELISA assay are shown in table 6:

Table 6

| Temp | 4°C | 56°C | 65°C | 65/37°C |
|---|---|---|---|---|
| Conc. (μg/ml) | 0.44 | 4.0 | >50 | 61 |

The released dAb was found to be active, with the high temperatures releasing a greater amount of protein from the particles. The samples treated at the two temperatures of 65 and 37 degrees C were found to exhibit the highest amount of released active dAb. The original level of activity in the formulation was hard to estimate as the release method is known to compromise activity, but, considering the PAGE result, the 65/37 method produced material with approximately 50% of the specific activity of the standards.
The released dAb was analysed by ELISA, which gave a reading of active dAb, as well as by SDS-PAGE, which detected total dAb. The dAb was analysed on the gel alongside a series of standards. The amount of dAb was then determined by means of a standard curve that was constructed by measuring the band intensities of the standards. It was found that the concentration of active dAb (61 ug/ml, as measured by ELISA) was 44% of that of total dAb (137.89 ug/ml as measured by SDS-PAGE).

Therefore, at least 50% of the formulated dAb in the nanoparticles was found to be active. This was considered to be a very good level of activity, considering that the formulation process involved solubilisation in an organic phase followed by exposure to mixing by homogenisation. Therefore, the particle preparation process appears to be suitable for the formulation of domain antibodies.

Example 19 - *In vivo* evaluation of HIP PBCA nanoparticles containing domain antibodies for their ability to deliver their protein load to the brain in the mouse via the intravenous route.

The nanoparticle formulation described in example 18 was evaluated for its ability to deliver its dAb load to the brain in the mouse model.
Nanoparticles containing encapsidated VEGF dAb were compared to free dAb in order to determine whether the particles could increased the brain uptake of the dAb compared to that of free dAb molecules. A batch of empty nanoparticles was also prepared and evaluated as a negative control.

Design of the *in vivo* study.

The study involved two different time points at which dAb brain levels were to be assessed: 10 minutes and 60 minutes post administration. The earlier time point was chosen in case the dAb concentration in the brain peaked within minutes after injection, as was found with the dalargin peptide. The later time point was selected in order to allow for some clearance of the dAb from the blood circulation to occur. Any dAb that was present in the blood could contaminate the brain samples and distort the data obtained. The short half life of the dAb in the blood circulation (20 minutes) could perhaps limit the blood contamination at the later time point, thus permitting a clearer reading of brain penetration.

Correction of blood contamination in brain samples:

In order to account for the amount of dAb in the brain samples that was not due to brain uptake but was merely present into the brain blood vessels and not in the brain tissue itself (blood contamination), a start-chase study was performed. All mice received a dose of a chase molecule that is known to remain in the blood and not to penetrate the brain. The chase molecule that was chosen was the anti-CD23 full length antibody as disclosed in WO99/58679 that exhibits negligible brain uptake. Therefore, any amount of anti-CD23 mAb detected in the brain samples would be solely due to its presence in the blood contaminating the brain tissue. The chase was given to the animals 5 minutes before sacrificing them to ensure that the antibody remained in the blood and no tissue uptake took place in other areas of the body.

Groups of animals:

A: Control particles, given at t=0, followed by chase at t=5 minutes.
B: dAb in nanoparticles, given at t=0, followed by chase at t=5 minutes.
C: dAb free in solution (control), given at t=0, followed by chase at t=5 minutes.
The above groups were sacrificed at t=10 minutes, at 5 minutes post administration of the chase.
D: Control particles, given at t=0, followed by chase at t=55 minutes.
E: dAb in nanoparticles, given at t=0, followed by chase at t=55 minutes.
F: dAb free in solution (unformulated control), given at t=0, followed by chase at t=55 minutes.

The above groups were sacrificed at t=60 minutes, at 5 minutes post administration of the chase.

Preparation of the doses.

Chase: anti-CD23 mAb as disclosed in PCT WO99/58679, 2.0 mg/kg.
The doses were prepared by diluting the 68 mg/ml mAb stock solution to 500 μg/ml. This amounted to a 50 μg dose in a 100 μl volume for a 25 g mouse.
Nanoparticles with dAb: 1.584 mg/kg, 50 mg/kg PBCA polymer.
The nanoparticle suspension was prepared for injection by adding 160 μl polysorbate 80 solution (25 % w/w) to 3,600 μl nanoparticle suspension. This resulted in a final concentration of formulated dAb of 396.1 μg/ml. This amounted to a dAb dose of 39.6 μg in a 100 μl volume for a 25 g mouse.
Empty nanoparticles (negative control): 50 mg/kg PBCA polymer.
The nanoparticle suspension was prepared for injection by adding 160 μl polysorbate 80 solution (25 % w/w) to 3,600 μl nanoparticle suspension as above. This resulted in a final concentration of PBCA polymer of 1.25 mg/ml. This amounted to a PBCA dose of 125 μg in a 100 μl volume for a 25 g mouse.
Free dAb in solution (unformulated control): 1.584 mg/kg.
The dAb solution was prepared for injection by diluting the 2.0 mg/ml stock solution to 396.1 μg/ml. This amounted to a dAb dose of 39.6 μg in a 100 μl volume for a 25 g mouse.
Injection of the mice: the CD1 mice were injected intravenously (tail vein injection).
The injection volumes were calculated on the basis of the weight of the mice. Following the end of the *in vivo* procedure, brains and serum samples were collected from all the mice and frozen. The tissue samples were snap frozen in liquid nitrogen. All samples were stored at -80°C.

Homogenisation of brains for analysis.

The brains were thawed and weighed. A volume of PBS that was twice the weight of the brain volume was added to each brain. The brains were then homogenized using a Covaris acoustic tissue processor (Covaris E210).

Analysis of brains by Meso Scale Discovery (MSD)

The brain homogenates and serum samples were analysed by MSD. This was achieved by adapting the anti-VEGF ELISA assay described in example 18 to an MSD format. The serum samples were analysed at 1:1,000 in 1: 10,000 dilutions. The brain samples were analysed in 1:5 dilutions.

Results

The data was processed and the results shown in figure 15 were generated. At 10 minutes post administration, the dAb in nanoparticles resulted in detectable brain uptake which amounted to 8.0 ng/ml. The free dAb was also detectable in the brain at the slightly lower concentration of 3.3 ng/ml (preliminary data). The preliminary data did not include the readings from two animals that could not be corrected for blood contamination as the readings from the serum were too high to be quanitified. In general, the nanoparticles appeared to marginally increase the brain uptake of the protein at the 10 minute time point.
However, at 60 minutes, the situation was reversed. The free dAb appeared to accumulate in the brain resulting in a further increase in its brain levels to 13.5 ng/ml. The observed result can be explained as follows:

1. The half life (t½) of the free dAb in the blood circulation was likely to be longer than that of the particles because of its hydrophilicity. This probably meant that more of the free dAb was available for brain uptake compared to dAb formulated in nanoparticles.
2. The loading of dAb into the particles was not high enough to offset the loss of formulation due to rapid elimination from the systemic circulation. The drug loading in the particles was 3.31% w/w. The dalargin formulations had previously required a loading of 5.0 % w/w in order to generate brain levels of 45 ng/ml. Higher peptide loadings of 8.9 % had given peptide concentrations in the brain that were up to 833 ng/ml. It seems that a loading of 3.31 % w/w, especially for the high molecular weight biopharm, is not sufficient for significant brain delivery therefore further optimisation of loading would be necessary.
3. The 10 and 60 minute time points were perhaps too late. Previous studies with dalargin and loperamide had all demonstrated that delivery is rapid, within 2-3 minutes of injection. The previous studies had also suggested that the highest drug levels in the brain were achieved within 5 minutes of administration or earlier. A time point of 10 minutes was chosen to ensure sufficient time to perform a start chase study and 60 minutes chosen to detect any long lasting effect of domain antibody.
4. The HIP PBCA system is only passively targeted to the brain. When given intravenously, such particulates that exhibit a hydrophobic surface are known to be passively targeted to a number of organs in addition to the brain. These organs include the liver and the spleen. When given intravenously, the nanoparticles will reach the liver and the spleen first, before they encounter the brain. As a result, the majority of the injected dose could be delivered to those tissues, leaving only a fraction available for delivery to the brain. This could have severely compromised the ability of the particles to reach the brain in this experiment.

To highlight the impact of the loss of dAb from the blood circulation, the brain to blood ratios were also calculated (figure 16). The results clearly show that much higher proportions of dAb were present in the brain compared to the blood when given with nanoparticles compared to when the dAb was given free in solution. In fact, the formulated dAb exhibited a brain to blood ratio of 0.04 (60 minutes), which is above the ratio at which a compound is considered to be brain penetrant. The free dAb did not exceed this brain penetration threshold at any of the time points that were analysed. Therefore, despite significant loss of the injected dose, in terms of overall ability to penetrate the blood brain barrier the particles may ultimately be superior to free dAb.
In general, the intravenous route is known to be the most challenging route of administration for passively targeted particles such as the HIP-PBCA system. Therefore, intravenous administration was not the ideal method of assessing the ability of the HIP-PBCA system to deliver its drug load across the BBB from the blood. For this reason, an intracarotid study was also carried out. Administration via the intracarotid route by-passes tissues such as the liver and spleen and provides a more direct route to the brain. As a result, more of the injected nanoparticle dose is available for brain delivery. In a head to head comparison between free and formulated drug, the intracarotid route is more likely to provide a true measure of the ability of the nanoparticles to overcome the BBB.

Example 20 - *In vivo* evaluation of HIP PBCA nanoparticles containing domain antibodies for their ability to deliver their protein load to the brain in the mouse via the intracarotid route.

*In vivo* evaluation of the nanoparticle formulation - intracarotid administration.

The nanoparticle formulation was evaluated for its ability to deliver its dAb load to the brain in the mouse via the intracarotid route.
The route was selected because it provides a direct avenue to the brain. When a substance is given into the carotid artery, the first tissue that is reached is the brain. In contrast, when a drug is given intravenously, it will encounter tissues such as the liver prior to reaching the brain. This was found to limit the ability of the nanoparticles to deliver to the brain, as they are also known to be taken up by tissues such as the liver and spleen. In fact, Kreuter *et al* had observed that with their PBCA adsorbed particles, the majority of the injected empty nanoparticle dose (~60%) when given via the tail vein was taken up by the liver.
In general, the intravenous route is well known to be the least favourable and most challenging route of administration for passively targeted delivery systems such as the HIP-PBCA nanoparticles.
Therefore, the intracarotid route was thought more likely to provide an accurate indication of the ability of the nanoparticles to overcome the blood brain barrier.

Design of the *in vivo* study.

The study design was the same as for the intravenous study, with the only differences being the following:

1. The animals were kept under terminal anaesthesia throughout the experiment. This was necessary due to the complexity of the surgical procedures involved.
2. The nanoparticle formulations and free dAb were administered via the intracarotid route by means of a surgically prepared cannula.
3. The chase was given intravenously via the tail vein, but unlike the previous study the antibody was given by means of cannula.

Groups of animals:

A: Control particles, given at t=0, followed by chase at t=5 minutes.
E: dAb in nanoparticles, given at t=0, followed by chase at t=5 minutes.
C: dAb free in solution (control), given at t=0, followed by chase at t=5 minutes.
The above groups were sacrificed at t=10 minutes, at 5 minutes post administration of the chase.

B: Control particles, given at t=0, followed by chase at t=55 minutes.
F: dAb in nanoparticles, given at t=0, followed by chase at t=55 minutes.
D: dAb free in solution (unformulated control), given at t=0, followed by chase at t=55 minutes.

The above groups were sacrificed at t=60 minutes, at 5 minutes post administration of the chase.

Preparation of the doses.

Chase: anti-CD23 mAb as disclosed in PCT WO99/58679, 2.0 mg/kg.
The doses were prepared by diluting the 68 mg/ml mAb stock solution to 500 μg/ml. This amounted to a 50 μg dose in a 100 μl volume for a 25 g mouse.
Nanoparticles with dAb: 1.584 mg/kg, 50 mg/kg PBCA polymer.
The nanoparticle suspension was prepared for injection by adding 160 μl polysorbate 80 solution (25 % w/w) to 3,600 μl nanoparticle suspension. This resulted in a final concentration of formulated dAb of 396.1 μg/ml. This amounted to a dAb dose of 39.6 μg in a 100 μl volume for a 25 g mouse.

Empty nanoparticles (negative control): 50 mg/kg PBCA polymer.

The nanoparticle suspension was prepared for injection by adding 160 μl polysorbate 80 solution (25 % w/w) to 3,600 μl nanoparticle suspension as above. This resulted in a final concentration of PBCA polymer of 1.25 mg/ml. This amounted to a PBCA dose of 125 μg in a 100 μl volume for a 25 g mouse.

Free dAb in solution (unformulated control): 1.584 mg/kg.

The dAb solution was prepared for injection by diluting the 2.0 mg/ml stock solution to 396.1 μg/ml. This amounted to a dAb dose of 39.6 μg in a 100 μl volume for a 25 g mouse.

Results

The data was processed and the results shown in figure 17 were generated. At 10 minutes post administration, the dAb in nanoparticles group exhibited high levels of dAb in the brain, at an average of 627.60 ng/ml. The actual concentration of dAb in the brain was probably higher, as the above figure did not include the readings from two animals. The two samples gave signals that were too high for quantification but unfortunately were not analysed in time for inclusion into this document. One of the animals was a clear outlier that gave a relatively low brain concentration of 45.45 ng/ml. This resulted in the large errors observed with the group. Nevertheless, the average concentration of formulated dAb in the brain was nearly 9-fold higher than that of free dAb, which was 71.67 ng/ml.
At 60 minutes post injection, the levels of dAb in the brain remained high at 146.51 ng/ml. The concentration of free dAb had instead fallen to an average of 3.17 ng/ml. Therefore, at 60 minutes post injection the brain concentration of dAb given in nanoparticles was 46-fold higher than that achieved with naked dAb.
In general, the nanoparticles were found to be very successful at delivering the dAb to the brain via the intracarotid route. This was also evident from the determination of the brain to blood ratios for the groups (figure 18). The dAb in nanoparticles group exhibited brain to blood ratios that were greater than 1 at both time points (1.569 and 1.845 at10 and 60 minutes respectively) suggesting that the majority of formulated dAb had successfully reached the brain. In contrast, the free dAb groups were characterised by brain to blood ratios that were significantly lower, at 0.012 and 0.286 for the 10 and 60 minute points respectively.
In conclusion, the nanoparticle delivery system was found to greatly improve the delivery of dAb to the brain when given via the intracarotid route. This was because the route reaches the brain prior to liver and the spleen, which are tissues to which the formulation is also passively targeted in addition to the brain.
The intravenous route was not as successful, providing a transient hint of an increase in brain uptake of dAb. This was probably due to the insufficient dAb loading into the particles, as well as due to the delivery system being taken up by other tissues resulting in only a fraction of the injected particles reaching the brain.
Therefore, in order to improve the system and achieve efficient delivery to the brain via the intravenous route, it is necessary to further improve the loading of dAb into the nanoparticles. This could be achieved by employing the hollow PBCA system, which has been shown to have a greater capacity for the loading of dAbs than the HIP PBCA system. Provided that it is stable enough in vivo, the hollow PBCA particles may be more successful than the HIP PBCA system at delivering the dAb to the brain. In order to ensure their stability, it may be necessary to employ blends of the PBCA polymer with other polymers of higher molecular weight such as PLGA, PLA or PCL. The delivery system may also benefit from the use of pegylated copolymers. Such polymers could improve the circulation time of the nanoparticles in the blood and thus improve brain delivery.
An additional means of improving the delivery system is to alter its mechanism of brain targeting. An actively targeted nanoparticle that exhibits a ligand that binds a target on the BBB is likely to improve brain uptake and concomitantly limit the loss of particles to other tissues. In order to achieve active targeting it will probably be necessary to extensively pegylate the nanoparticle surface in order to limit any nonspecific targeting to other organs.
In general, the nanoparticle system described in this document has great potential to achieve the efficient delivery of domain antibodies to the brain, however in order to achieve this significant optimisation is still required.

Example 21 -Encapsulation of domain antibodies in PCL microspheres using a modified HIP process.

The polymer polycaprolactone, (PCL, Lactel), was used as a test case both for the generation of microspheres and the use of slow-release polymers with the HIP process for dAb encapsidation. Initial experiments used modifications of the HIP encapsulation protocols described in Examples 14 and 17 to accommodate the use of PCL to generate both empty nanoparticles and microspheres.
Initial formulations used 100mg/ml stocks of PCL in dichlorormethane, (DCM), and 1% pluronic F68, (Sigma) as surfactant with homogenisation speeds of 4000-7500rpm for 45 seconds, but few microspheres or nanoparticles were made, (data not shown). The process was further optimised by trying different surfactants: also 1% sodium cholate, (Sigma), or 1% Lutrol F127 Poloxamer 407, (BASF Corp.), or 1% Vitamin E TPGS, (d-ALPHA tocopheryl polyethylene glycol 1000 succinate), (Peboc /Eastman) with little initial improvement, (data not shown), until the amount of input polymer was reduced to 10mg/ml, when small numbers of large fragile microspheres of >20um could be seen under the light microscope,(data not shown), but the majority of PCL came out of suspension as macroscopic particles once the organic solvent had evaporated, (data not shown). The process was further improved in terms of particle stability by using the two most promising surfactants from the experiments described above at 2% to help stabilise the suspensions more: Lutrol F127 Polaxomer 407, (BASF Corp.) or Vitamin E TPGS and homogenisation speeds of 7500-9000rpm for 45 seconds to 2mins. Using this process around 1 μm sized particles were generated in all cases, (data not shown), but 2% Vitamin E TPGS was chosen as surfactant with a 2 min. homogenisation for a protocol to encapsidate dAbs shown below.

Preparation of HIP-PCL microspheres, (M/P), which encapsidate dAb.

HIP-PCL microspheres were prepared according to the methodology of example 16 above and any changes to this protocol detailed below.

Formulations used:

Four PCL (poly-e-caprolactone) formulations were prepared:

i) empty particles with HIP for PCL as M/P - 4000 rpm (2% Vitamin E [TPGS] as surfactant) - 2mins
ii) empty particles with HIP for PCL as M/P - 7500 rpm (2% Vitamin E [TPGS] as surfactant) - 2mins
iii) Particles with HIP for PCL as M/P + dAb for analysis, (dAb1) - 7500 rpm (2% Vitamin E [TPGS] as surfactant) - 2 mins
iv) Particles with HIP for PCL as M/P + dAb for sizing, (dAb2)- 7500 rpm (2% Vitamin E [TPGS] as surfactant) - 2 mins

Solutions needed:

(1) dAb to be extracted: anti-VEGF (DOM15-26-593 as disclosed in W02008/149147.), batch TB090220 1.5 mg/ml (14,246 Da) - used 4 x 5ml (actual re-reading of the dAb concentration using a Nanodrop 1000 Spectrophotometer, Thermo Scientific, confirmed concentration as in fact 1.04mg/ml)
(2) 82:1 HIP solution (1 ml, 30.58 mg/ml)
(3) Acidified dAb solution (25 mg/ml) N/A
(4) Aqueous phase: 1% w/v dextran , 2% w/v surfactant* in PBS
(5) PCL polymer dissolve in DCM

*Stock 10% Vitamin E [TPGS]

Preparation of PCL solution in DCM.

Aim was to provide 10mg of PCL dissolved in DCM per formulation - solubility was about -100mg / ml in DCM, maximum, but more could be dissolved @ -10mg/ml. Enough PCL was made for 5 formulations i.e. 50mg in 5ml DCM. Weighed out 50mg PCL, (opened thawed vacuum dessicator when warmed to room temperature from - 20°C), weighed (fine balance) - stirred in 10 ml beaker with PCL + 4mls DCM - stirred with glass stirrer in fume hood under cover. Once dissolved, measured in glass measuring cylinder, and topped up to 5ml with DCM then re-mix, (stirred beaker), and used quickly.

Concentration of dAb solution.

Preparation was done intially O/N at RT 600rpm and diluted back to correct concentration. The solution was concentrated using Vivaspin concentrators (Vivaspin 6, Sartorius, VS0691, MWCO 3,000 PES) and following manufacturers instructions in a Sorvall legend RT Bench Top centrifuge The concentration was from the expected 1.5 mg/ml (20.0 ml as 5ml in 4 x Vivaspin)) to 25 mg/ml (-700 μl). The process took 2 hours at 1000-1500 rpm and a further -1 hours at 3000 rpm. Initial dAb 400ul at 1 in 75 dilution gave 0.56 mg/ml by Nanodrop - this was then diluted to 760ul at 25mg/ml and acid treated to reduce the pH to -3.7.The mock material was acid treated at pH -2.5. dAb was at 25mg/ml -pH 5.0/4.5 - aim is to get down to pH3.7 - pH was checked with pH 2.5 to 4.5 paper-380ul of dAb was used, for example only 9.5mg per formulation, (and fitted an inital input concentration of 1mg/ml not 1.5mg/ml)

Table 7: Preparation of acidified solutions.

| Formulation | Empty control | dAb solution |
|---|---|---|
| Volume of 25.0 mg/ml dAb solution needed (μl) | 0 | 760, (2x 380) |
| Volume of water needed (μl) | 500.0 x 10 | 12.0 |
| Volume of 2M HCl solution added (μl) | 5.0x10 | 6.0 |
| Volume of 1 M CaCl2 added (μl) optional, ADDED LAST | 0 | 0 |
| pH following addition | <<=2.5 | ~3.7 |

Table 8; Formulations and reagents for HIP extractions (standard volume protocol).

(A) Organic phase dAb

| Formulation dAb | 10-12.0mg dAb, 82:1 H I P, 10 mg PCL |
|---|---|
| dAb amount (mg) or w/o dAb | ~9.5. |
| Concentration (mg/ml) | 25.00 |
| Volume needed (μl) | 380 |

(B) Organic phase HIP

| Formulation HIP AOT | 12.0 mg dAb, 82:1 HIP, 10 mg PCL |
|---|---|
| Concentration of AOT needed (mg/ml) | 30.58 |
| Volume of 30.58 mg/ml AOT solution needed (μl) | 1000 |

Aim was to make a 1: 2 mix of dAb (aq): DCM / HIP - above is for a 1x mix, i.e. ~500ul : 1000ul organic phase.

Table 9: Preparation of empty controls (organic phase).

| Formulation | Empty control, 82:1 HIP, 100 mg BCA |
|---|---|
| dAb amount (mg) | 0 use just PBS 480μl + 20μl of HCl to acidify |
| Concentration of AOT needed (mg/ml) | 30.58 |

(continued)

| Formulation | Empty control, 82:1 HIP, 100 mg BCA |
|---|---|
| Volume of 30.58 mg/ml AOT solution needed (μl) | 1000 |

Extraction of dAb or mock (empty particles) into the organic phase using docusate sodium as a HIP agent.

The acidified dAb or 'mock' solution and organic phase was mixed in 2 ml eppendorf tubes and added to the aqueous phase. The mixtures were vortex-mixed maximum speed for 1 minute and then placed in Bench top mixer 5432 for 5 minutes. The resulting white mixtures were centrifuged at maximum speed (20,817 rcf, 14000rpm in microfuge) for 50 minutes. The dAb-HIP complex appeared to form a thick white precipitate at the interface. The aqueous phase was collected and stored at 4 degrees C. The top aqueous phase was removed and stored and the procedure continued with the bottom organic phase.

Homogenisation of HIP-dAb complex into organic phase.

The organic phase was homogenised in the 2 ml eppendorf tube using an IKA T25 homogeniser (polytron, speed setting 1) for 7-10 seconds. The aim was to achieve complete homogenisation of the white precipitate (dAb and HIP complex) into the organic solvent (DCM).The HIP-dAb complex was easily solubilised into the organic phase to form an emulsion that appeared homogeneous. The organic phase was homogenised for a total of 10 seconds. There was little precipitate left in the tube following homogenisation and removal of the organic phase.

Preparation of microspheres.

The 1 ml organic phase of the homogenates were taken and 1 ml of PCL dissolved in DCM (100mg) was mixed in by pipetting up and down.
The resulting white suspension (2ml) was pipetted into the aqueous phase (10 ml dextran in water and 2% surfactant solution in PBS in a 25 ml beaker) at the point of probe entry below the liquid surface. The aqueous phase was being homogenised at either 7,500 rpm, (M/P) or 4000 rpm (M/P) using a Silverson L4RT homogeniser. The emulsion was homogenised for 2 minutes. The formulation was then incubated in a fume hood with stirring (speed setting 4) for 3 hours in order to evaporate the organic phase. The setting was lowered to 3 at 1 h into the incubation to prevent over-mixing of the emulsion as that resulted in the deposit of aggregates on the surface of the beaker.

Example 22 - Sizing of the microspheres.

(a) Light microscopy.

All four formulations, (i) to (iv) above were sized on the Nikon Eclipse E400 Microscope using visible light. The data showing images of these particles is shown in Fig. 19. Visible microspheres of a similar size range could be seen from all four formulations both those with and without dAb. The data suggests that microspheres are similarly formed in the presence of dAb using this process.

(b) Multi-angle static light scattering.

All four samples were sized on the Micromeritics Saturn DigiSizer 5200, High Definition Particle Size Analyser.

Samples were sized by loading sufficient material from the micro-particle sizings above into the low-volume sample handling unit attached to the Saturn Digisizer 5200 to allow an obscuration of 5-30%, preferably above 15% in a matrix of de-gassed PBS, (which needs 50-100% of the formulation). Samples were then analysed using a polycaprolactone model of analysis using a real partitioning of refractive index of 1.476 and an imaginary partitioning of refractive index of 0.0001. The flow rate was at 6L / min, the stop beam angle was at 45 degrees, the media was PBS and counts were made in triplicate. Both volume and number distribution were reported, data obtained was as a combined report, a cumulative graph and a frequency graph, for details of methods see the Micromeritics Saturn Digisizer 5200 Operators Manual V1.12, (March 2007) and the Quick reference guide.

The data is displayed for the relevant formulations: (i) to (iv) in Figs 20 (a) to (d) as graphs plotting frequency of number of particles against particle size. See below the data corresponding to these graphs.

Fig 20 (a)

**Summary Report**

**Sample**
Sample Concentration: 0.01069%
Obscuration: 29.1%

**Volume Distribution Geometric Statistics**

| | | Std Dev of 8 | | | Std Dev of 8 |
|---|---|---|---|---|---|
| Mean | 8.355 | 2.926 | Mode | 10.00 | 4.017 |
| Median | 9.754 | 3.951 | Std. Dev. Log. | 0.382 | 0.047 |
| Skewness | -0.267 | 0.106 | Kurtosis | -0.436 | 0.180 |

**Number Distribution Geometric Statistics**

| | | Std Dev of 8 | | | Std Dev of 8 |
|---|---|---|---|---|---|
| Mean | 1.393 | 0.070 | Mode | 1.000 | 0.000 |
| Median | 1.033 | 0.009 | Std. Dev. Log. | 0.214 | 0.022 |
| Skewness | 1.504 | 0.388 | Kurtosis | 2.001 | 2.445 |

Fig 20 (b)

**Summary Report**

**Sample**
Sample Concentration: 0.00284%
Obscuration: 16.7%

**Volume Distribution Geometric Statistics**

| | | Std Dev of 8 | | | Std Dev of 8 |
|---|---|---|---|---|---|
| Mean | 5.366 | 3.943 | Mode | 2.239 | 2.744 |
| Median | 4.304 | 5.816 | Std. Dev. Log. | 0.521 | 0.033 |
| Skewness | 0.523 | 0.450 | Kurtosis | -0.370 | 0.178 |

**Number Distribution Geometric Statistics**

| | | Std Dev of 8 | | | Std Dev of 8 |
|---|---|---|---|---|---|
| Mean | 1.231 | 0.016 | Mode | 1.000 | 0.000 |
| Median | 1.048 | 0.013 | Std. Dev. Log. | 0.141 | 0.012 |
| Skewness | 1.954 | 0.198 | Kurtosis | 4.737 | 1.339 |

Fig 20 (c)

**Summary Report**

**Sample**
Sample Concentration: 0.00771%
Obscuration: 21.6%

**Volume Distribution Geometric Statistics**

| | | Std Dev of 6 | | | Std Dev of 6 |
|---|---|---|---|---|---|
| Mean | 9.346 | 7.368 | Mode | 2.239 | 12.53 |
| Median | 10.45 | 7.174 | Std. Dev. Log. | 0.421 | 0.115 |
| Skewness | -0.234 | 0.252 | Kurtosis | -0.185 | 3.125 |

**Number Distribution Geometric Statistics**

| | | Std Dev of 6 | | | Std Dev of 6 |
|---|---|---|---|---|---|
| Mean | 1.355 | 1.046 | Mode | 1.000 | 0.751 |
| Median | 1.048 | 0.731 | Std. Dev. Log. | 0.196 | 0.070 |
| Skewness | 1.711 | 0.234 | Kurtosis | 3.196 | 1.059 |

Fig 20 (d)

**Summary Report**

**Sample**
Sample Concentration: 0.01069%
Obscuration: 29.1%

**Volume Distribution Geometric Statistics**

| | | Std Dev of 8 | | | Std Dev of 8 |
|---|---|---|---|---|---|
| Mean | 8.355 | 2.926 | Mode | 10.00 | 4.017 |
| Median | 9.754 | 3.951 | Std. Dev. Log. | 0.382 | 0.047 |
| Skewness | -0.267 | 0.106 | Kurtosis | -0.436 | 0.180 |

**Number Distribution Geometric Statistics**

| | | Std Dev of 8 | | | Std Dev of 8 |
|---|---|---|---|---|---|
| Mean | 1.393 | 0.070 | Mode | 1.000 | 0.000 |
| Median | 1.033 | 0.009 | Std. Dev. Log. | 0.214 | 0.022 |
| Skewness | 1.504 | 0.388 | Kurtosis | 2.001 | 2.445 |

The clearest determination of mean particle size is from the geometric mean number distribution which gave the following mean particle sizes:

i) empty particles with HIP for PCL as M/P - 4000 rpm (2% Vitamin E [TPGS] as surfactant) - 2mins mean particle size 1.231
ii) empty particles with HIP for PCL as M/P - 7500 rpm (2% Vitamin E [TPGS] as surfactant) - 2mins mean particle size 1.181
iii) Particles with HIP for PCL as M/P + dAb 1 for analysis - 7500 rpm (2% Vitamin E [TPGS] as surfactant) - 2 mins mean particle size 1.355
iv) Particles with HIP for PCL as M/P + dAb 2 for sizing- 7500 rpm (2% Vitamin E [TPGS] as surfactant) - 2 mins mean particle size 1.393

The conclusion was that although a lower speed under these conditions generated slightly large microspheres the impact was not great - the conditions described for homogenisation at 7,500 rpm generated microspheres in the presence of dAb with a mean diameter of 1.4μm so slightly larger than empty particles in this process.

Example 23 - Analysis of HIP-PCL Microspheres containing dAb.

HIP PCL microspheres containing dAb were prepared as in example 21 above.
50pi of each formulation, (dAb1 and dAb2) was taken and either:-

i) Spun in a 1.5ml microfuge tube at 3K rpm for 5' to generate a supernatant (S) - 30μl removed to a fresh microfuge tube and a pellet (P) fraction resuspended in 50μl PBS;
ii) Spun in a 1.5ml microfuge tube at 13K rpm for 5' to generate a supernatant (S) - 30μl removed to a fresh microfuge tube and a pellet (P) fraction resuspended in 50μl PBS;
iii) Spun in a Vivaspin 500 (1,000,000 molecular weight cut off) at 5K rpm for 5' to remove any incorporated dAb as particles are retained in the column and the 50μl PBS as supernatant (F) passed through and was collected. The Vivaspin 500 (Sartorius stedim biotech) was used according to manufacturer's instructions.

Samples were prepared for loading by adding 21 μl of sample to 8μl of 4x loading dye to 3μl of 10x reducing agent to generate a final volume of 32μl of which 10μl was loaded after heating to 80 degrees C in a 96 well PCR plate placed in a PCR block, (PTC-100, MJ research Inc) for 5 minutes.
Samples were then loaded on to a gel run according to manufacturer's instructions in MES SDS (2-N morpholino ethanesulphonic acid, sodium dodecyl sulphate), buffer for 35 minutes,(invitrogen) andstained using the microwave mediated version of the SimplyBlue SafeStain protocol, (invitrogen) Loading standards of unencapsidated dAb were run also alongside on the gel to help calculate concentrations, i.e. as 3.28μg, 0.82μg, 0.21μg and 0.05μg from diluted, as described above in sample preparation, 10μl loadings of 500ng/μl, 125ng/μl, 31.25ng/μl and 7.8ng/μl stocks. An image of the stained gel is shown in Fig. 21.
The gel was set up as follows:

Lane 1: Whole dAb1, Lane 2: dAb1 3K S, Lane 3: dAb1 3K P, Lane 4: dAb1 13K S, Lane 5: dAb1 13K P, Lane 6:

dAb1 F, Lane 7: Whole dAb2, Lane 8: dAb2 3K S, Lane 9: dAb2 3K P, Lane 10: dAb2 13K S, Lane 11: dAb2 13K P,Lane 12: dAb2 F, Lane 13: Molecular markers - SeeBlue Plus 2 pre-stained standard, (invitrogen), molecular weight (kd), Lane 14: 3.28pg dAb standard, Lane 15: 0.82μg dAb standard, Lane 16: 0.21 μg dAb standard, Lane 17: 0.05μg dAb standard, The gel confirmed that encapsidation of the dAbs had taken place. The gel also confirmed that the dAbs were intact and that they had not fragmented due to the particle preparation process.

The amount of material in the whole, supernatant and pellet fractions of the PCL HIP particles were ascertained for dAb 1 using band capture and the 1 D Gel quantitation package of Labworks 4.6 software (UVP). Images for analysis were captured using a Vision works station fitted with an Olympus camera under white light. The data is shown in Table 9.

Table 10: Determination of dAb loading in PCL-HIP microspheres.

| Lanes | dAb band intensity | dAb in μg |
|---|---|---|
| 1 | 997.63 | 3.5 |
| 2 | 277.54 | 1.0 |
| 3 | 865.5 | 3.0 |
| 4 | 241.3 | 0.9 |
| 5 | 927.56 | 3.1 |
| 6 | 214.26 | 0.73 |
| 7 | 1225.4 | 4.5 |
| 8 | 317.93 | nd |
| 9 | 628.75 | nd |
| 10 | 126.42 | nd |
| 11 | 1056 | nd |
| 12 | 23.896 | nd |
| 14 | 925.94 | 3.28* |
| 15 | 272.34 | 0.82* |
| 16 | 40.492 | 0.21* |
| 17 | 6.7923 | 0.05* |

Lane 1: Whole dAb1, Lane 2: dAb1 3K S, Lane 3: dAb1 3K P, Lane 4: dAb1 13K S, Lane 5: dAb1 13K P, Lane 6: dAb1 F, Lane 7: Whole dAb2, Lane 8: dAb2 3K S, Lane9: dAb2 3K P, Lane 10: dAb2 13K S, Lane 11: dAb2 13K P, Lane 12: dAb2 F, Lane13: Molecular markers - SeeBlue Plus 2 pre-stained standard, (Invitrogen), molecular weight (kd), Lane 14: 3.28μg dAb standard, Lane 15: 0.82μg dAb standard, Lane 16: 0.21 μg dAb standard, Lane17: 0.05μg dAb standard,

Gel readings for intensity of dAb were converted to dAb amounts in μg, (Table 10) using a plot of the dAb standards against band intensity, (data not shown).

The mean whole dAb formulation was read as, (lanes 1 and 7), 3.5μg + 4.5μg / 2 = 4μg - sample was diluted 21 in 32 in loading the 10μl so total dAb in 10μl = 32/21 x 4 = 6μg.

dAb 1 supernatant, (lanes 2, 4 and 6) = 1.0 + 0.9 + 0.73/3 = 0.9 μg corrected for dilution is 1.4μg

dAb1 pellet, (lanes 3 and 5) = 3.0 + 3.1 / 2 = 3.0μg corrected for dilution is 4.6μg

The fractions appear to tally as whole dAb at 6μg = dAb 1 supernatant (1.4μg) + dAb1 pellet (4.6μg) using these figures the percentage of encapsidated dAb is 77% of whole dAb, (4.6/6.0)

However percentage of input dAb - 10μl (9.5μg) that is encapsidated is 4.6/9.5 x 100 = 48%.

Example 24 - Release of dAb from HIP PCL microspheres.

In order to release dAb from HIP PCL particles for analysis of functional activity - samples of 50ut aliquots were taken from the dAb1 and dAb2 HIP PCL formulations and placed in a 1.5ml eppendorf. These were washed 2x with 1 ml of PBS, with a 5 min 5000 rpm spin in an Eppendorf 5417C microfuge. The pellet was re-suspended in 50μl PBS and a time course of 0, 20, 40 and 60 mins incubation at 56°C in a Techne heating block. The sample was then spun down, 5 min 5000 rpm spin and 30µl of supernatant, (S) removed and placed on ice for analysis. The pellet, (P), was then dried and re-suspended in 50µl.

All the fractions were subject to gel analysis - released supernatants were analysed on one gel and the released pellets were analysed on another gel. The supernatant gel is shown in Fig. 22 loadings were set up as described for the initial analysis gel in Fig 21.

The amount of material in the released supernatant and pellet fractions of the PCL HIP particles were ascertained for dAb 1 and dAb 2 using band capture and the 1 D Gel quantitation package of Labworks 4.6 software (UVP). Images for analysis were captured using a Vision works station fitted with an Olympus camera under white light.

Gel readings for intensity of dAb were converted to dAb amounts in ng, (Table 10, column 2), using a graph plot of the dAb standards against band intensity, (data not shown).

Note that the amount of material released by this process ranged from 120-189 ng (data not shown), from a pellet source of around 882-1000 ng in the particles -where 12-19% of the material was released.

Functional analysis of dAb released from PCL HIP particles by ELISA.

ELISA assay protocol describes a binding assay for measuring the ability of soluble domain antibodies (VEGF dAb) to bind to recombinant VEGF. The assay uses recombinant human VEGF (R&D Systems) coated onto the surface of ELISA plates (Nunc Immunosorb) to capture VEGF dAb. The plates are washed to remove any unbound dAb. Bound dAb is subsequently detected using an antibody to the Myc tag of the VEGF dab (9E10, Sigma). Excess antibody is removed by washing and the bound anti-myc antibody is detected using an anti-mouse IgG peroxidase conjugate (Sigma). The assay is developed using TMB solution and stopped using acid. The signal from the assay is proportional to the amount of dAb.

An ELISA plate was set up to analyse the samples listed below and also dAb1 and dAb 2 samples 'released' after 0 mins. From the removed released sample of 30µl - 21 µl was used for SDS PAGE analysis and 9µl was left to make dilutions of 1 in 100, 1 in 1000 and 1 in 10000.

A comparison of calculated total released dAb and functionally active dAb measured by ELISA is shown in Table 11.

Table 11 - Quantification of functionally active and total released dAb

| Sample | corrected dAb ng/ µl from SDS PAGE intensity conversion | corrected dAb ng/µl from functional ELISA | Percentage active dAb |
|---|---|---|---|
| dAb1 - 20 mins | 20.57143 | 33 | ~100 |
| dAb1 - 40 mins | 28.8 | 22 | 76 |
| dAb1 - 60 mins | 18.59048 | 30 | ~100 |
| dAb 2 - 20 mins | 18.28571 | 14 | 77 |
| dAb2 - 40 mins | 18.28571 | 11 | 61 |
| dAb2 - 60 mins | 21.33333 | 17 | 80 |

It can be seen that using this release procedure functionally active dAb can be detected from material released from microspheres at a range consistent with 60-100% of the encapsidated retaining activity as measured by ELISA. Total versus active dAb will fluctuate according to dAb released, heat inactivation or any degradation of dAb however, the variance is not considered to be significantly different.

## Claims

1. A particulate carrier comprising a biologically active agent obtainable by the steps of:

    a) mixing a biologically active agent in an aqueous phase with a hydrophobic ion pairing (HIP) agent in an organic solvent phase to form a biologically active agent-HIP complex;
    b) separation of the complex from the aqueous phase.
    c) removal of the aqueous phase and homogenisation of the complex with the organic phase;
    d)

        (i) dissolving a polymer in the organic phase formed in (c) and then forming an emulsion of the organic phase in a continuous aqueous phase; or
        (ii) dissolving a monomer or oligomer of a polymer forming substance, in the organic phase formed in (c) and then forming an emulsion of the organic phase in a continuous aqueous phase; to allow polymerisation of the monomer or oligomer to form a polymer; and

    e) obtaining a particulate carrier formed from the emulsion of step (d);

    for use in the treatment or prophylaxis of a disease or disorder of the eye.

2. The particulate carrier as claimed in claim 1 wherein the polymer comprises polycaprolactone (PCL), poly-L-lactide (PLA), poly butylcyanoacrylate (PBCA) or poly(lactide-co-glycolide)(PLG), or poly(hydroxybutyrate) and/or copolymers and blends thereof.

3. The particulate carrier as claimed in claim 1, wherein the polymer is selected from the group consisting of: poly (dienes) such as poly(butadiene) and the like; poly(alkenes) such as polyethylene, polypropylene, and the like; poly (acrylics) such as poly(acrylic acid) and the like; poly(methacrylics) such as poly(methyl methacrylate), poly(hydroxyethylmethacrylate), and the like; poly(vinyl ethers); poly(vinyl alcohols); poly(vinylketones); poly(vinylhalides) such as poly(vinyl chloride) and the like; poly(vinyl nitriles), poly(vinyl esters) such as poly(vinyl acetate) and the like; poly (vinyl pyridines) such as poly(2-vinyl pyridine), poly(5-methyl-2-vinyl pyridine) and the like; poly(styrenes); poly (carbonates); poly(esters); poly(orthoesters); poly(esterarnides); poly(anhydrides); poly(urethanes); poly(amides); polyacrylates, polymethacrylates, polybutylcyanoacrylates, polyalkylcyanoacrylates, polyarylamides, polyanhydrates, polyorthoesters, N,N-L-lysinediylterephthalate, polyanhydrates, polyglutaraldehydes and derivatives, and copolymers and polymer blends thereof.

4. The particulate carrier of claim 1 wherein the monomer comprises alkylcyanoacrylate (ACA) optionally butylcyanoacrylate (BCA).

5. The particulate carrier of claim 2 wherein the continuous aqueous phase has a pH of about 7 or higher when the protein is anionic and the HIP agent is cationic or a pH of about 7 or lower when the protein is cationic and the HIP agent is anionic.

6. The particulate carrier of any one of claims 1 to 5 wherein the particulate carrier is a microsphere.

7. The particulate carrier of any one of claims 1 to 6 wherein the biologically active agent is a protein or peptide optionally wherein the biologically active agent is an antigen binding molecule, optionally wherein the antigen binding molecule comprises a domain, is an antibody, or is a domain antibody.

8. The particulate carrier of any preceding claim wherein the biologically active agent is insoluble in the organic phase without the presence of hydrophobic ion pairing agents.

9. The particulate carrier of any one of claims 1 to 8, wherein the HIP agent is an anionic HIP agent when the biologically active agent is cationic, optionally wherein the HIP agent is docusate sodium, the biologically active agent is a protein and the HIP agent is present in stoichiometric amounts equal to or greater than the number of net positive charges on the protein.

10. The particulate carrier of any one of claims 1 to 8, wherein the HIP agent is a cationic HIP agent when the biologically active agent is anionic, optionally wherein the HIP agent is dimethyldioctadecyl-ammonium bromide (DDAB18); 1,2-dioleoyloxy - 3(trimethylammonium)propane (DOTAP); or cetrimonium bromide (CTAB), the biologically active agent is a protein and the HIP agent is present in stoichiometric amounts equal to or greater than the number of net negative charges on the protein.

**11.** A particulate carrier according to claim 6 wherein the protein to polymer ratio is at least about 1.0% w/w or is at least about 2.5% w/w, or is at least about 5% w/w.

**12.** A particulate carrier according to claim 6 wherein the peptide to polymer ratio is at least about 9% w/w.

**13.** A particulate carrier according to claim 6 wherein the antibody to polymer ratio is at least about 2% w/w.

**14.** A particulate carrier according to claim 6 wherein the domain antibody to polymer ratio is at least about 2.5% w/w.

**15.** The particulate carrier as claimed in anyone of the preceding claims wherein the polymer is pegylated.

**16.** A pharmaceutical composition comprising the particulate carriers of any one of the preceding claims.

**17.** Use of a particulate carrier as claimed in any one of claims 1 to 15, or a pharmaceutical composition as claimed in claim 16 for the treatment or prophylaxis of a disease or disorder of the eye.

**18.** A use as claimed in claim 17, wherein the disease or disorder of the eye is age related macular degeneration (wet AMD or dry AMD), diabetic retinopathy, retinal vein occlusive disease, uveitis, corneal neovascularisation or glaucoma.

**19.** A particulate carrier as claimed in any one of claims 1 to 15 or a pharmaceutical composition as claimed in claim 16, wherein the particulate carrier or composition is to be administered by ocular delivery, optionally wherein the ocular delivery is periocular, for example trans-scleral, subconjunctival, sub-tenon, peribulbar, topical, retrobulbar or is delivered to the inferior, superior or lateral rectus muscle or wherein the ocular delivery is intravitreal.

## Figures

Figure 1

(1a)

PBCA de dAb formulation (Combined)

| | |
|---|---|
| **Effective Diameter:** | 262.6 nm |
| **Polydispersity:** | 0.252 |
| **Avg. Count Rate:** | 2.9 Mcps |
| **Baseline Index:** | 9.1 |
| **Elapsed Time:** | 00:10:00 |

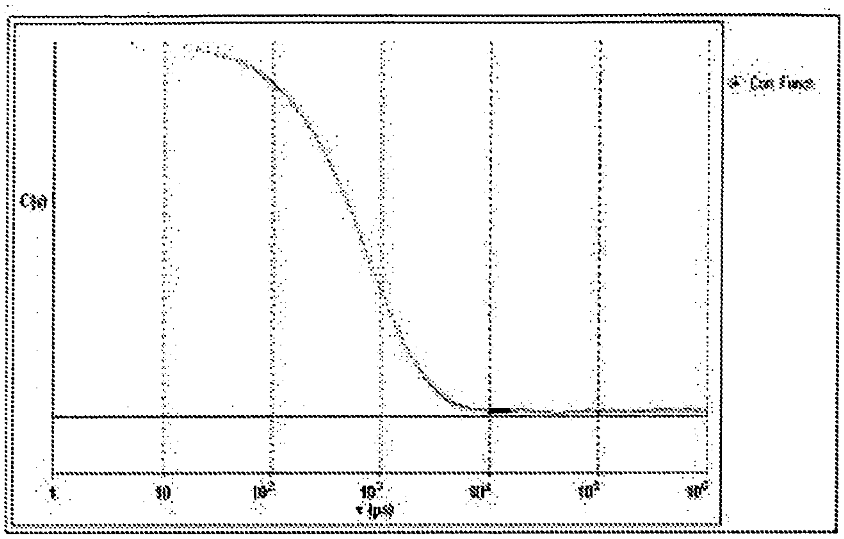


| Run | Eff. Diam. (nm) | Half Width (nm) | Polydispersity | Baseline Index |
|---|---|---|---|---|
| 1 | 300.1 | 150.9 | 0.243 | 9.3 |
| 2 | 281.8 | 138.8 | 0.243 | 9.3 |
| 3 | 263.7 | 134.5 | 0.260 | 9.3 |
| 4 | 248.0 | 124.8 | 0.251 | 7.4 |
| 5 | 250.0 | 125.8 | 0.252 | 9.3 |
| 6 | 254.5 | 132.0 | 0.273 | 9.6 |
| 7 | 258.4 | 135.0 | 0.263 | 9.1 |
| 8 | 259.3 | 134.5 | 0.270 | 10.0 |
| 9 | 266.9 | 139.7 | 0.274 | 9.5 |
| 10 | 255.2 | 134.9 | 0.280 | 9.6 |
| Mean | 263.4 | 135.1 | 0.260 | 9.2 |
| Std Error | 5.5 | 2.4 | 0.004 | 0.2 |
| Combined | 262.6 | 134.4 | 0.252 | 9.1 |

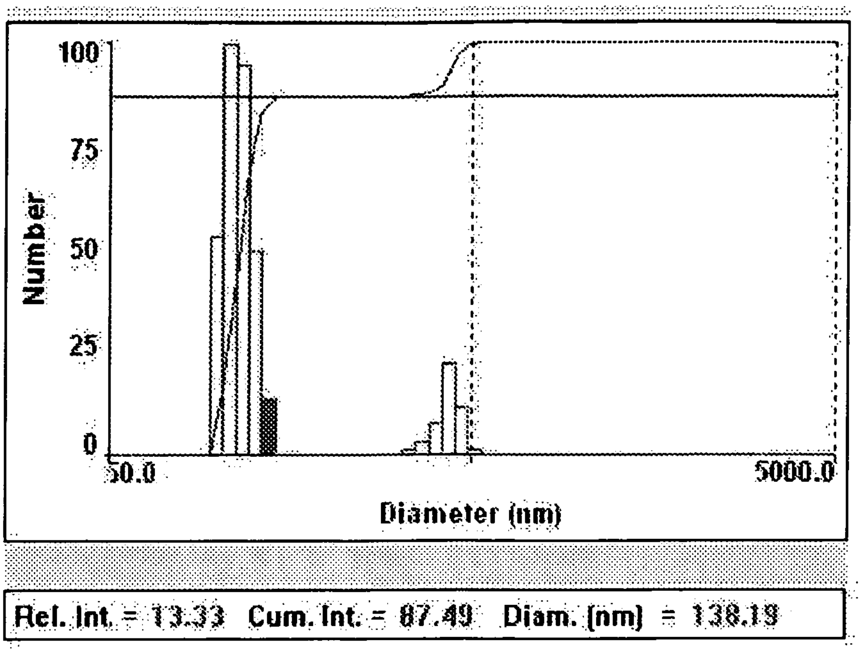
100
75
50
25
0
Number
50.0
5000.0
Diameter (nm)
Rel. Int. = 13.33 Cum. Int. = 87.49 Diam. (nm) = 138.19

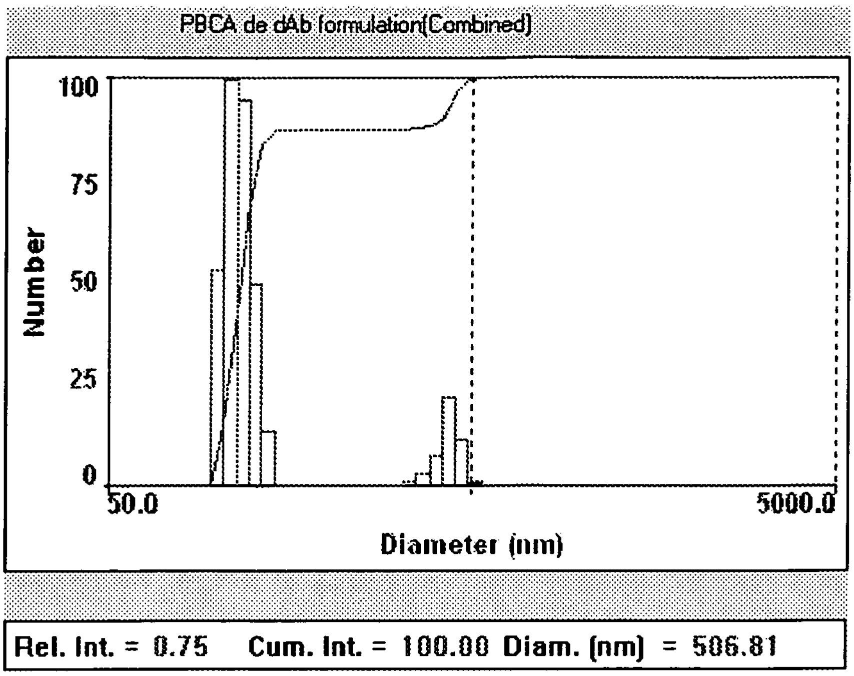
PBCA de dAb formulation(Combined)
100
75
50
25
0
Number
50.0
5000.0
Diameter (nm)
Rel. Int. = 0.75 Cum. Int. = 100.00 Diam. (nm) = 506.81

1d)

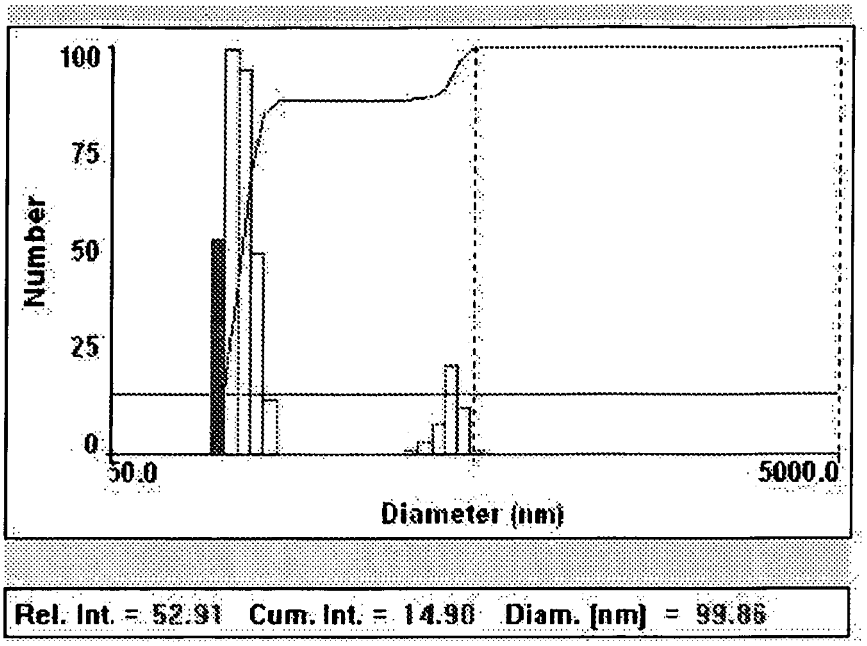
100
75
50
25
0
Number
50.0
5000.0
Diameter (nm)
Rel. Int. = 52.91 Cum. Int. = 14.90 Diam. [nm] = 99.86

Figure 2

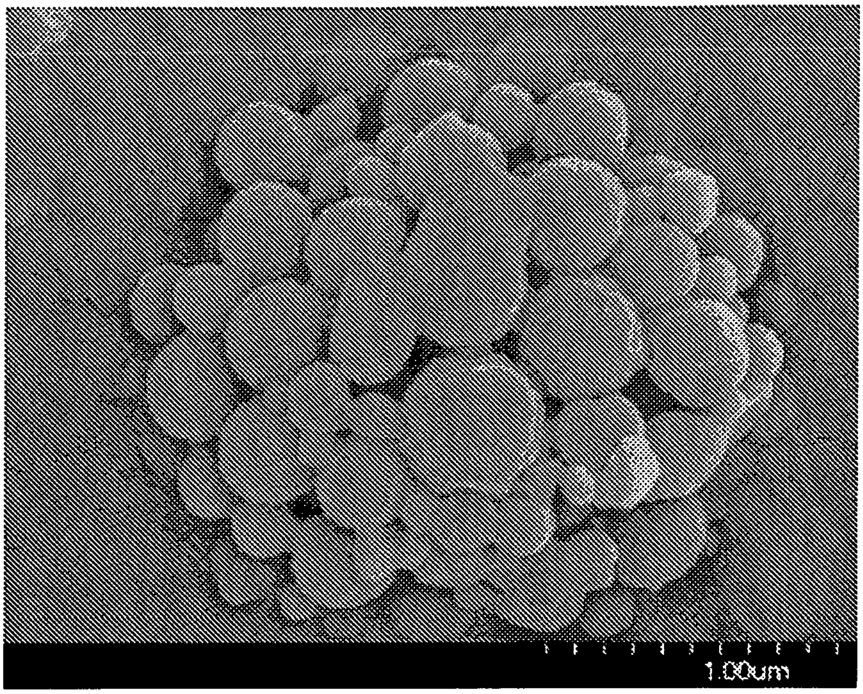

Figure 3

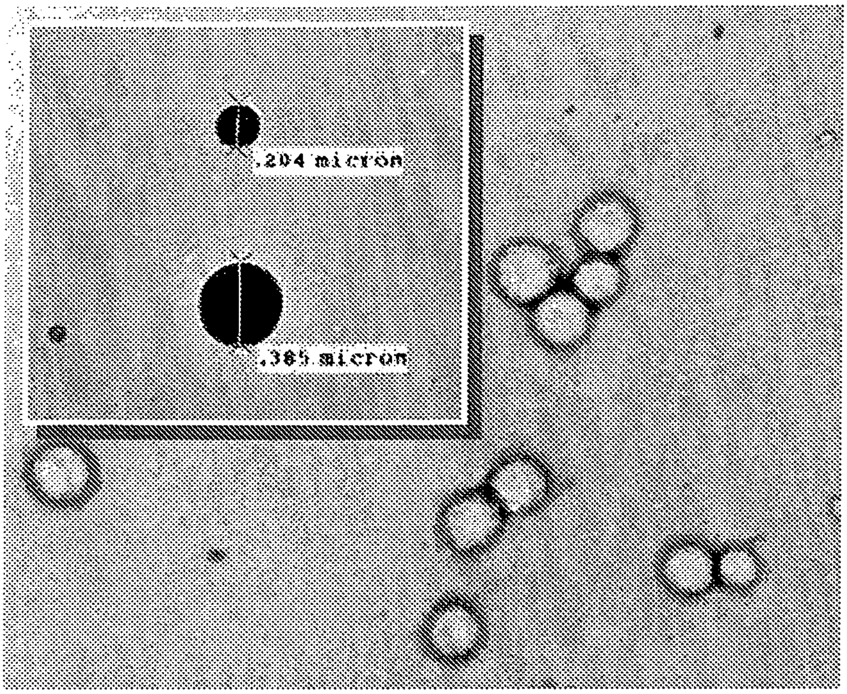

Figure 4

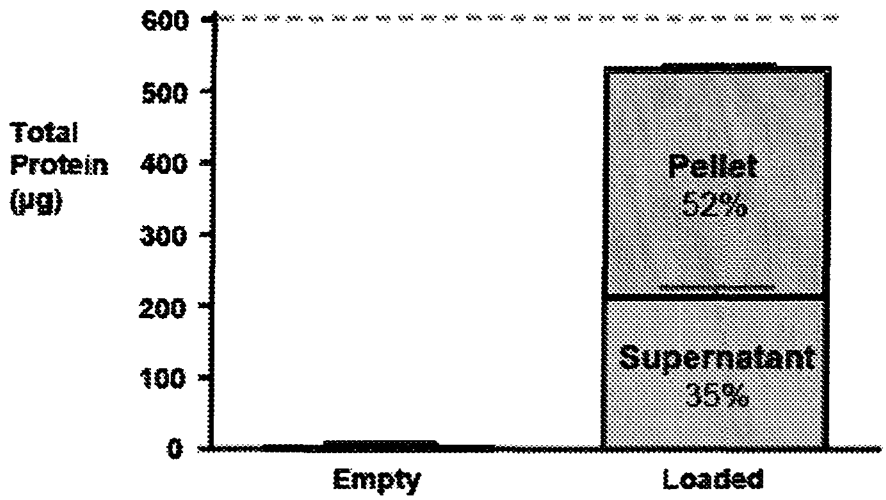
Total Protein (µg)
600
500
400
300
200
100
0
Pellet
52%
Supernatant
35%
Empty
Loaded

Figure 5

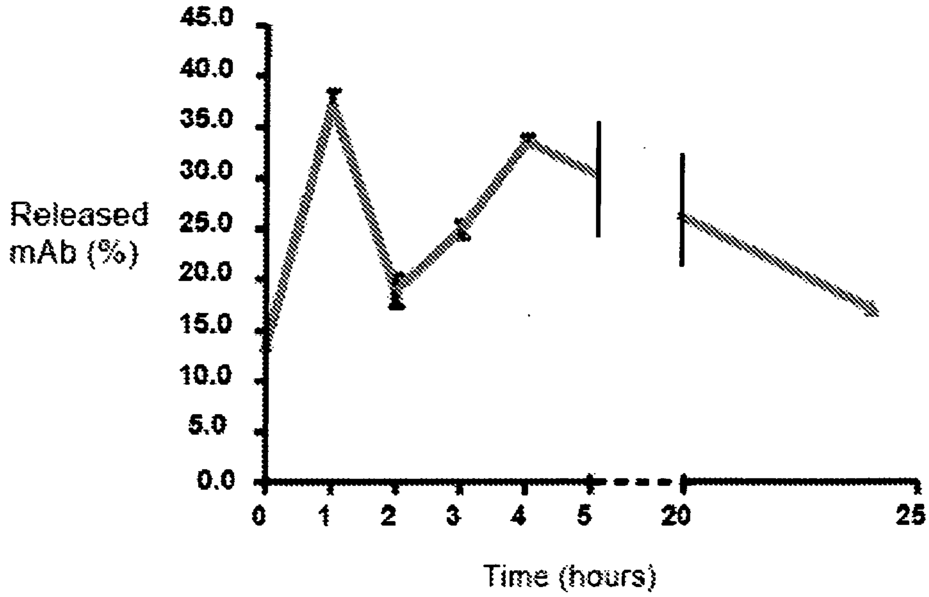


Figure 6

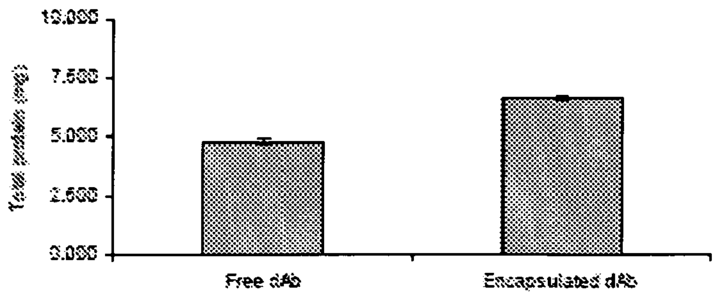

Figure 7

(7a)

PBCA np no dalargin, 10:1 HIP (Combined)

**Effective Diameter:** 291.4 nm
**Polydispersity:** 0.242
**Avg. Count Rate:** 1.9 Mcps
**Baseline Index:** 6.4
**Elapsed Time:** 00:10:00

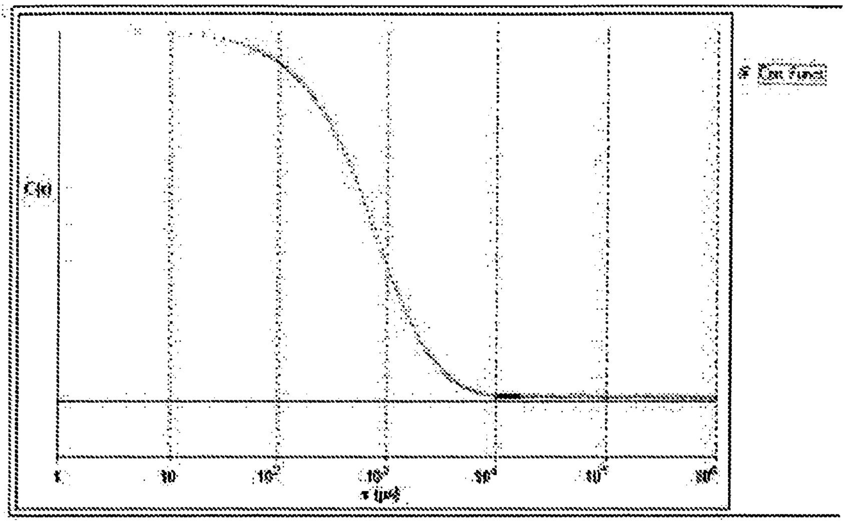


| Run | Eff. Diam. (nm) | Half Width (nm) | Polydispersity | Baseline Index |
|---|---|---|---|---|
| 1 | 302.4 | 135.1 | 0.200 | 8.8 |
| 2 | 290.7 | 151.0 | 0.270 | 10.0 |
| 3 | 281.0 | 121.7 | 0.205 | 7.8 |
| 4 | 293.1 | 148.2 | 0.259 | 9.2 |
| 5 | 298.4 | 149.4 | 0.266 | 9.9 |
| 6 | 289.2 | 146.9 | 0.251 | 5.6 |
| 7 | 320.1 | 138.9 | 0.188 | 9.7 |
| 8 | 281.3 | 133.7 | 0.226 | 7.9 |
| 9 | 283.6 | 143.1 | 0.238 | 9.7 |
| 10 | 282.7 | 147.5 | 0.272 | 7.4 |
| Mean | 294.3 | 142.6 | 0.237 | 8.5 |
| Std. Error | 3.5 | 2.3 | 0.010 | 0.3 |
| Combined | 291.4 | 143.2 | 0.242 | 6.4 |

(7b)

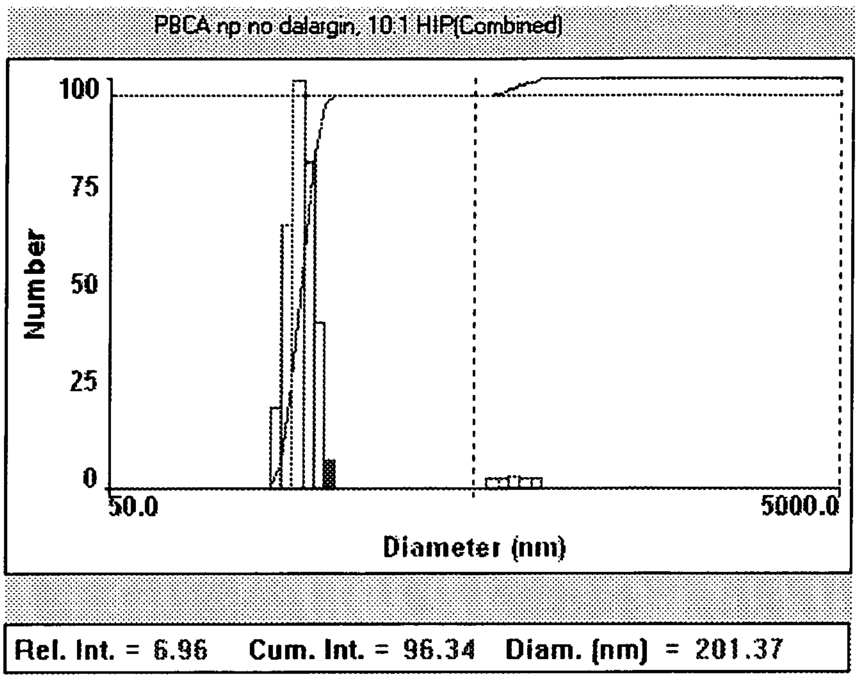
PBCA np no dalargin, 10:1 HIP(Combined)
100
75
50
25
0
Number
50.0
5000.0
Diameter (nm)
Rel. Int. = 6.96 Cum. Int. = 96.34 Diam. (nm) = 201.37

7c

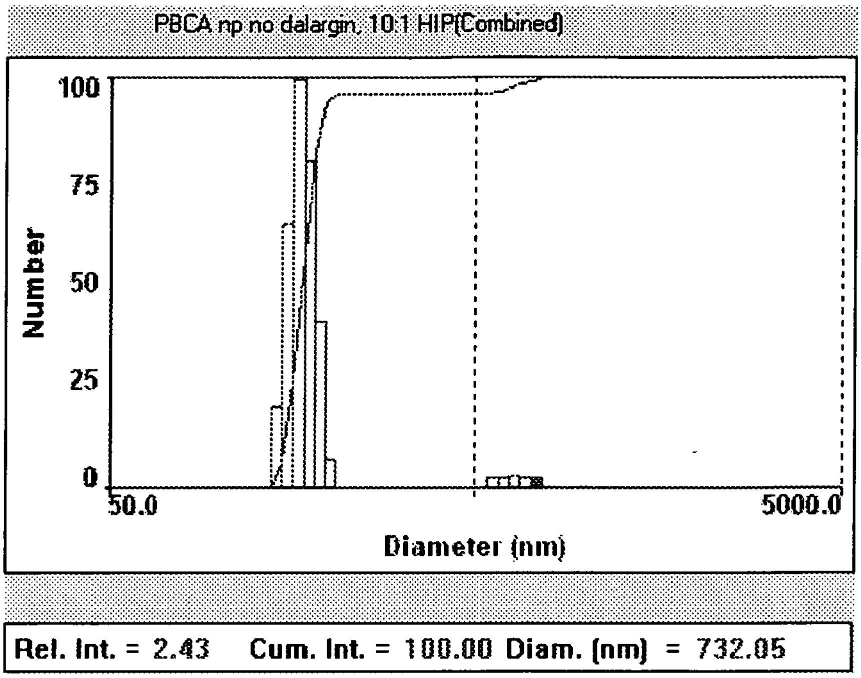
PBCA np no dalargin, 10:1 HIP(Combined)
100
75
50
25
0
Number
50.0
5000.0
Diameter (nm)
Rel. Int. = 2.43 Cum. Int. = 100.00 Diam. (nm) = 732.05

7d

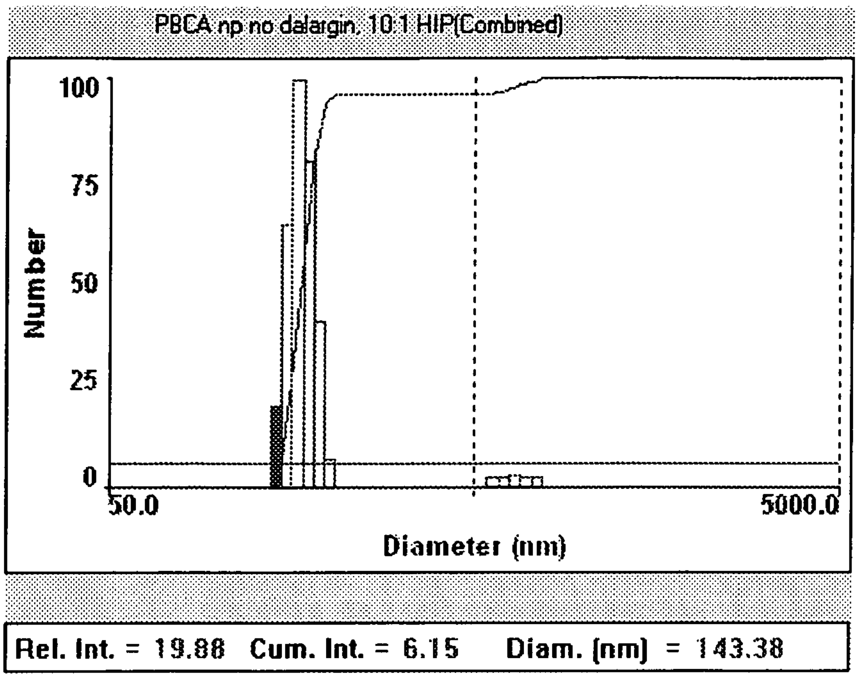
PBCA np no dalargin. 10:1 HIP(Combined)
100
75
50
25
0
Number
50.0
5000.0
Diameter (nm)
Rel. Int. = 19.88 Cum. Int. = 6.15 Diam. (nm) = 143.38

Figure 8.

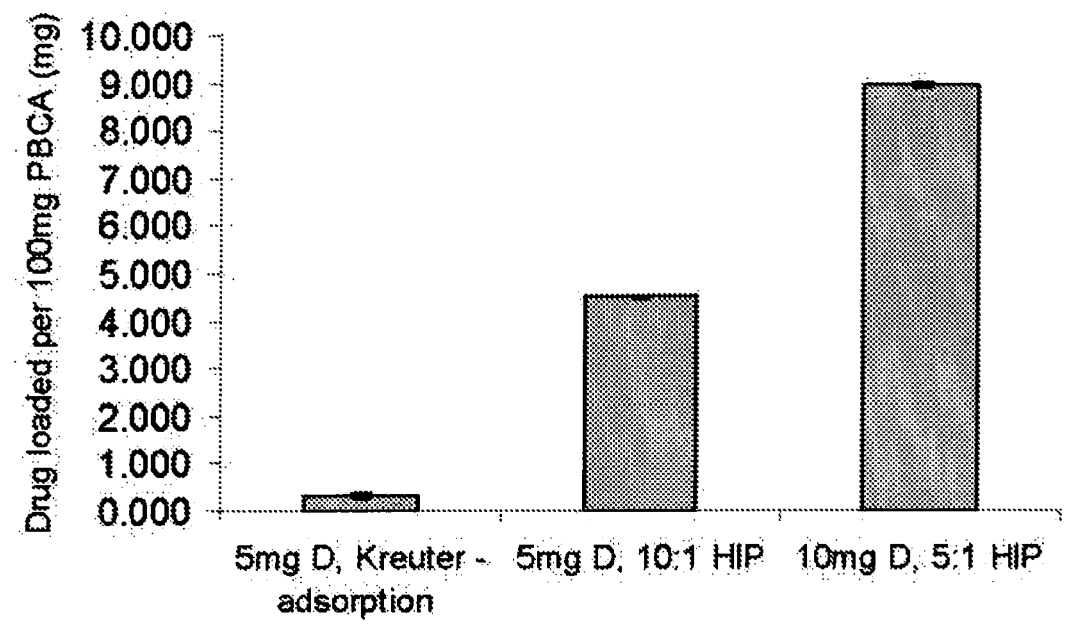


Figure 9

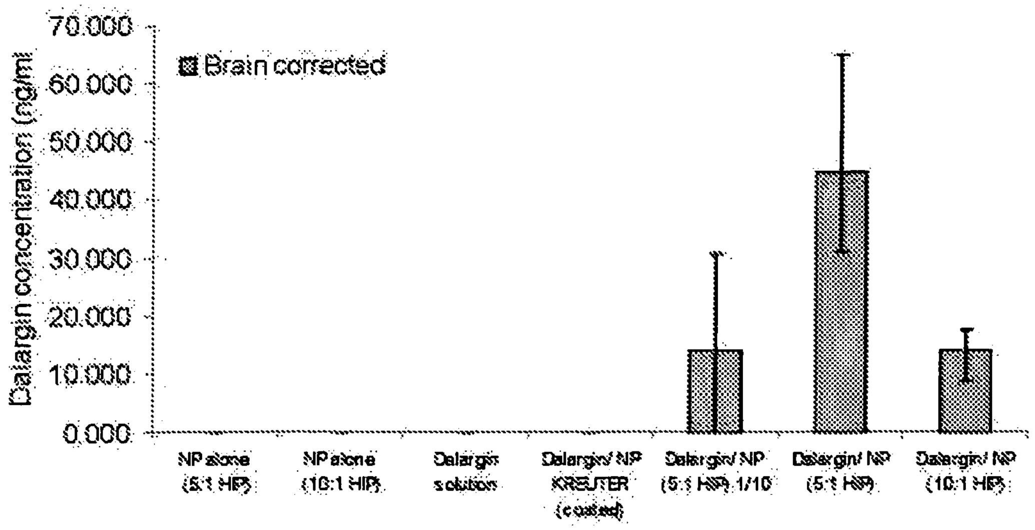

Figure 10

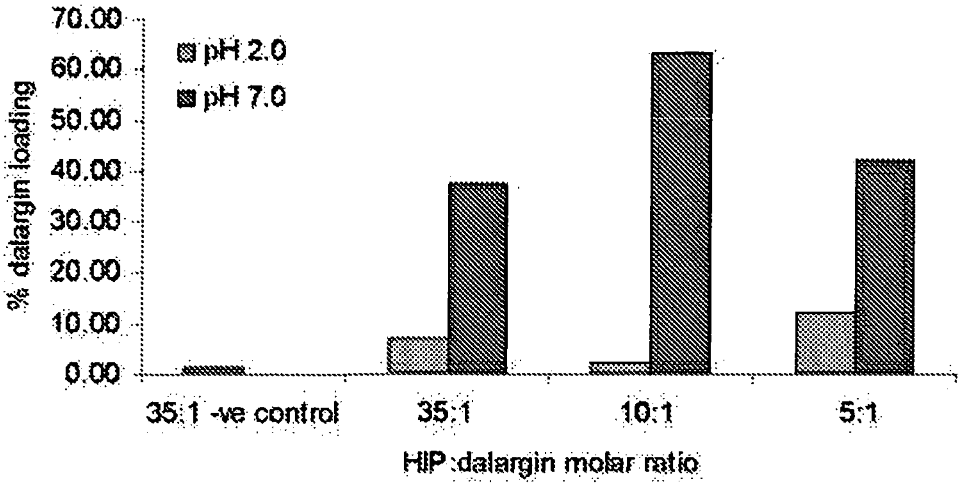


Figure 11

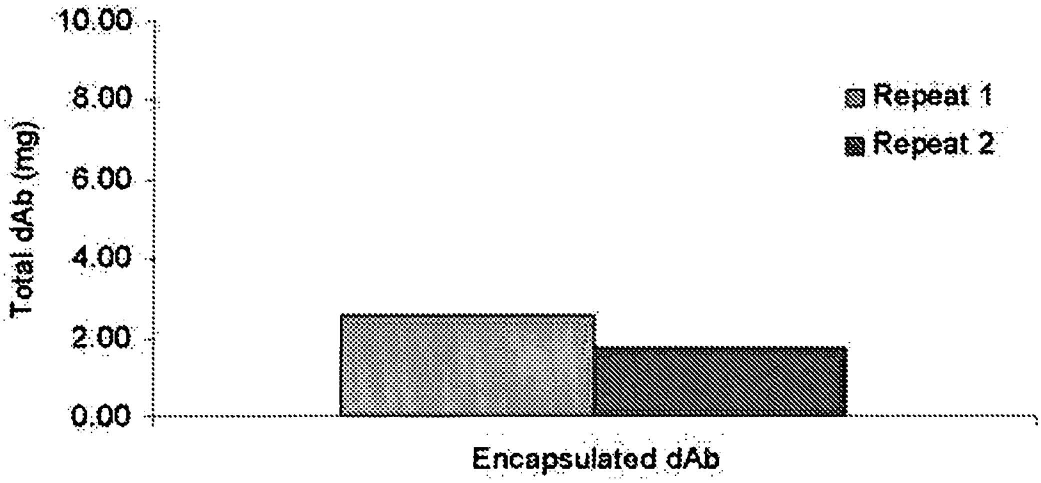

Figure 12.

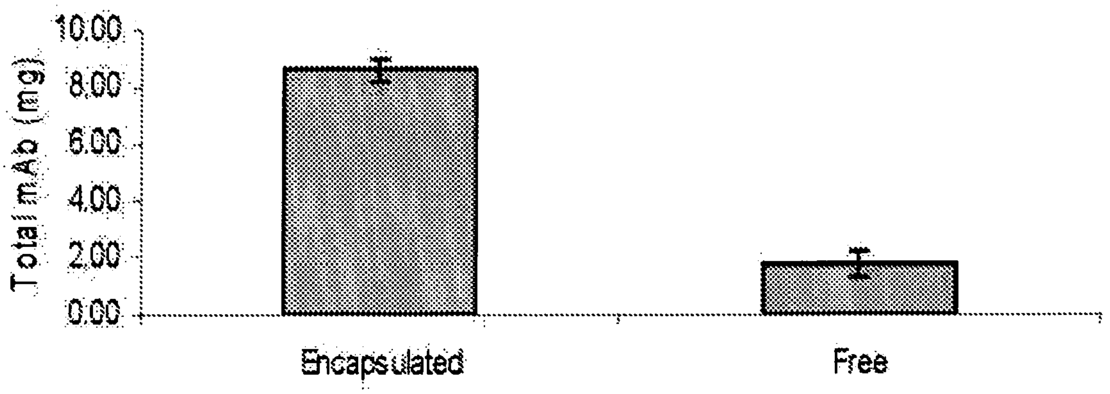

Figure 13.

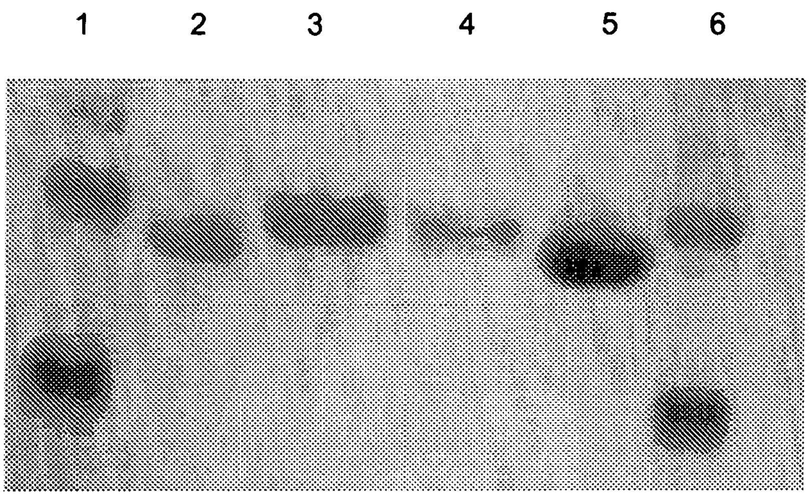

Figure 14

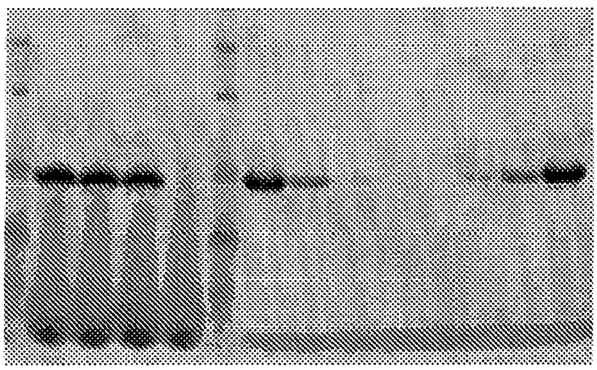

Figure 15

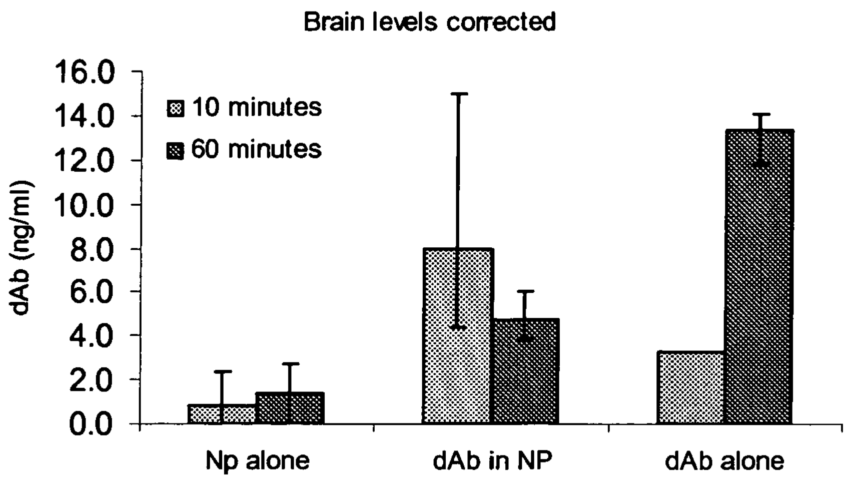
Brain levels corrected
16.0
14.0
12.0
10.0
8.0
6.0
4.0
2.0
0.0
dAb (ng/ml)
10 minutes
60 minutes
Np alone
dAb in NP
dAb alone

Figure 16

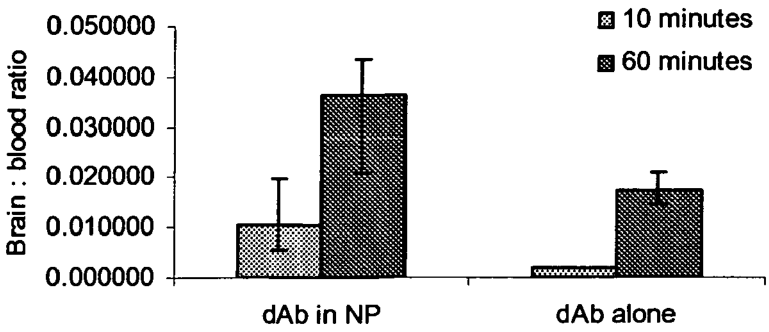
0.050000
0.040000
0.030000
0.020000
0.010000
0.000000
Brain : blood ratio
10 minutes
60 minutes
dAb in NP
dAb alone

Figure 17

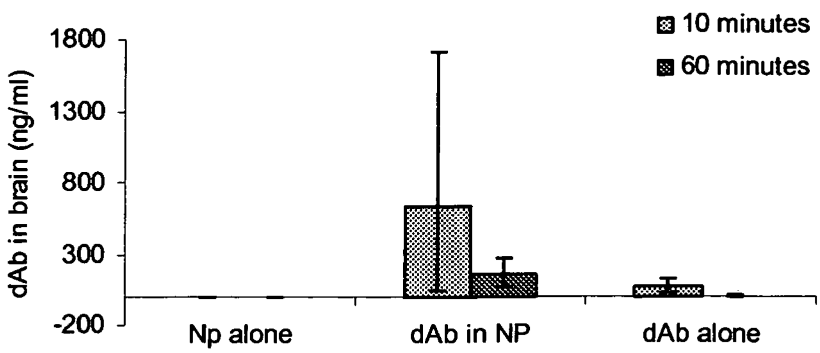


Figure 18

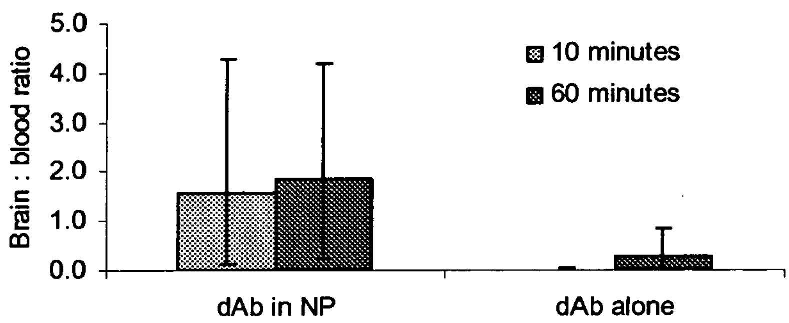

Figure 19.

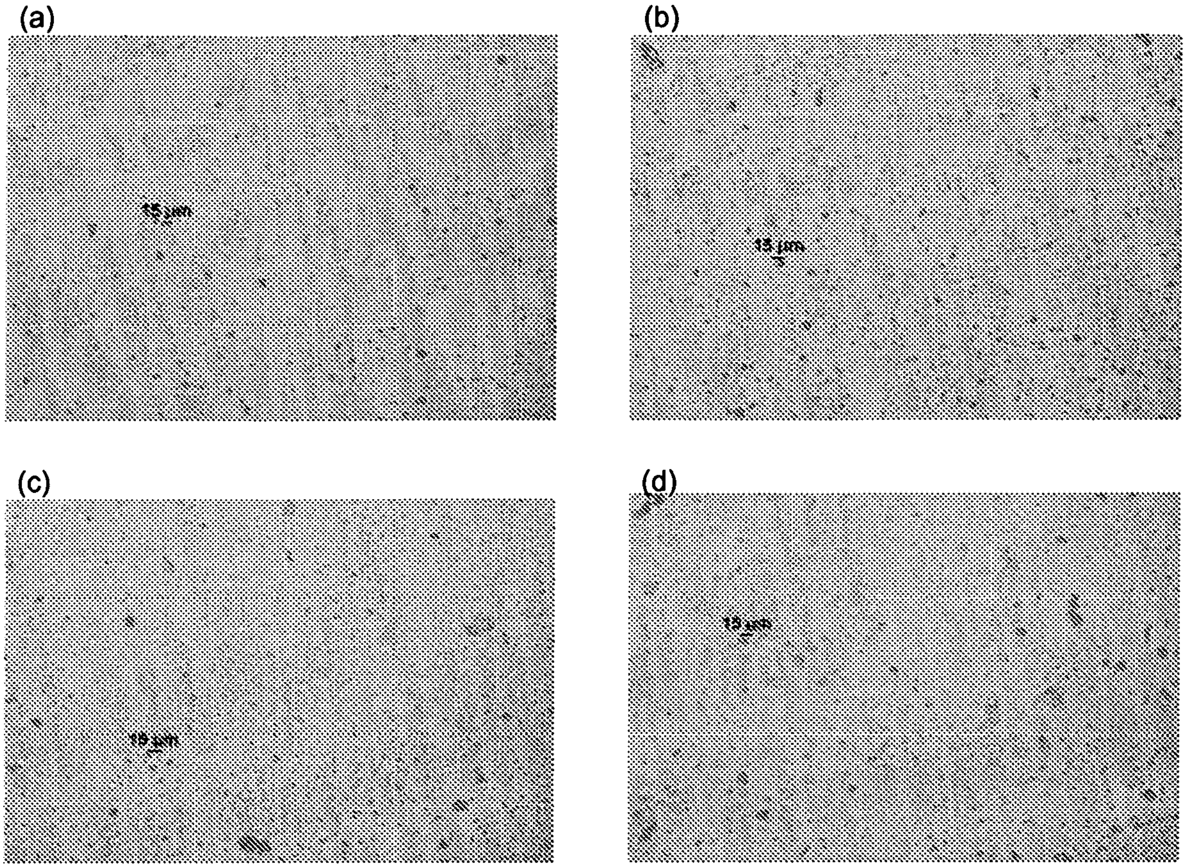

Figure 20

(a)

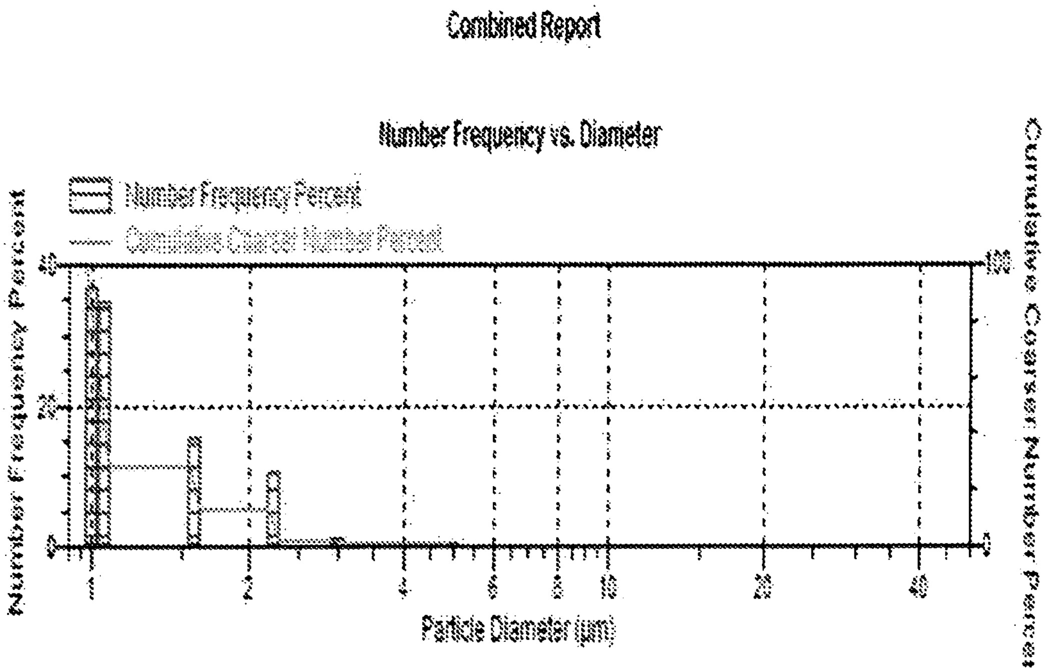
Combined Report
Number Frequency vs. Diameter
Number Frequency Percent
Cumulative Coarser Number Percent
Number Frequency Percent
Cumulative Coarser Number Percent
Particle Diameter (µm)

(b)

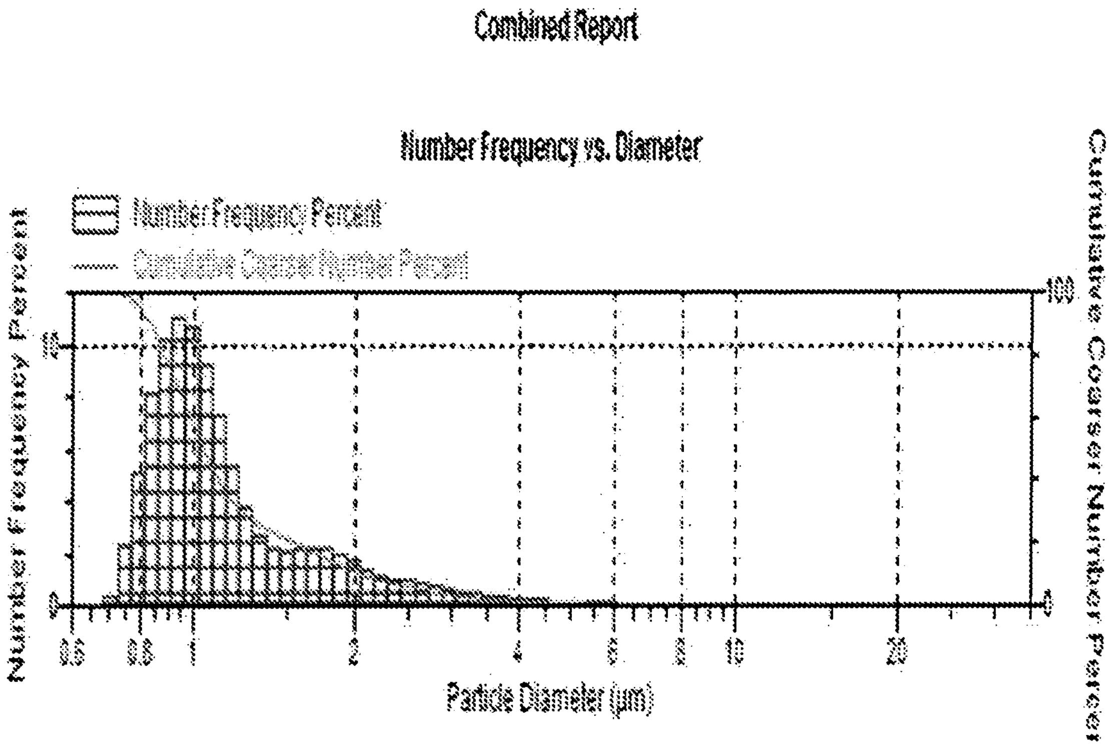
Combined Report
Number Frequency vs. Diameter
Number Frequency Percent
Cumulative Coarser Number Percent
Number Frequency Percent
Cumulative Coarser Number Percent
10
0
100
0
0.6
0.8
1
2
4
6
8
10
20
Particle Diameter (μm)

(c)

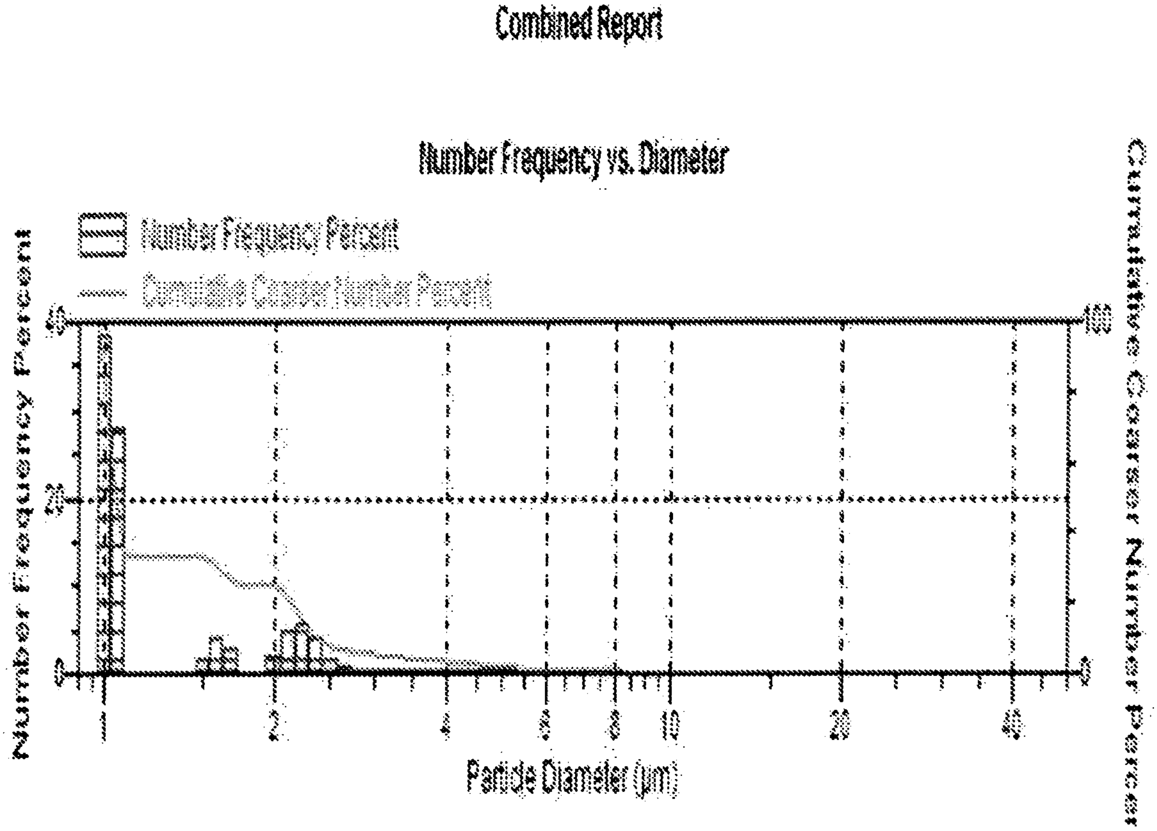
Combined Report
Number Frequency vs. Diameter
Number Frequency Percent
Cumulative Coarser Number Percent
Number Frequency Percent
Cumulative Coarser Number Percent
40
20
0
100
0
1
2
4
6
8
10
20
40
Particle Diameter (µm)

(d)

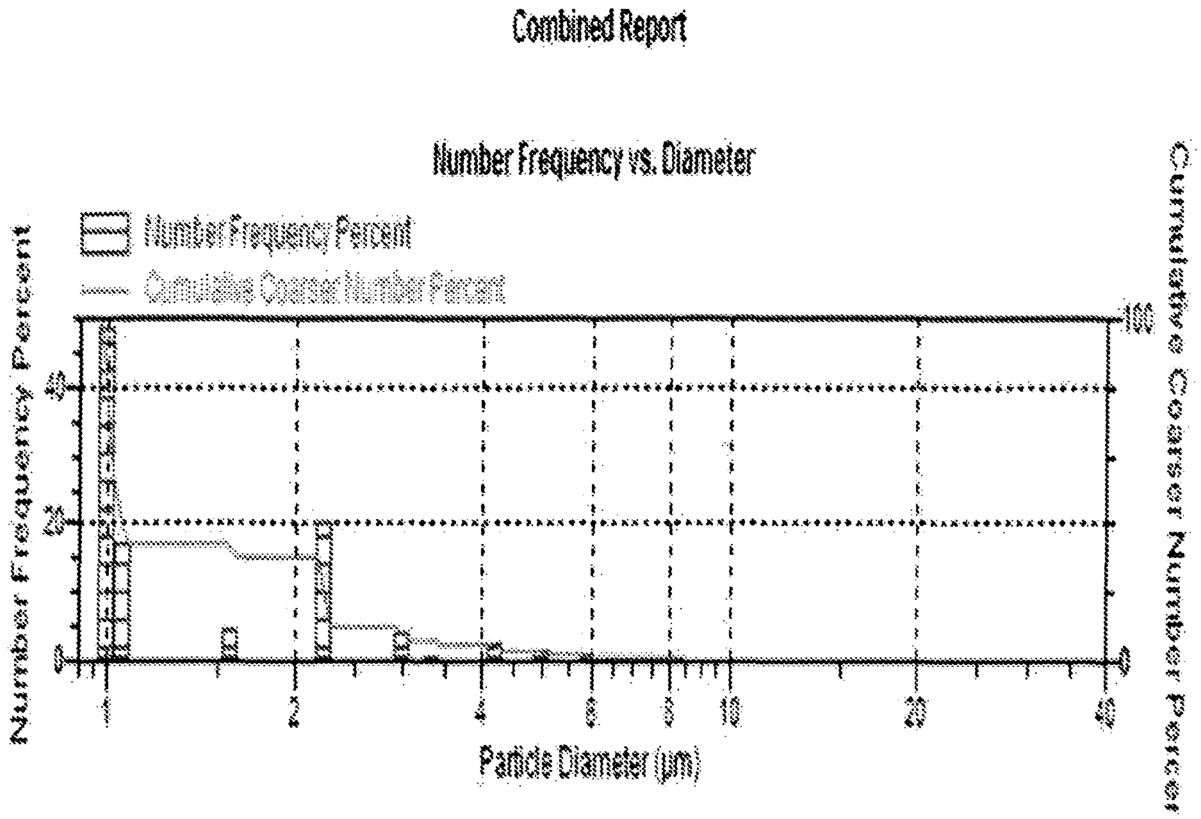
Combined Report
Number Frequency vs. Diameter
Number Frequency Percent
Cumulative Coarser Number Percent
Number Frequency Percent
Cumulative Coarser Number Percent
0
20
40
100
0
1
2
4
6
8
10
20
40
Particle Diameter (µm)

Figure 21.

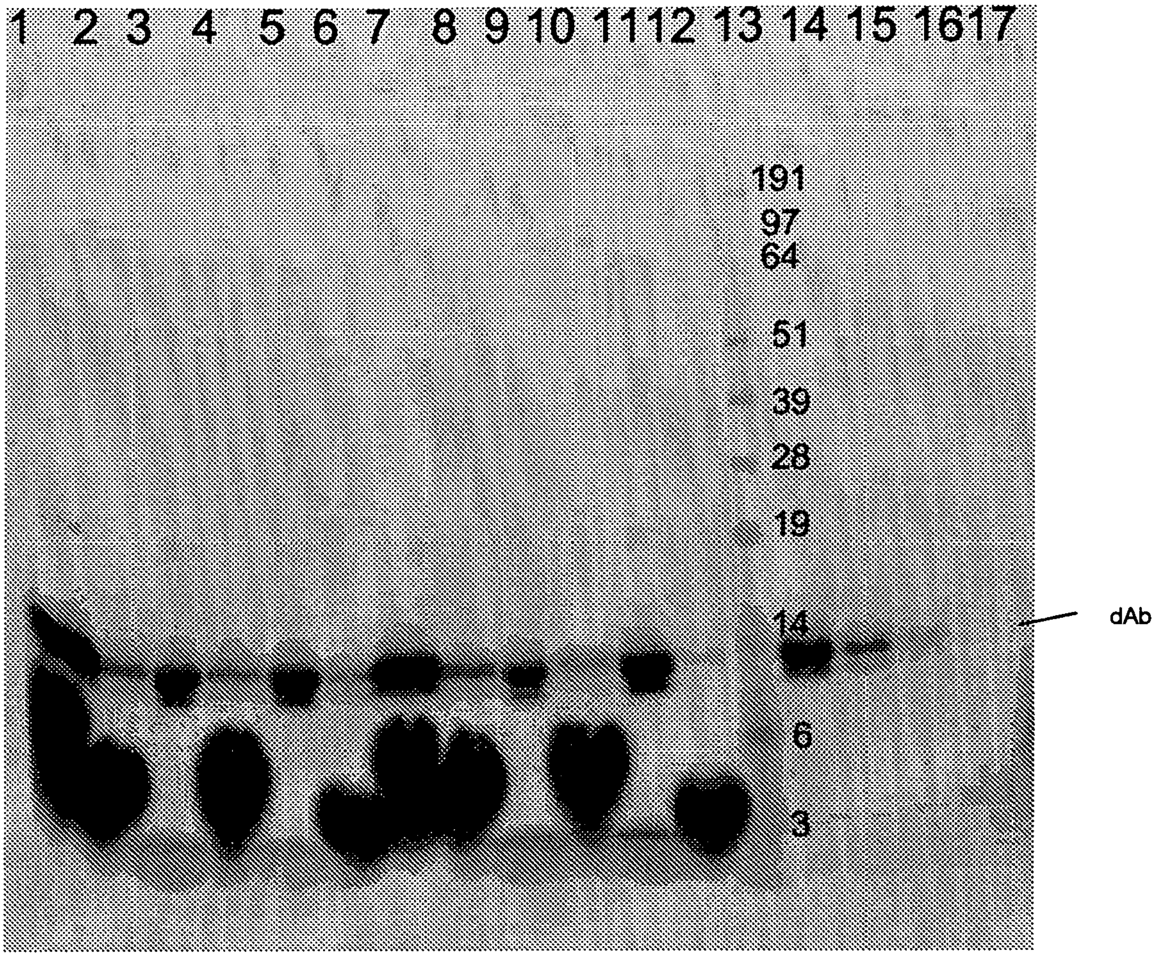

Figure 22.

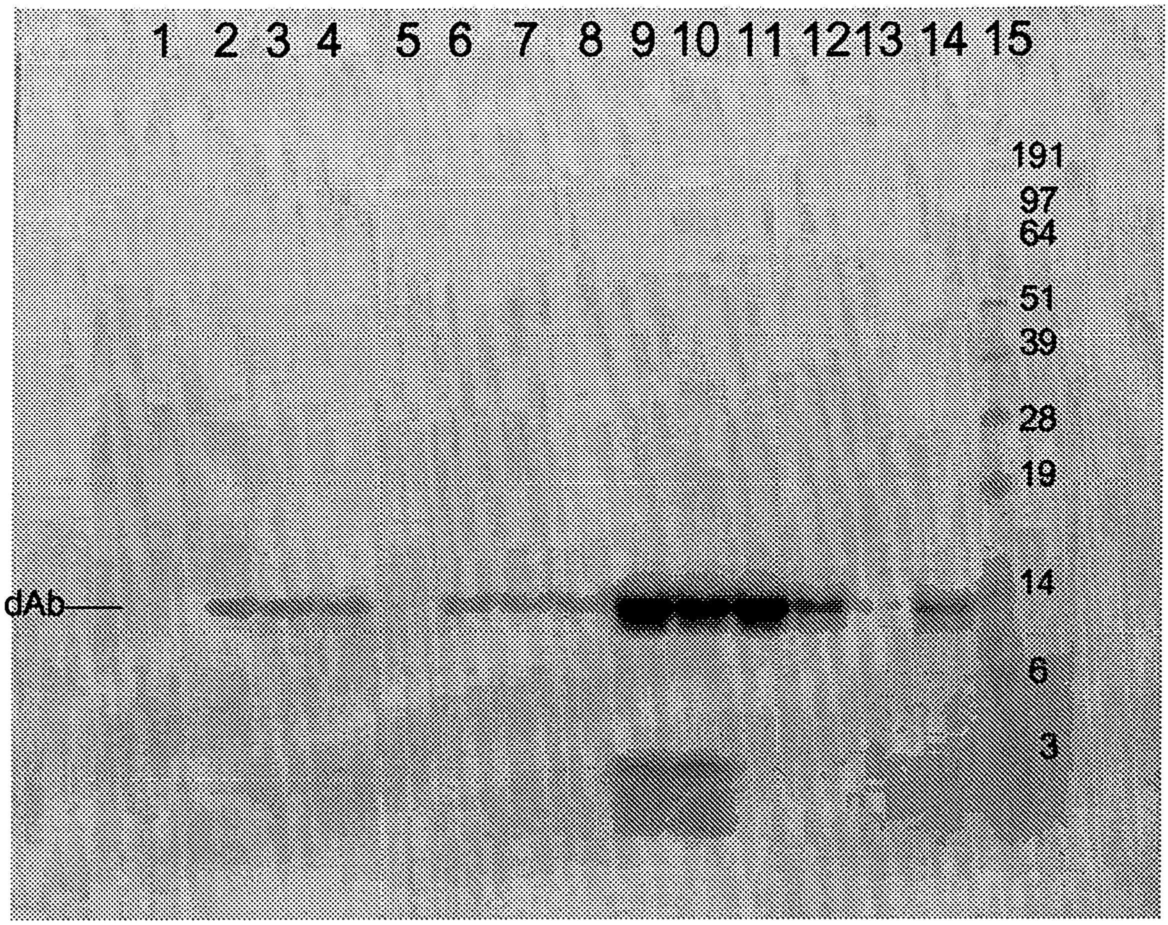

Europäisches Patentamt
European Patent Office
Office européen des brevets

# EUROPEAN SEARCH REPORT

Application Number
EP 11 19 2165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/005952 A2 (CORIXA CORP [US]) 23 January 2003 (2003-01-23) * page 2, line 20 - line 24; claims; examples * ----- | 1-19 | INV. A61K9/51 |
| A | US 6 284 280 B1 (WEITSCHIES WERNER [DE] ET AL) 4 September 2001 (2001-09-04) * column 1, line 13 - line 21 * * column 3, line 6 - line 31 * * column 4, line 8 - line 26 * * column 5, line 34 - column 6, line 17; claims; examples 1, 3, 7 * ----- | 1-19 | |
| A | VAUTHIER CHRISTINE ET AL: "Design aspects of poly(alkylcyanoacrylate) nanoparticles for drug delivery", JOURNAL OF DRUG TARGETING, HARWOOD ACADEMIC PUBLISHERS GMBH, DE, vol. 15, no. 10, 1 December 2007 (2007-12-01), pages 641-663, XP008126608, ISSN: 1061-186X, DOI: DOI:10.1080/10611860701603372 * page 649, left-hand column, line 29 - page 658, right-hand column, line 9 * ----- | 1-19 | TECHNICAL FIELDS SEARCHED (IPC) A61K |
| A | WO 94/17789 A1 (SHIELD RESEARCH LTD [IE]; DYATLOV VALERY A [RU]; KATZ GEORGY ARKADIEVI) 18 August 1994 (1994-08-18) * claims 1-5 10 11; examples 1-4 * ----- | 1-19 | |

The present search report has been drawn up for all claims

2

| Place of search | Date of completion of the search | Examiner |
|---|---|---|
| The Hague | 9 March 2012 | Couckuyt, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 11 19 2165

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2012

| Patent document cited in search report | Publication date | Patent family member(s) | Publication date |
|---|---|---|---|
| WO 03005952 A2 | 23-01-2003 | CA 2452382 A1 | 23-01-2003 |
| | | EP 1417236 A2 | 12-05-2004 |
| | | EP 2241309 A2 | 20-10-2010 |
| | | JP 2005514326 A | 19-05-2005 |
| | | NZ 530315 A | 26-01-2007 |
| | | US 2003108565 A1 | 12-06-2003 |
| | | US 2007148254 A1 | 28-06-2007 |
| | | WO 03005952 A2 | 23-01-2003 |
| US 6284280 B1 | 04-09-2001 | AT 197124 T | 15-11-2000 |
| | | AU 7655194 A | 27-03-1995 |
| | | CA 2171303 A1 | 16-03-1995 |
| | | DK 717617 T3 | 05-02-2001 |
| | | EP 0717617 A1 | 26-06-1996 |
| | | ES 2152990 T3 | 16-02-2001 |
| | | GR 3035219 T3 | 30-04-2001 |
| | | JP H09502191 A | 04-03-1997 |
| | | NO 960973 A | 08-03-1996 |
| | | PT 717617 E | 30-04-2001 |
| | | US 6068857 A | 30-05-2000 |
| | | US 6284280 B1 | 04-09-2001 |
| | | WO 9507072 A2 | 16-03-1995 |
| WO 9417789 A1 | 18-08-1994 | AU 3462193 A | 29-08-1994 |
| | | EP 0683667 A1 | 29-11-1995 |
| | | WO 9417789 A1 | 18-08-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006029845 A
- US 4833666 A, sable
- US 7250297 B1
- US 20070224633 A
- EP 1641818 A1
- US 20040132028 A1
- US 20080139791 A
- WO 2005056764 A
- US 6818418 B1
- WO 2008098796 A
- WO 2007068750 A
- WO 2004014953 A
- WO 2007113172 A
- WO 0029004 A
- WO 9958679 A
- WO 2008149147 A
- WO 2008149146 A


### Non-patent literature cited in the description


- **SAIJA A et al.** *J Pharm. Pha.,* 1990, vol. 42, 135-138
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- Handbook of Therapeutic Antibodies. 2007
- *Protein Science,* 2006, vol. 15, 14-27
- **KABAT et al.** *Sequences of proteins of Immunological Interest NI H,* 1987
- **AL-LAZIKANI et al.** *JMB,* 1997, vol. 273, 927-948
- **MACCALLUM R.M. ; MARTIN A.C.R. ; THORNTON J.M.** *Journal of Molecular Biology,* 1996, vol. 262 (5), 732-745

SEQUENCE LISTING

<110> PAPANICOLOAU, Irene
CATCHPOLE, Ian
GOUGH, Gerald

<120> Encapsulation of biologically active agents

<130> PB63619

<150> US61/050775
<151> 2008-05-06

<150> US61/074171
<151> 2008-06-20

<160> 15

<170> FastSEQ for Windows Version 4.0

<210> 1
<211> 462
<212> PRT
<213> Artificial Sequence

<220>
<223> Humanised sequence of mus musculus and homo sapiens

<400> 1

```
Met Gly Trp Ser Cys Ile Ile Leu Phe Leu Val Ala Thr Ala Thr Gly
 1               5                  10                  15
Val His Ser Gln Val Gln Leu Val Gln Ser Gly Ala Glu Val Lys Lys
            20                  25                  30
Pro Gly Ala Ser Val Lys Val Ser Cys Lys Ala Ser Gly Tyr Thr Phe
        35                  40                  45
Thr Ser Tyr Trp Met His Trp Val Arg Gln Ala Pro Gly Gln Gly Leu
    50                  55                  60
Glu Trp Ile Gly Asn Ile Asn Pro Ser Asn Gly Gly Thr Asn Tyr Asn
65                  70                  75                  80
Glu Lys Phe Lys Ser Lys Ala Thr Met Thr Arg Asp Thr Ser Thr Ser
                85                  90                  95
Thr Ala Tyr Met Glu Leu Ser Ser Leu Arg Ser Glu Asp Thr Ala Val
            100                 105                 110
Tyr Tyr Cys Glu Leu Met Gln Gly Tyr Trp Gly Gln Gly Thr Leu Val
        115                 120                 125
Thr Val Ser Ser Ala Ser Thr Lys Gly Pro Ser Val Phe Pro Leu Ala
    130                 135                 140
Pro Ser Ser Lys Ser Thr Ser Gly Gly Thr Ala Ala Leu Gly Cys Leu
145                 150                 155                 160
Val Lys Asp Tyr Phe Pro Glu Pro Val Thr Val Ser Trp Asn Ser Gly
                165                 170                 175
Ala Leu Thr Ser Gly Val His Thr Phe Pro Ala Val Leu Gln Ser Ser
            180                 185                 190
Gly Leu Tyr Ser Leu Ser Ser Val Val Thr Val Pro Ser Ser Ser Leu
        195                 200                 205
Gly Thr Gln Thr Tyr Ile Cys Asn Val Asn His Lys Pro Ser Asn Thr
    210                 215                 220
Lys Val Asp Lys Lys Val Glu Pro Lys Ser Cys Asp Lys Thr His Thr
225                 230                 235                 240
Cys Pro Pro Cys Pro Ala Pro Glu Leu Ala Gly Ala Pro Ser Val Phe
                245                 250                 255
Leu Phe Pro Pro Lys Pro Lys Asp Thr Leu Met Ile Ser Arg Thr Pro
```

```
        260                 265                 270
Glu Val Thr Cys Val Val Val Asp Val Ser His Glu Asp Pro Glu Val
        275                 280                 285
Lys Phe Asn Trp Tyr Val Asp Gly Val Glu Val His Asn Ala Lys Thr
    290                 295                 300
Lys Pro Arg Glu Glu Gln Tyr Asn Ser Thr Tyr Arg Val Val Ser Val
305                 310                 315                 320
Leu Thr Val Leu His Gln Asp Trp Leu Asn Gly Lys Glu Tyr Lys Cys
                325                 330                 335
Lys Val Ser Asn Lys Ala Leu Pro Ala Pro Ile Glu Lys Thr Ile Ser
            340                 345                 350
Lys Ala Lys Gly Gln Pro Arg Glu Pro Gln Val Tyr Thr Leu Pro Pro
        355                 360                 365
Ser Arg Asp Glu Leu Thr Lys Asn Gln Val Ser Leu Thr Cys Leu Val
    370                 375                 380
Lys Gly Phe Tyr Pro Ser Asp Ile Ala Val Glu Trp Glu Ser Asn Gly
385                 390                 395                 400
Gln Pro Glu Asn Asn Tyr Lys Thr Thr Pro Pro Val Leu Asp Ser Asp
                405                 410                 415
Gly Ser Phe Phe Leu Tyr Ser Lys Leu Thr Val Asp Lys Ser Arg Trp
            420                 425                 430
Gln Gln Gly Asn Val Phe Ser Cys Ser Val Met His Glu Ala Leu His
        435                 440                 445
Asn His Tyr Thr Gln Lys Ser Leu Ser Leu Ser Pro Gly Lys
    450                 455                 460


<210> 2
<211> 238
<212> PRT
<213> Artificial Sequence

<220>
<223> Humanised sequence of mus musculus and homo
      sapiens

<400> 2
Met Gly Trp Ser Cys Ile Ile Leu Phe Leu Val Ala Thr Ala Thr Gly
 1               5                  10                  15
Val His Ser Asp Ile Val Met Thr Gln Ser Pro Leu Ser Asn Pro Val
            20                  25                  30
Thr Leu Gly Gln Pro Val Ser Ile Ser Cys Arg Ser Ser Lys Ser Leu
        35                  40                  45
Leu Tyr Lys Asp Gly Lys Thr Tyr Leu Asn Trp Phe Leu Gln Arg Pro
    50                  55                  60
Gly Gln Ser Pro Gln Leu Leu Ile Tyr Leu Met Ser Thr Arg Ala Ser
65                  70                  75                  80
Gly Val Pro Asp Arg Phe Ser Gly Gly Gly Ser Gly Thr Asp Phe Thr
                85                  90                  95
Leu Lys Ile Ser Arg Val Glu Ala Glu Asp Val Gly Val Tyr Tyr Cys
            100                 105                 110
Gln Gln Leu Val Glu Tyr Pro Leu Thr Phe Gly Gln Gly Thr Lys Leu
        115                 120                 125
Glu Ile Lys Arg Thr Val Ala Ala Pro Ser Val Phe Ile Phe Pro Pro
    130                 135                 140
Ser Asp Glu Gln Leu Lys Ser Gly Thr Ala Ser Val Val Cys Leu Leu
145                 150                 155                 160
Asn Asn Phe Tyr Pro Arg Glu Ala Lys Val Gln Trp Lys Val Asp Asn
                165                 170                 175
Ala Leu Gln Ser Gly Asn Ser Gln Glu Ser Val Thr Glu Gln Asp Ser
            180                 185                 190
Lys Asp Ser Thr Tyr Ser Leu Ser Ser Thr Leu Thr Leu Ser Lys Ala
        195                 200                 205
Asp Tyr Glu Lys His Lys Val Tyr Ala Cys Glu Val Thr His Gln Gly
    210                 215                 220
```

```
Leu Ser Ser Pro Val Thr Lys Ser Phe Asn Arg Gly Glu Cys
225                 230                 235


<210> 3
<211> 1389
<212> DNA
<213> Artificial Sequence

<220>
<223> Humanised sequence of mus musculus and homo
      sapiens

<400> 3
atgggatgga gctgtatcat cctcttcttg gtagcaacag ctacaggtgt ccactcccag 60
gtgcagctgg tgcagtctgg ggctgaggtg aagaagcctg gggcctcagt gaaggtttcc 120
tgcaaggcat ctggatacac cttcaccagc tactggatgc actgggtgcg acaggcccct 180
ggacaagggc ttgagtggat cggaaatatt aatcctagca atggtggtac taactacaat 240
gagaagttca agagcaaggc caccatgacc agggacacgt ccacgagcac agcctacatg 300
gagctgagca gcctgagatc tgaggacacg gccgtgtatt actgtgaact gatgcagggc 360
tactggggcc agggaacact agtcacagtc tcctcagcct ccaccaaggg cccatcggtc 420
ttccccctgg caccctcctc caagagcacc tctgggggca cagcggccct gggctgcctg 480
gtcaaggact acttccccga accggtgacg gtgtcgtgga actcaggcgc cctgaccagc 540
ggcgtgcaca ccttcccggc tgtcctacag tcctcaggac tctactccct cagcagcgtg 600
gtgaccgtgc cctccagcag cttgggcacc cagacctaca tctgcaacgt gaatcacaag 660
cccagcaaca ccaaggtgga caagaaagtt gagcccaaat cttgtgacaa aactcacaca 720
tgcccaccgt gcccagcacc tgaactcgcg ggggcaccgt cagtcttcct cttcccccca 780
aaacccaagg acaccctcat gatctcccgg acccctgagg tcacatgcgt ggtggtggac 840
gtgagccacg aagaccctga ggtcaagttc aactggtacg tggacggcgt ggaggtgcat 900
aatgccaaga caaagccgcg ggaggagcag tacaacagca cgtaccgtgt ggtcagcgtc 960
ctcaccgtcc tgcaccagga ctggctgaat ggcaaggagt acaagtgcaa ggtctccaac 1020
aaagccctcc cagcccccat cgagaaaacc atctccaaag ccaaagggca gccccgagaa 1080
ccacaggtgt acaccctgcc cccatcccgg gatgagctga ccaagaacca ggtcagcctg 1140
acctgcctgg tcaaaggctt ctatcccagc gacatcgccg tggagtggga gagcaatggg 1200
cagccggaga acaactacaa gaccacgcct cccgtgctgg actccgacgg ctccttcttc 1260
ctctacagca agctcaccgt ggacaagagc aggtggcagc aggggaacgt cttctcatgc 1320
tccgtgatgc atgaggctct gcacaaccac tacacgcaga agagcctctc cctgtctccg 1380
ggtaaatga                                                         1389

<210> 4
<211> 717
<212> DNA
<213> Artificial Sequence

<220>
<223> Humanised sequence of mus musculus and homo
      sapiens

<400> 4
atgggatgga gctgtatcat cctcttcttg gtagcaacag ctacaggtgt ccactccgat 60
attgtgatga cccagtctcc actctccaac cccgtcaccc ttggacagcc ggtctccatc 120
tcctgcaggt ctagtaagag tctcctatat aaggatggga agacatactt gaattggttt 180
ctccagaggc caggccaatc tccacagctc ctaatttatt tgatgtccac ccgtgcatct 240
ggggtcccag acagattcag cggcggtggg tcaggcactg atttcacact gaaaatcagc 300
agggtggagg ctgaggatgt tggggtttat tactgccaac aacttgtaga gtatccgctc 360
acgtttggcc aggggaccaa gctggagatc aaacgtacgg tggctgcacc atctgtcttc 420
atcttcccgc catctgatga gcagttgaaa tctggaactg cctctgttgt gtgcctgctg 480
aataacttct atcccagaga ggccaaagta cagtggaagg tggacaacgc cctccaatcg 540
ggtaactccc aggagagtgt cacagagcag gacagcaagg acagcaccta cagcctcagc 600
agcaccctga cgctgagcaa agcagactac gagaaacaca aagtctacgc ctgcgaagtc 660
acccatcagg gcctgagctc gcccgtcaca aagagcttca acaggggaga gtgttag    717

<210> 5
<211> 445
<212> PRT
```

<213> Artificial Sequence

<220>
<223> Humanised sequence of mus musculus and homo sapiens

<400> 5

```
Glu Val Gln Leu Val Glu Ser Gly Gly Gly Leu Val Gln Pro Gly Gly
 1               5                  10                  15
Ser Leu Arg Leu Ser Cys Ala Val Ser Gly Phe Thr Phe Ser Asp Asn
            20                  25                  30
Gly Met Ala Trp Val Arg Gln Ala Pro Gly Lys Gly Leu Glu Trp Val
        35                  40                  45
Ser Phe Ile Ser Asn Leu Ala Tyr Ser Ile Asp Tyr Ala Asp Thr Val
    50                  55                  60
Thr Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Val Ser Gly Thr Trp Phe Ala Tyr Trp Gly Gln Gly Thr Leu Val Thr
            100                 105                 110
Val Ser Ser Ala Ser Thr Lys Gly Pro Ser Val Phe Pro Leu Ala Pro
        115                 120                 125
Ser Ser Lys Ser Thr Ser Gly Gly Thr Ala Ala Leu Gly Cys Leu Val
    130                 135                 140
Lys Asp Tyr Phe Pro Glu Pro Val Thr Val Ser Trp Asn Ser Gly Ala
145                 150                 155                 160
Leu Thr Ser Gly Val His Thr Phe Pro Ala Val Leu Gln Ser Ser Gly
                165                 170                 175
Leu Tyr Ser Leu Ser Ser Val Val Thr Val Pro Ser Ser Ser Leu Gly
            180                 185                 190
Thr Gln Thr Tyr Ile Cys Asn Val Asn His Lys Pro Ser Asn Thr Lys
        195                 200                 205
Val Asp Lys Lys Val Glu Pro Lys Ser Cys Asp Lys Thr His Thr Cys
    210                 215                 220
Pro Pro Cys Pro Ala Pro Glu Leu Ala Gly Ala Pro Ser Val Phe Leu
225                 230                 235                 240
Phe Pro Pro Lys Pro Lys Asp Thr Leu Met Ile Ser Arg Thr Pro Glu
                245                 250                 255
Val Thr Cys Val Val Val Asp Val Ser His Glu Asp Pro Glu Val Lys
            260                 265                 270
Phe Asn Trp Tyr Val Asp Gly Val Glu Val His Asn Ala Lys Thr Lys
        275                 280                 285
Pro Arg Glu Glu Gln Tyr Asn Ser Thr Tyr Arg Val Val Ser Val Leu
    290                 295                 300
Thr Val Leu His Gln Asp Trp Leu Asn Gly Lys Glu Tyr Lys Cys Lys
305                 310                 315                 320
Val Ser Asn Lys Ala Leu Pro Ala Pro Ile Glu Lys Thr Ile Ser Lys
                325                 330                 335
Ala Lys Gly Gln Pro Arg Glu Pro Gln Val Tyr Thr Leu Pro Pro Ser
            340                 345                 350
Arg Asp Glu Leu Thr Lys Asn Gln Val Ser Leu Thr Cys Leu Val Lys
        355                 360                 365
Gly Phe Tyr Pro Ser Asp Ile Ala Val Glu Trp Glu Ser Asn Gly Gln
    370                 375                 380
Pro Glu Asn Asn Tyr Lys Thr Thr Pro Pro Val Leu Asp Ser Asp Gly
385                 390                 395                 400
Ser Phe Phe Leu Tyr Ser Lys Leu Thr Val Asp Lys Ser Arg Trp Gln
                405                 410                 415
Gln Gly Asn Val Phe Ser Cys Ser Val Met His Glu Ala Leu His Asn
            420                 425                 430
His Tyr Thr Gln Lys Ser Leu Ser Leu Ser Pro Gly Lys
        435                 440                 445
```

<210> 6
<211> 219
<212> PRT
<213> Artificial Sequence

<220>
<223> Humanised sequence of mus musculus and homo sapiens

<400> 6
Asp Ile Val Met Thr Gln Ser Pro Leu Ser Leu Pro Val Thr Pro Gly
1               5                   10                  15
Glu Pro Ala Ser Ile Ser Cys Arg Val Ser Gln Ser Leu Leu His Ser
            20                  25                  30
Asn Gly Tyr Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Thr
                85                  90                  95
Arg His Val Pro Tyr Thr Phe Gly Gly Gly Thr Lys Val Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215

<210> 7
<211> 442
<212> PRT
<213> Artificial Sequence

<220>
<223> Humanised sequence of mus musculus and homo sapiens

<400> 7
Gln Val Gln Leu Val Gln Ser Gly Ala Glu Val Lys Glu Pro Gly Ala
1               5                   10                  15
Ser Val Lys Val Ser Cys Lys Gly Ser Gly Phe Asn Ile Lys Val Tyr
            20                  25                  30
Tyr Val His Trp Leu Arg Gln Leu Pro Gly Lys Gly Leu Glu Trp Ile
        35                  40                  45
Gly Arg Ile Asp Pro Glu Asn Gly Glu Thr Ile Tyr Thr Pro Lys Phe
    50                  55                  60
Gln Asp Lys Ala Thr Leu Thr Val Asp Thr Ser Thr Asp Thr Ala Tyr
65                  70                  75                  80
Met Glu Leu Ser Ser Leu Arg Ser Glu Asp Thr Ala Val Tyr Tyr Cys
                85                  90                  95
Val Ser Ser Gly Tyr Trp Gly Gln Gly Thr Leu Val Thr Val Ser Ser
            100                 105                 110
Ala Ser Thr Lys Gly Pro Ser Val Phe Pro Leu Ala Pro Ser Ser Lys

```
        115                 120                 125
Ser Thr Ser Gly Gly Thr Ala Ala Leu Gly Cys Leu Val Lys Asp Tyr
    130                 135                 140
Phe Pro Glu Pro Val Thr Val Ser Trp Asn Ser Gly Ala Leu Thr Ser
145                 150                 155                 160
Gly Val His Thr Phe Pro Ala Val Leu Gln Ser Ser Gly Leu Tyr Ser
                165                 170                 175
Leu Ser Ser Val Val Thr Val Pro Ser Ser Ser Leu Gly Thr Gln Thr
            180                 185                 190
Tyr Ile Cys Asn Val Asn His Lys Pro Ser Asn Thr Lys Val Asp Lys
        195                 200                 205
Lys Val Glu Pro Lys Ser Cys Asp Lys Thr His Thr Cys Pro Pro Cys
    210                 215                 220
Pro Ala Pro Glu Leu Ala Gly Ala Pro Ser Val Phe Leu Phe Pro Pro
225                 230                 235                 240
Lys Pro Lys Asp Thr Leu Met Ile Ser Arg Thr Pro Glu Val Thr Cys
                245                 250                 255
Val Val Val Asp Val Ser His Glu Asp Pro Glu Val Lys Phe Asn Trp
            260                 265                 270
Tyr Val Asp Gly Val Glu Val His Asn Ala Lys Thr Lys Pro Arg Glu
        275                 280                 285
Glu Gln Tyr Asn Ser Thr Tyr Arg Val Val Ser Val Leu Thr Val Leu
    290                 295                 300
His Gln Asp Trp Leu Asn Gly Lys Glu Tyr Lys Cys Lys Val Ser Asn
305                 310                 315                 320
Lys Ala Leu Pro Ala Pro Ile Glu Lys Thr Ile Ser Lys Ala Lys Gly
                325                 330                 335
Gln Pro Arg Glu Pro Gln Val Tyr Thr Leu Pro Pro Ser Arg Asp Glu
            340                 345                 350
Leu Thr Lys Asn Gln Val Ser Leu Thr Cys Leu Val Lys Gly Phe Tyr
        355                 360                 365
Pro Ser Asp Ile Ala Val Glu Trp Glu Ser Asn Gly Gln Pro Glu Asn
    370                 375                 380
Asn Tyr Lys Thr Thr Pro Pro Val Leu Asp Ser Asp Gly Ser Phe Phe
385                 390                 395                 400
Leu Tyr Ser Lys Leu Thr Val Asp Lys Ser Arg Trp Gln Gln Gly Asn
                405                 410                 415
Val Phe Ser Cys Ser Val Met His Glu Ala Leu His Asn His Tyr Thr
            420                 425                 430
Gln Lys Ser Leu Ser Leu Ser Pro Gly Lys
        435                 440
```

<210> 8
<211> 218
<212> PRT
<213> Artificial Sequence

<220>
<223> Humanised sequence of mus musculus and homo sapiens

<400> 8

```
Asp Ile Val Met Thr Gln Ser Pro Leu Ser Asn Pro Val Thr Pro Gly
 1               5                  10                  15
Glu Pro Ala Ser Ile Ser Cys Arg Ser Ser Lys Ser Leu Leu His Arg
            20                  25                  30
Asn Gly Ile Thr Tyr Leu Tyr Trp Tyr Leu Gln Lys Pro Gly Gln Ser
        35                  40                  45
Pro Gln Leu Leu Ile Tyr Gln Met Ser Asn Leu Ala Ser Gly Val Pro
    50                  55                  60
Asp Arg Phe Ser Ser Ser Gly Ser Gly Thr Asp Phe Thr Leu Lys Ile
65                  70                  75                  80
Ser Arg Val Glu Ala Glu Asp Val Gly Val Tyr Tyr Cys Ala Gln Asn
                85                  90                  95
```

```
Leu Glu Leu Trp Thr Phe Gly Gln Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215


<210> 9
<211> 6
<212> PRT
<213> Artificial Sequence

<220>
<223> Humanised sequence of mus musculus and homo
      sapiens

<400> 9
Tyr Ala Gly Phe Leu Arg
1               5


<210> 10
<211> 130
<212> PRT
<213> Artificial Sequence

<220>
<223> Humanised sequence of mus musculus and homo
      sapiens

<400> 10
Glu Val Gln Leu Leu Val Ser Gly Gly Gly Leu Val Gln Pro Gly Gly
1               5                   10                  15
Ser Leu Arg Leu Ser Cys Ala Ala Ser Gly Phe Thr Phe Lys Ala Tyr
            20                  25                  30
Pro Met Met Trp Val Arg Gln Ala Pro Gly Lys Gly Leu Glu Trp Val
        35                  40                  45
Ser Glu Ile Ser Pro Ser Gly Ser Tyr Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Pro Arg Lys Leu Asp Tyr Trp Gly Gln Gly Thr Leu Val
            100                 105                 110
Thr Val Ser Ser Ala Ala Ala Glu Gln Lys Leu Ile Ser Glu Glu Asp
        115                 120                 125
Leu Asn
    130


<210> 11
<211> 115
<212> PRT
```

<213> Artificial Sequence

<220>
<223> Humanised sequence of mus musculus and homo sapiens

<400> 11

```
Asp Ile Val Met Thr Gln Ser Pro Asp Ser Leu Ala Val Ser Leu Gly
 1               5                  10                  15
Glu Arg Ala Thr Ile Asn Cys Lys Ser Ser His Ser Val Leu Tyr Ser
            20                  25                  30
Ser Asn Gln Lys Asn Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys His Gln
                85                  90                  95
Tyr Leu Ser Ser Leu Thr Phe Gly Gln Gly Thr Lys Leu Glu Ile Lys
            100                 105                 110
Arg Thr Val
        115
```

<210> 12
<211> 126
<212> PRT
<213> Artificial Sequence

<220>
<223> Humanised sequence of mus musculus and homo sapiens

<400> 12

```
Gln Val Gln Leu Val Gln Ser Gly Ser Glu Leu Lys Lys Pro Gly Ala
 1               5                  10                  15
Ser Val Lys Val Ser Cys Lys Ala Ser Gly Tyr Thr Phe Thr Asn Tyr
            20                  25                  30
Gly Met Asn Trp Val Arg Gln Ala Pro Gly Gln Gly Leu Glu Trp Met
        35                  40                  45
Gly Trp Ile Asn Thr Tyr Thr Gly Glu Pro Thr Tyr Ala Asp Asp Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Asn Pro Ile Asn Tyr Tyr Gly Ile Asn Tyr Glu Gly Tyr Val
            100                 105                 110
Met Asp Tyr Trp Gly Gln Gly Thr Leu Val Thr Val Ser Ser
        115                 120                 125
```

<210> 13
<211> 1335
<212> DNA
<213> Artificial Sequence

<220>
<223> Humanised sequence of mus musculus and homo sapiens

<400> 13

```
gaggtgcagc tggtggagtc tgggggaggc ttggtacagc ctggggggtc cctgagactc 60
tcctgtgcag tctctggatt caccttcagt gacaacggaa tggcgtgggt ccgccaggct 120
```

```
ccagggaagg ggctggagtg ggtttcattc attagtaatt tggcatatag tatcgactac 180
gcagacactg tgacgggccg attcaccatc tccagagaca atgccaagaa ctcactgtat 240
ctgcaaatga acagcctgag agccgaggac acggctgtgt attactgtgt cagcgggacc 300
tggtttgctt actggggcca gggcacacta gtcacagtct cctcagcctc caccaagggc 360
ccatcggtct tccccctggc accctcctcc aagagcacct ctgggggcac agcggccctg 420
ggctgcctgg tcaaggacta cttccccgaa ccggtgacgg tgtcgtggaa ctcaggcgcc 480
ctgaccagcg gcgtgcacac cttcccggct gtcctacagt cctcaggact ctactccctc 540
agcagcgtgg tgaccgtgcc ctccagcagc ttgggcaccc agacctacat ctgcaacgtg 600
aatcacaagc ccagcaacac caaggtggac aagaaagttg agcccaaatc ttgtgacaaa 660
actcacacat gcccaccgtg cccagcacct gaactcgcgg gggcaccgtc agtcttcctc 720
ttccccccaa aacccaagga caccctcatg atctcccgga cccctgaggt cacatgcgtg 780
gtggtggacg tgagccacga agaccctgag gtcaagttca actggtacgt ggacggcgtg 840
gaggtgcata atgccaagac aaagccgcgg gaggagcagt acaacagcac gtaccgtgtg 900
gtcagcgtcc tcaccgtcct gcaccaggac tggctgaatg gcaaggagta caagtgcaag 960
gtctccaaca aagccctccc agcccccatc gagaaaacca tctccaaagc caaagggcag 1020
ccccgagaac cacaggtgta caccctgccc ccatcccggg atgagctgac caagaaccag 1080
gtcagcctga cctgcctggt caaaggcttc tatcccagcg acatcgccgt ggagtgggag 1140
agcaatgggc agccggagaa caactacaag accacgcctc ccgtgctgga ctccgacggc 1200
tccttcttcc tctacagcaa gctcaccgtg gacaagagca ggtggcagca ggggaacgtc 1260
ttctcatgct ccgtgatgca tgaggctctg cacaaccact acacgcagaa gagcctctcc 1320
ctgtctccgg gtaaa                                                  1335
```

```
<210> 14
<211> 657
<212> DNA
<213> Artificial Sequence

<220>
<223> Humanised sequence of mus musculus and homo
      sapiens

<400> 14
gacatcgtga tgacccagag cccccctgagc ctgcccgtga cccctggcga gcccgccagc 60
```

```
atcagctgta gagtgagcca gagcctgctg cacagcaacg gctacaccta cctgcactgg 120
tatctgcaga agcctggcca gagccctcag ctgctgatct acaaggtgtc caaccggttc 180
agcggcgtgc ctgatagatt cagcggcagc ggctccggca ccgacttcac cctgaagatc 240
agcagagtgg aggccgagga tgtgggcgtg tactactgct cccagaccag acacgtgcct 300
tacacctttg gcggcggaac aaaggtggag atcaagcgta cggtggccgc ccccagcgtg 360
ttcatcttcc cccccagcga tgagcagctg aagagcggca ccgccagcgt ggtgtgtctg 420
ctgaacaact tctacccccg ggaggccaag gtgcagtgga aggtggacaa tgccctgcag 480
agcggcaaca gccaggagag cgtgaccgag caggacagca aggactccac ctacagcctg 540
agcagcaccc tgaccctgag caaggccgac tacgagaagc acaaggtgta cgcctgtgag 600
gtgacccacc agggcctgtc cagccccgtg accaagagct tcaaccgggg cgagtgc    657
```

```
<210> 15
<211> 657
<212> DNA
<213> Artificial Sequence

<220>
<223> Humanised sequence of mus musculus and homo
      sapiens

<400> 15
gatattgtga tgactcagtc tccactctcc ctgcccgtca cccctggaga gccggcctcc 60
atctcctgca gagttagtca gagcctttta cacagtaatg gatacaccta tttacattgg 120
tacctgcaga agccagggca gtctccacag ctcctgatct ataaagtttc caaccgattt 180
tctggggtcc ctgacaggtt cagtggcagt ggatcaggca cagattttac actgaaaatc 240
agcagagtgg aggctgagga tgttggggtt tattactgct ctcaaactag acatgttccg 300
tacacgttcg gcggagggac caaggtggaa atcaaacgta cggtggctgc accatctgtc 360
ttcatcttcc cgccatctga tgagcagttg aaatctggaa ctgcctctgt tgtgtgcctg 420
ctgaataact tctatcccag agaggccaaa gtacagtgga aggtggacaa cgccctccaa 480
tcgggtaact cccaggagag tgtcacagag caggacagca aggacagcac ctacagcctc 540
agcagcaccc tgacgctgag caaagcagac tacgagaaac acaaagtcta cgcctgcgaa 600
gtcacccatc agggcctgag ctcgcccgtc acaaagagct tcaacagggg agagtgt    657
```